(19) Europäisches Patentamt
European Patent Office
Office européen des brevets

(11) Veröffentlichungsnummer : **0 420 803 B1**

(12) # EUROPÄISCHE PATENTSCHRIFT

(45) Veröffentlichungstag der Patentschrift : **09.03.94 Patentblatt 94/10**

(51) Int. Cl.$^5$ : **C07D 285/14,** C07D 417/12, A01N 43/82

(21) Anmeldenummer : **90810705.5**

(22) Anmeldetag : **17.09.90**

(54) **Benzo-1,2,3-thiadiazol-Derivate und ihre Verwendung zum Schutz von Pflanzen gegen Krankheiten.**

Die Akte enthält technische Angaben, die nach dem Eingang der Anmeldung eingereicht wurden und die nicht in dieser Patentschrift enthalten sind.

(30) Priorität : **26.09.89 CH 3480/89**
**25.07.90 CH 2483/90**

(43) Veröffentlichungstag der Anmeldung : **03.04.91 Patentblatt 91/14**

(45) Bekanntmachung des Hinweises auf die Patenterteilung : **09.03.94 Patentblatt 94/10**

(84) Benannte Vertragsstaaten : **AT BE CH DE DK ES FR GB GR IT LI LU NL SE**

(56) Entgegenhaltungen :
**EP-A- 0 313 512**
**EP-A- 0 332 104**
**GB-A- 1 176 799**
**US-A- 2 494 355**

(73) Patentinhaber : **CIBA-GEIGY AG**
**Klybeckstrasse 141**
**CH-4002 Basel (CH)**

(72) Erfinder : **Kunz, Walter, Dr.**
**Buchenstrasse 9**
**CH-4104 Oberwil (CH)**
Erfinder : **Schurter, Rolf, Dr.**
**Holzmattstrasse 45**
**CH-4102 Binningen (CH)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Benzo-1,2,3-thiadiazol-Derivate der nachstehenden Formel I. Die Erfindung betrifft ferner die Herstellung dieser Substanzen sowie die als Wirkstoffe mindestens eine dieser Verbindungen enthaltenden Mittel. Die Erfindung betrifft darüber hinaus die Herstellung der genannten Mittel sowie die Verwendung der Wirkstoffe oder der Mittel zum Schutz von Pflanzen gegen den Befall durch schädliche Mikroorganismen, beispielsweise pflanzenschädigende Pilze, Bakterien und Viren.

Die erfindungsgemässen Verbindungen entsprechen der allgemeinen Formel I

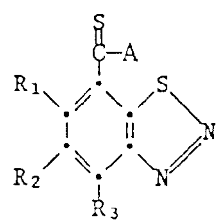

(I),

worin bedeuten:

$R_1$, $R_2$ und $R_3$ voneinander unabhängig Wasserstoff, Halogen, Methyl, Methoxy oder Methylthio;

A $-OR_4$, $-SR_4$, $-N(R_5)R_6$, $-N(R_7)-N(R_5)R_6$, $-O-N=C(R_5)R_9$, $-N(R_7)-OR_{10}$,

oder

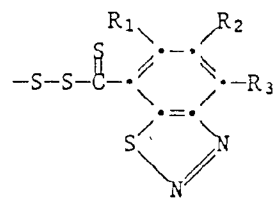

,

$R_4$ $C_1$-$C_{18}$-Alkyl, durch ein Sauerstoff- oder Schwefelatom unterbrochenes $C_3$-$C_{35}$-Alkyl, durch Halogen oder $-COOR_7$ substituiertes $C_1$-$C_8$-Alkyl, unsubstituiertes oder durch Halogen substituiertes $C_3$-$C_6$-Alkenyl, unsubstituiertes oder durch Halogen substituiertes $C_3$-$C_6$-Alkinyl, unsubstituiertes oder durch Halogen oder Methyl substituiertes $C_4$-$C_7$-Cycloalkyl, einen Aryl- oder heterocyclischen Rest U mit maximal 3 Heteroatomen O, N und/oder S, einen über eine maximal 6 Kohlenstoffatome und bis zu 2 Sauerstoffatome enthaltende Alkylenbrucke verknüpften Rest U;

U unsubstituiertes oder durch Halogen, $C_1$-$C_3$-Alkyl oder $C_1$-$C_4$-Alkoxy substituiertes Phenyl, Phenoxyphenyl, Biphenyl, 1- oder 2-Naphthyl, oder einen unsubstituierten oder durch Halogen, $C_1$-$C_3$-Alkyl und/oder $C_1$-$C_3$-Alkoxy ein- oder mehrfach substituierten gesättigten oder ungesättigten 5- bis 7-gliedrigen Heterocyclus T;

$R_5$ und $R_6$ unabhängig voneinander Wasserstoff, $C_1$-$C_8$-Alkyl, durch $C_1$-$C_3$-Alkoxy und/oder Cyano substituiertes $C_1$-$C_6$-Alkyl, oder $C_3$-$C_6$-Alkenyl, $C_3$-$C_6$-Alkinyl, $C_3$-$C_7$-Cycloalkyl, einen Rest U, einen über eine maximal 2 C-Atome umfassende Alkylenbrücke verknüpften Rest U;

oder $R_5$ und R6 mit dem Stickstoffatom zusammen einen 5- bis 7-gliedrigen unsubstituierten oder durch Methyl substituierten Heterocyclus W mit maximal 2 weiteren Heteroatomen O, N und/oder S;

$R_7$ Wasserstoff oder $C_1$-$C_4$-Alkyl;

$R_8$ und $R_9$ unabhängig voneinander Wasserstoff, $C_1$-$C_6$-Alkyl, Cyano, $CONH_2$, $CONHCONHR_7$, unsubstituiertes oder durch Halogen, $C_1$-$C_3$-Alkyl oder $C_1$-$C_3$-Alkoxy substituiertes Phenyl; oder, zusammen mit dem verbindenden Kohlenstoffatom auch einen 5-7-gliedrigen Carbocyclus; und

$R_{10}$ Wasserstoff, $C_1$-$C_6$-Alkyl, $C_3$-$C_6$-Alkenyl, $C_5$-$C_7$-Cycloalkyl, Phenyl oder Benzyl.

Als Cycloalkyle und carbocyclische Ringe seien z.B. genannt Cyclopropan, Cyclobutan, Cyclopentan, Cyclohexan oder Cycloheptan, vorzugsweise Cyclopropan, Cyclopentan und Cyclohexan. Unter Heterocyclus T ist z.B. zu verstehen Pyrrolidin-1-yl, Piperidin-1-yl, Azacyclohept-1-yl, Morpholin-4-yl, 2,6-Dimethylmorpholin-4-yl, Imidazol-l-yl, 1,2,4-Triazol-1-yl, Pyrrol-1-yl, 2-, 3- oder 4-Pyridyl, 2-, 4- oder 5-Pyrimidyl, Isoxazolyl-3- oder 5-yl, 2-Thienyl, 2-Furanyl, 2-Thiazolyl, 1,3,4-Thiadiazol-2-yl, Tetrahydrofuran-2-on-3-yl, Tetrahydropyran-2-on-3-yl oder Tetramethylpiperidin-4-yl.

Als heterocyclische Reste W seien Pyrrolidin, Piperidin, Azacycloheptan, Morpholin, 2,6-Dimethylmorpholin, Imidazol-1-yl, 1,2,4-Triazol-1-yl oder Pyrrol-1-yl genannt.

Halogen bedeutet Fluor, Chlor, Brom oder Jod, bevorzugt Fluor und weiter in der Reihenfolge Chlor, Brom und Jod. Als Substituent in einzelnen Resten kann Halogen 1- bis 3-fach vertreten sein.

Unter Alkyl selbst oder als Bestandteil eines anderen Substituenten sind gerad- und verzweigtkettige Alkyle zu verstehen. Sie stellen je nach Zahl der angegebenen Kohlenstoffatome beispielsweise folgende bevorzugte Gruppen dar: Methyl, Ethyl sowie die Isomeren von Propyl, Butyl, Pentyl oder Hexyl, wie z.B. Isopropyl, Isobutyl, tert.-Butyl, sek.-Butyl oder Isopentyl.

Alkenyl steht z.B. für Propenyl-(1), Allyl, Butenyl-(1), Butenyl-(2) oder Butenyl-(3) und Alkinyl bedeutet z.B. Propinyl-(2), Butinyl-(1) oder Pentinyl-(4).

Als salzbildende Amine sind z.B. anzusehen:

Trimethylamin, Triethylamin, Tripropylamin, Tributylamin, Tribenzylamin, Tricyclohexylamin, Triamylamin, Trihexylamin, N,N-Dimethylanilin, N,N-Dimethyltoluidin, N,N-Dimethyl-p-amino-pyridin, N-Methylpyrrolidin, N-Methylpiperidin, N-Methyl-pyrrolidin, N-Methylimidazol, N-Methylpyrrol, N-Methylmorpholin, N-Methylhexamethylenimin, Pyridin, Chinolin, alpha-Picolin, β-Picolin, Isochinolin, Pyrimidin, Acridin, N,N,N',N'-Tetra-methylethylendiamin, N,N,N',N'-Tetraethylethylendiamin, Chinoxalin, N-Propyldiisopropylamin, N,N-Dimethylcyclohexylamin, 2,6-Lutidin, 2,4-Lutidin, Triethylendiamin sowie heterocyclische Amine vom Morpholintyp.

Als Basen oder Verbindungen basischen Charakters kommen anorganische Basen oder Basenbildner in Betracht, wie z.B. Hydroxide, Carbonate und Hydrogencarbonate der Alkali- und Erdalkalimetalle, vorzugsweise LiOH, NaOH, KOH, $Mg(OH)_2$ oder $Ca(OH)_2$; und ferner $NaHCO_3$, $KHCO_3$, $Na_2CO_3$ und $K_2CO_3$.

Aufgrund ihrer besonderen pflanzenschützenden Eigenschaften lassen sich die Verbindungen der Formel I in folgende Gruppen einteilen:

1. Verbindungen der Formel I, worin bedeuten:

$R_1$, $R_2$ und $R_3$ unabhängig voneinander Wasserstoff, Fluor oder Methyl; A $-OR_4$, $-SR_4$, $-N(R_5)R_6$, $-N(R_7)-N(R_5)R_5$, $-O-N=C(R_8)R_9$, $-N(R_7)-OR_{10}$,

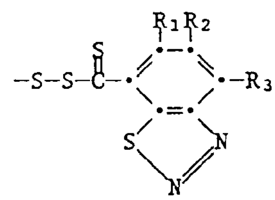 ;

$R_4$ $C_1$-$C_6$-Alkyl, $C_5$-$C_6$-Cycloalkyl, Phenyl, Benzyl, Tetrahydrofuran-2-on-3-yl, 2-, 3- oder 4-Pyridyl, 2-, 4- oder 5-Pyrimidyl, (2,2-Dimethyldioxolan-4-yl)-methyl;

$R_5$ und $R_5$ unabhängig voneinander Wasserstoff, $C_1$-$C_4$-Alkyl, durch Methoxy, Ethoxy und/oder Cyano substituiertes $C_1$-$C_3$-Alkyl, oder Allyl, Propargyl, $C_3$-$C_6$-Cycloalkyl, Phenyl, Benzyl, Phenethyl, 2-Furfurylmethyl, Tetrahydrofuran-2-on-3-yl, 2-, 3- oder 4-Pyridyl, 2-, 4- oder 5-Pyrimidyl, $R_7$ Wasserstoff, Methyl oder Ethyl; oder

die Gruppe $N(R_5)R_5$ zusammen einen Pyrrol-, Pyrrolidin-, Imidazol-1-yl, 1,2,4-Triazol-1-yl-, Piperidin-, Morpholin- oder 2,6-Dimethylmorpholin-Ring bildet;

$R_8$ und $R_9$ unabhängig voneinander Wasserstoff, $C_1$-$C_6$-Alkyl, Cyano, die Reste $CONN_2$, $CONHCONHR_7$, Phenyl oder zusammen mit dem verbindenden Kohlenstoffatom einen 5- bis 7-gliedrigen Carbocyclus bilden; und

$R_{10}$ Wasserstoff, $C_1$-$C_3$-Alkyl, Allyl, $C_5$-$C_6$-Cycloalkyl, Phenyl oder Benzyl.

2. Verbindungen der Formel I, worin bedeuten:

$R_1$, $R_2$ und $R_3$ unabhängig voneinander Wasserstoff, Fluor oder Methyl;

A $-OR_4$, $-SR_4$, $-N(R_5)R_5$, $-N(R_7)-N(R_5)R_6$, $-O-N=C(R_8)R_9$ oder $-N(R_7)-OR_{10}$;

$R_4$ $C_1$-$C_6$-Alkyl, $C_5$-$C_6$-Cycloalkyl, Phenyl, Benzyl, Tetrahydrofuran-2-on-3-yl, 2-, 3- oder 4-Pyridyl;

$R_5$ und $R_5$ unabhängig voneinander Wasserstoff, $C_1$-$C_3$-Alkyl, durch Methoxy und/oder Cyano substituiertes $C_1$-$C_3$-Alkyl, oder Allyl, Propargyl, $C_3$-$C_5$-Cycloalkyl, Phenyl, Benzyl, 2-Furfuryl, Tetrahydrofuran-2-on-3-yl, 2-, 3- oder 4-Pyridyl, 2-, 4-oder 5-Pyrimidyl, Piperidin-1-yl-, oder die Gruppe $N(R_5)R_5$ zusammen einen Pyrrolidin-, Imidazol-1-yl-, Piperidin-, Morpholin- oder 2,6-Dimethylmorpholin-Ring darstellt;

$R_7$ Wasserstoff, Methyl oder Ethyl;

$R_8$ und $R_9$ unabhängig voneinander Wasserstoff, Methyl, Ethyl oder die Reste Cyano, $CONH_2$, Phenyl, Benzyl oder zusammen mit dem verbindenden Kohlenstoffatom Cyclopentyl oder Cyclohexyl; und

$R_{10}$ Wasserstoff, Methyl, Ethyl, Allyl, $C_3$-$C_5$-Cycloalkyl oder Phenyl.

3. Verbindungen der Formel I, worin bedeuten:

$R_1$, $R_2$ und $R_3$ unabhängig voneinander Wasserstoff oder Fluor;
A $-OR_4$, $-SR_4$, $-N(R_5)R_6$, $-N(R_7)-N(R_6)R_6$;
$R_4$ $C_1$-$C_4$-Alkyl, $C_5$-$C_6$-Cycloalkyl, Phenyl, Benzyl, 2-, 3- oder 4-Pyridyl, 2-, 4- oder 5-Pyrimidyl;
$R_5$ und $R_6$ unabhängig voneinander Wasserstoff, Methyl, Ethyl, Allyl, Propargyl, Phenyl, Benzyl, 2-Furanylmethyl, 2-, 3- oder 4-Pyridyl, oder die Gruppe $N(R_6)R_6$ gemeinsam für einen Pyrrolidin-, Piperidin-, Morpholin-, 2,6-Dimethylmorpholin- oder Imidazol-1-yl-Ring steht; und
$R_7$ Wasserstoff, Methyl oder Ethyl.

Folgende Verbindungen zeichnen sich durch besonders vorteilhafte pflanzenschützende Eigenschaften aus:
Benzo-1,2,3-thiadiazol-7-thio-carbonsäure-O-ethylester ;
Benzo-1,2,3-thiadiazol-7-dithio-carbonsäure--methylester ;
Benzo-1,2,3-thiadiazol-7-carbonsäurethioamid ;
7-[Benzo-1,2,3-thiadiazol]-N',N'-dimethylthiohydrazid ;
Benzo-1,2,3-thiadiazol-7-dithiocarbonsäure--ethylester ;
Benzo-1,2,3-thiadiazol-7-dithiocarbonsäure--propylester ;
Benzo-1,2,3-thiadiazol-7-dithiocarbonsäure--cyclopentylester ;
Benzo-1,2,3-thiadiazol-7-thiocarbonsäure-morpholid ;
Benzo-1,2,3-thiadiazol-7-thiocarbonsäure-piperidid.

Benzo-1,2,3-thiadiazol-7-carbonsäuren und Derivate sowie ihre Verwendung im Pflanzenschutz sind bekannt, z.B. Benzo-1,2,3-thiadiazol-7-carbonsäureester mit biocider Wirkung aus GB-A-1,176,799, Benzo-1,2,3-thiadiazol-7-carbonsäure-Derivate mit pflanzenimmunisierender Wirkung aus EP-A-313,512 und Benzo-1,2,3-thiadiazol-7-thiocarbonsäuren und Derivate als Regulatoren für Pflanzengene aus EP-A-332,104. Ferner sind aromatische Dithiocarbonsäuren mit fungizider Wirkung aus US-A-2,494,355 bekannt.

Es wurde nun uberraschenderweise gefunden, dass die erfindungsgemässen Verbindungen der Formel I durch ihre Verwendung den Befall von Pflanzen durch schädliche Mikroorganismen verhindern und damit den befallsbedingten Schädigungen der Pflanzen vorbeugen. Für die erfindungsgemässen Wirkstoffe ist charakteristisch, dass der Schutz der Pflanzen sowohl durch direkte Einwirkung auf die pflanzenschädigenden Mikroorganismen mittels Blattapplikation oder Bodenapplikation als auch durch Aktivierung und Stimulierung des pflanzeneigenen Abwehrsystems (Immunisierung) eintreten kann. Der grosse Vorteil der Verbindungen der Formel I besteht darin, dass die Gesunderhaltung der mit diesen Stoffen behandelten Pflanzen auch aus eigener Kraft ohne Einsatz weiterer mikrobizider Substanzen während der Vegetationsperiode gewährleistet werden kann. Demzufolge ist es möglich, durch die Anwendung der erfindungsgemässen Wirkstoffe nachteilige Nebeneffekte, wie sie bei der direkten Parasitenbekämpfung mit chemischen Substanzen z.B. einerseits durch Schädigung der Nutzpflanzen (Phytotoxizität) und andererseits durch Hervorrufung von Resistenzerscheinungen bei den schädlichen Mikroorganismen in Erscheinung treten können, zu vermeiden, was vorteilhafterweise ein völlig ungestörtes Wachstum der Nutzpflanzen zur Folge hat.

Aufgrund der doppelten Wirkungsweise der erfindungsgemässen Verbindungen der Formel I, nämlich einerseits der direkten Bekämpfung der Pflanzenpathogene und andererseits der Erhöhung der allgemeinen Abwehrbereitschaft der mit diesen Wirkstoffen behandelten Pflanzen durch Immunisierung kann ein breit gefächerter Schutz der Pflanzen gegen Krankheiten erzielt werden. Die Anwendung der erfindungsgemässen Wirkstoffe ist deshalb besonders für praktische Bedingungen geeignet. Darüber hinaus bewirkt die den Verbindungen der Formel I eigene systemische Aktivität, dass sich der Schutzeffekt auch auf zuwachsende Pflanzenteile der behandelten Pflanzen erstreckt.

Die allgemein pflanzenschützende Aktivität der erfindungsgemässen Wirkstoffe ist z.B. gegen die den folgenden Klassen angehörenden phytopathogenen Fungi wirksam: Fungi imperfecti (z.B. Botrytis, Helminthosporium, Fusarium, Septoria, Cercospora und Alternaria); Basidiomyceten (z.B. Hemileia, Rhizocotonia, Puccinia); Ascomyceten (z.B. Venturia, Podosphaera, Erysiphe, Monilinia, Uncinula).

Darüber hinaus können die Wirkstoffe besonders vorteilhaft gegen folgende Schadorganismen eingesetzt werden:
Pilze, wie z.B. Oomyceten (z.B. Plasmopara viticola, Phytophthora infestans, Peronospora tabacina, Pseudoperonospora, Bremia letucae), Fungi imperfecti (z.B. Colletotrichum lagenarium, Piricularia oryzae, Cercospora nicotinae), Ascomyceten (z.B. Venturia inaequalis); Bakterien, wie z.B. Pseudomonaden (Pseudomonas lachrymans, Pseudomonas tomato, Pseudomonas tabaci); Xanthomonaden (z.B. Xanthomonas oryzae, Xanthomonas vesicatoria); Erwinia (z.B. Erwinia amylovora); und Viren, wie z.B. das Tabakmosaikvirus.

Die erfindungsgemässen Verbindungen können zum Schutz von Pflanzen unterschiedlicher Nutzkulturen eingesetzt werden.

Für den Einsatz der erfindungsgemässen Verbindungen der Formel I im Rahmen der Erfindung sind beispielsweise folgende Pflanzenarten geeignet: Getreide (Weizen, Gerste, Roggen, Hafer, Reis, Sorghum und Verwandte); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Grapefruit, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Als besonders geeignete Zielkulturen für die Anwendung des erfindungsgemässen Verfahrens sind folgende Pflanzen anzusehen: Gurke, Tabak, Reben, Reis, Pfeffer, Kartoffeln, Tomaten, Weizen, Gerste, Birnen und Aepfel.

Darüber hinaus stellen Verbindungen der Formeln III, V und VI

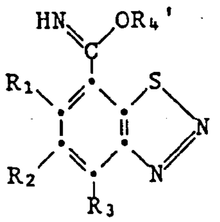

(III)

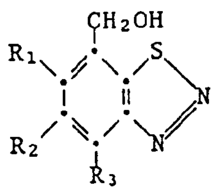

(V)

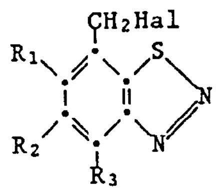

(VI)

,

worin bedeuten:

$R_1$, $R_2$ und $R_3$ voneinander unabhängig Wasserstoff, Halogen, Methyl, Methoxy oder Methylthio;

$R_4$' $C_1$-$C_{18}$-Alkyl, durch ein Sauerstoff- oder Schwefelatom unterbrochenes $C_3$-$C_{35}$-Alkyl, durch Halogen oder -$COOR_7$ substituiertes $C_1$-$C_8$-Alkyl, unsubstituiertes oder durch Halogen substituiertes $C_3$-$C_6$-Alkenyl, unsubstituiertes oder durch Halogen substituiertes $C_3$-$C_6$-Alkinyl, unsubstituiertes oder durch Halogen oder Methyl substituiertes $C_4$-$C_7$-Cycloalkyl, einen Aryl- oder heterocyclischen Rest U mit maximal 3 Heteroatomen O, N und/oder S, einen über eine maximal 6 Kohlenstoffatome und bis zu 2 Sauerstoffatome enthaltende Alkylenbrücke verknüpften Rest U;

U unsubstituiertes oder durch Halogen, $C_1$-$C_3$-Alkyl oder $C_1$-$C_4$-Alkoxy substituiertes Phenyl, Phenoxyphenyl, Biphenyl, 1- oder 2-Naphthyl, oder einen unsubstituierten oder durch Halogen, $C_1$-$C_3$-Alkyl und/oder $C_1$-$C_3$-Alkoxy ein- oder mehrfach substituierten gesättigten oder ungesättigten 5- bis 7-gliedrigen Heterocyclus T; und

Hal Halogen, vorzugsweise Chlor;

einen Teil der vorliegenden Erfindung dar. Diese Verbindungen sind neu. Sie fungieren erfindungsgemäss einerseits als Vorstufen in der Synthese der Verbindungen der Formel I und bilden andererseits selbständige Gruppen von fungizid wirksamen Verbindungen. Sie sind als Wirkstoffe ebenso wie die Verbindungen der Formel I gegen phytopathogene Mikroorganismen, insbesondere gegen Fungi, zum Schutz der vorgenannten Pflanzenkulturen einsetzbar.

Als Wirkstoffe mit besonders vorteilhaften pflanzenschützenden Eigenschaften sind folgende Verbindungen hervorzuheben:

7-Hydroxymethyl-benzo-1,2,3-thiadiazol (Verb.Nr. 6.1);

7-Chlormethyl-benzo-1,2,3-thiadiazol (Verb.Nr. 7.1);

Benzo-1,2,3-thiadiazol-7-ethylester-imid-Hydrochlorid (Verb.Nr. 5.2).

Die Verbindungen der Formel I werden wie folgt gruppenweise hergestellt:

A) Umsetzung einer Verbindung der Formel IIa

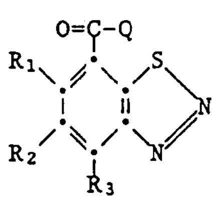

(IIa)

mit einem Thionierungsreagens, z.B. mit Phosphorpentasulfid ($P_4S_{10}$) oder Lawesson-Reagens [2,4-Bis-(4-methoxyphenyl)-1,3,2,4-dithiadiphosphetan-2,4-disulfid] in einem aprotischen unpolaren oder leicht polaren Lösungsmittel bei Temperaturen von 20° bis 200°C, bevorzugt 60° bis 150°C, wobei Q die Reste $OR_4$, $-SR_4$ oder $-N(R_5)R_6$ darstellt, zu einer Verbindung der Formel Ia

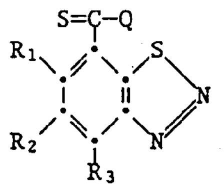

(Ia)

worin $R_1$, $R_2$ und $R_3$ die unter Formel I angegebenen Bedeutungen besitzen;
$B_1$) Umsetzung der Lösung eines Nitrils der Formel II

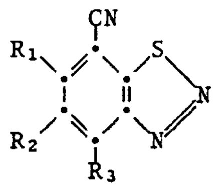

(II)

in einem Alkohol der Formel $R_4$'OH, wobei $R_4$' wie unter Formel III definiert, durch Einleiten von gasförmigem Halogenwasserstoff, vorzugsweise Chlorwasserstoff, bei Temperaturen von -20° bis 80°C, vorzugsweise -10° bis 40°C, zu einem Imidat der Formel III

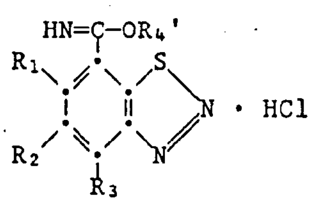

(III);

und
$B_2$) anschliessende Umsetzung der erhaltenen Verbindung der Formel III mit Schwefelwasserstoff in unpolaren oder schwach polaren Lösungsmitteln in Gegenwart einer Base bei Temperaturen von -20° bis 80°C, vorzugsweise -5° bis 40°C, zu Thiocarbonsäureestern der Formel Ia'

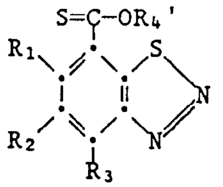

(Ia')

wobei $R_1$, $R_2$, $R_3$ die unter Formel I und $R_4$' die unter Formel III angegebenen Bedeutungen besitzen;
C) Umsetzung einer Thiono-Verbindung der Formel IV

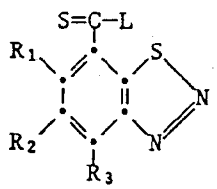

(IV),

worin L die Bedeutung von Chlor oder eines Azolids der Formel

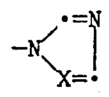

,

in welcher X CH oder N darstellt, sowie die Bedeutung von -S-$C_1$-$C_4$-Alkyl, -S-$CH_2COOM$ oder SM hat, wobei M für Wasserstoff oder ein Alkaliion steht;

($C_1$) mit einem Alkohol oder einem Thiol der Formel $R_4$'OH oder $R_4$'SH, wobei $R_4$' die Bedeutungen von $R_4$ hat, zu einer Verbindung der Formel Ia' oder Ia"

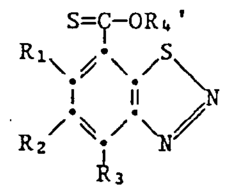

(Ia')

oder

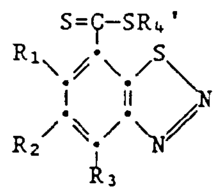

(Ia")

oder

($C_2$) mit einem Oxim der Formel HON=C($R_7$)$R_8$ zu einer Verbindung der Formel Ib

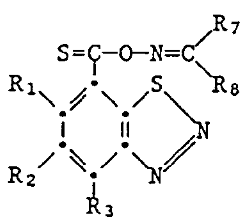

(Ib);

oder

($C_3$) mit einem Amin der Formel HN($R_8$)$R_6$ zu einer Verbindung der Formel Ic

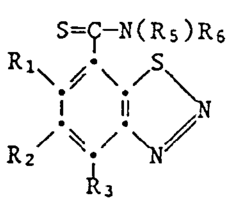

(Ic);

oder

($C_4$) mit einem Hydroxylamin-Derivat der Formel HN($R_7$)-O-$R_{10}$ zu einer Verbindung der Formel Id

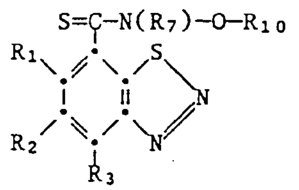

(Id);

oder
($C_5$) mit einem Hydrazin-Derivat der Formel $HN(R_7)-N(R_5)R_6$ zu einer Verbindung der Formel Ie

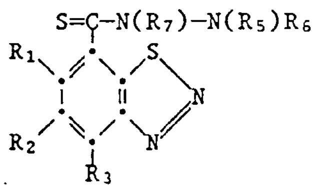

(Ie)

in inerten Lösungsmitteln mit oder ohne Base bei einer Temperatur von -20° bis 170°C;
D) Alkylierung der Dithiosäure oder ihres Salzes der Formel IVa

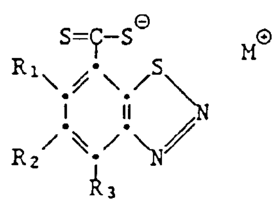

(IVa) .

($M^{\oplus}$ = Wasserstoff oder Alkaliion)
mit einem Alkylierungsmittel der Formel $R_4'$-L, wobei $R_4'$ die Bedeutung von $R_4$ hat und L eine Abgangsgruppe darstellt, in Gegenwart einer Base in inerten Lösungsmitteln bei Temperaturen von -30° bis 110°C zu einer Verbindung der Formel Ia"

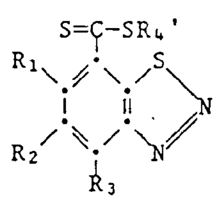

(Ia")

E) Oxidation der Dithiosäure oder ihres Salzes der Formel IVa mit Luftsauerstoff, Kaliumhexacyanoferrat(III) oder $KJ/J_2$ in wässriger Lösung bei Temperaturen von 10° bis 60°C zu einer Verbindung der Formel If

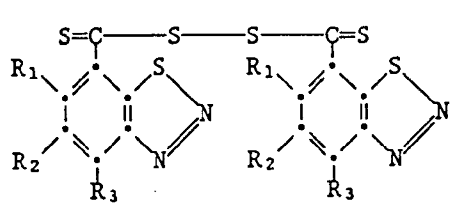

(If),

wobei in den vorstehenden Formeln $R_1$ bis $R_{10}$ die unter Formel I angegebenen Bedeutungen haben.

Für die vorstehend beschriebenen Herstellungsverfahren kommen soweit erforderlich organische und anorganische Basen in Betracht, z.B. tertiäre Amine wie Trialkylamine (Trimethylamin, Triethylamin, Tripropylamin usw.), Pyridinbasen (Pyridin, 4-Dimethylaminopyridin, 4-Pyrrolidylaminopyridin, Collidin), Oxide und Hydroxide, Carbonate und Hydrogencarbonate von Alkali- und Erdalkalimetallen sowie Alkaliacetate.

Als Reaktionsmedien in Anpassung an die jeweiligen Reaktionsbedingungen werden geeignete reaktionsinerte Lösungs- und Verdünnungsmittel verwendet. Als Beispiele sind zu nennen: aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Ketone wie Aceton, Diethylketon, Methylethylketon; sowie Gemische solcher Lösungsmittel untereinander.

Die Verfahren zur Herstellung der Verbindungen der Formel I stellen für den Fachmann bekannte oder in der Literatur beschriebene Methoden dar (vgl. Tetrahedron 41, 5061 (1985); Thio- und Dithiocarboxylic Acids and their Derivatives in A. Senning, Topics in Sulfur Chemistry Bd. 4, 1979; Houben-Weyl E5, Teil 2, S. 790, 796, 906 - 909, 921, 930/931).

Die Herstellung der als Zwischenprodukte für die Verbindungen der Formel I fungierenden Verbindungen der Formel IV kann nach bekannten Methoden folgendermassen durchgeführt werden:

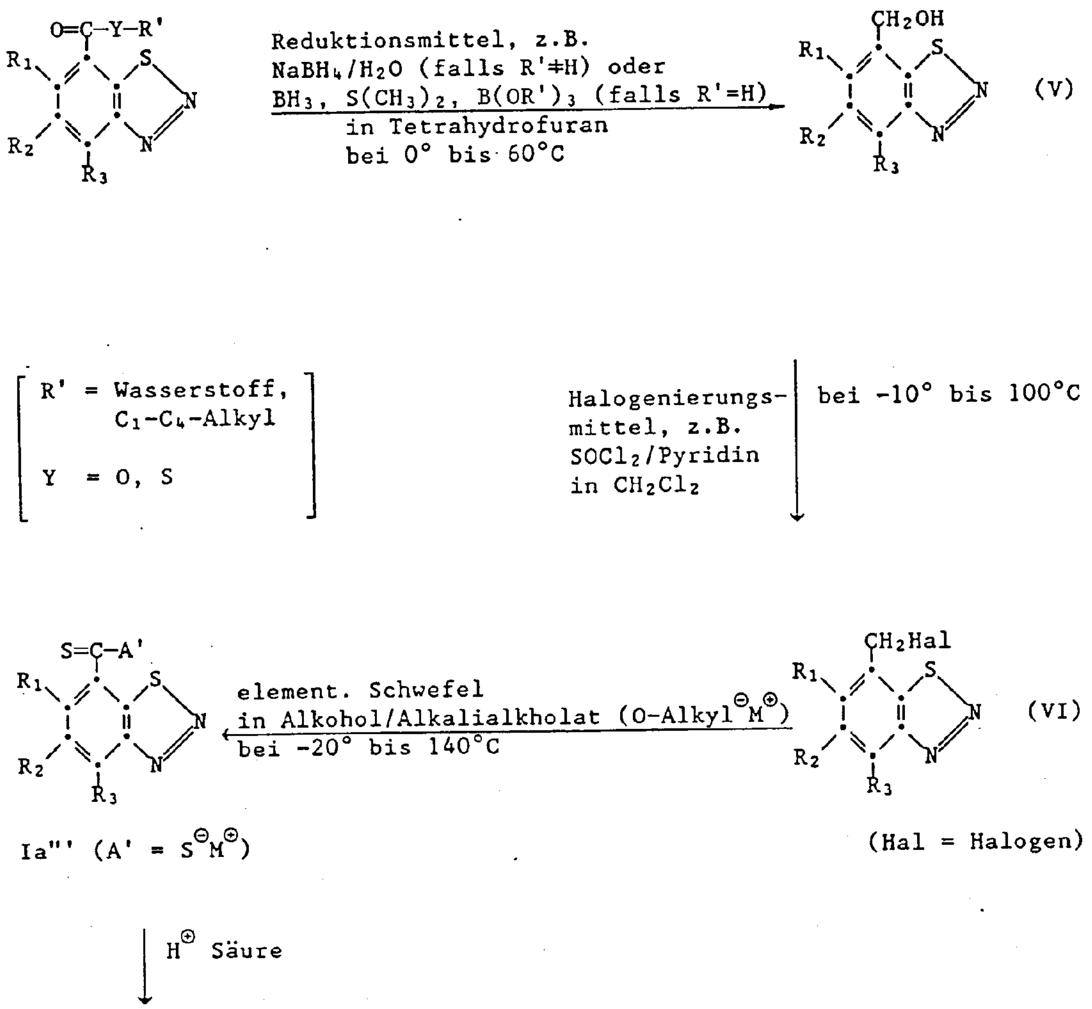

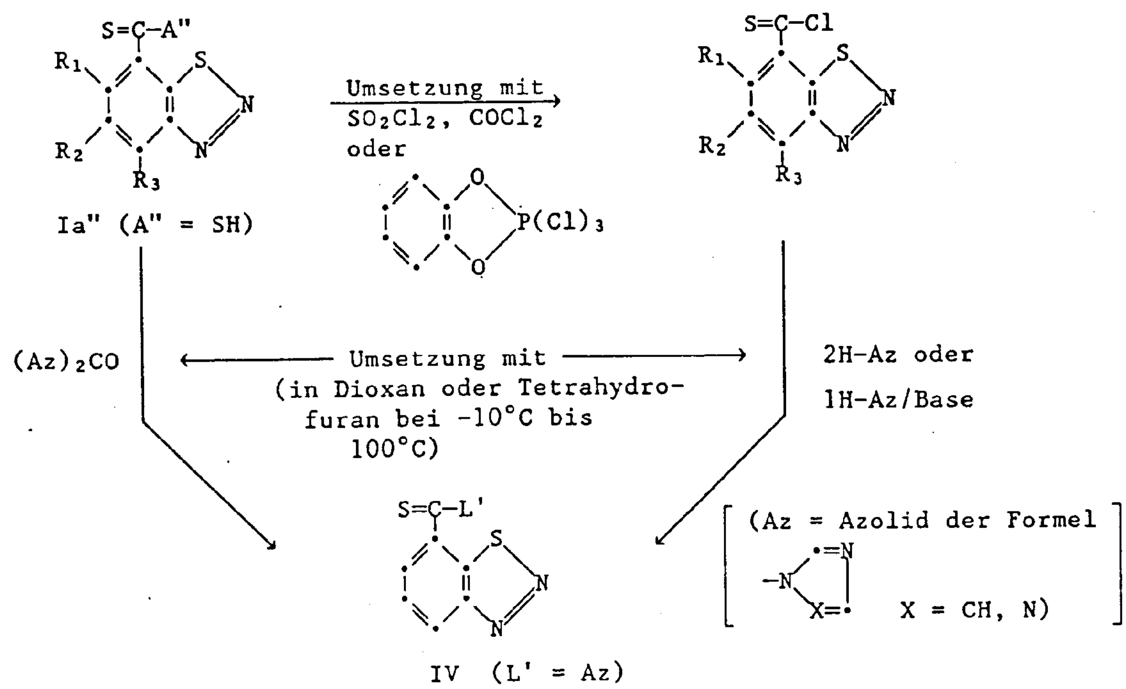


Die vorstehend beschriebenen Herstellungsverfahren stellen für den Fachmann bekannte oder in der Literatur beschriebene Methoden dar (vgl. Chem. Ber. 98,829 (1965); Helv. Chim. Acta 3, 824 (1920); DOS 2 306 543, Ann. Chem. 739,201 (1970); Houben-Weyl E5, 914; EP 313 512).

Die im Rahmen der Erfindung zur Anwendung gelangenden mikrobiziden Mittel zum Schutz von Pflanzen gegen Krankheiten, welche die Verbindungen der Formel I als Aktivstoffe enthalten, sind als Teil der Erfindung zu betrachten.

Wirkstoffe der Formel I werden üblicherweise in Form von Zusammensetzungen verwendet und können gleichzeitig oder nacheinander mit weiteren Wirkstoffen auf die Pflanze oder deren Umgebung gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein Verfahren zur Anwendung eines Wirkstoffes der Formel I bzw. eines agrochemischen Mittels, das mindestens einen dieser Wirkstoffe enthält, ist das Aufbringen auf die Pflanze (Blattapplikation). Die Wirkstoffe der Formel I können aber auch über den Erdboden durch das Wurzelwerk in die Pflanze gelangen (Bodenapplikation), indem man den Standort der Pflanze mit einer flüssigen Zubereitung tränkt oder die Substanzen in fester Form in den Boden einbringt z.B. in Form von Granulat. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet (Beizapplikation). Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt. Zu diesem Zweck werden sie z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt bei 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden hergestellt durch inniges Vermischen und/oder Vermahlen der Wirkstoffe mit Streckmitteln, wie z.B. mit Lösungsmitteln, festen Trägerstoffen, und gegebenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid; sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I nicht-ionogene, kation- und/oder anionaktive Tenside mit guten Emulgier-, Dispergier- und Netzeigenschaften in Betracht. Unter Tensiden sind auch Tensidgemische zu verstehen.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedere, gegebenenfalls halogenierte Alkyl-, Benzyl- oder niedere Hydroxyalkylreste aufweisen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure oder von natürlichen Fettsäuregemischen, die z.B. aus Kokosnuss- oder Talgöl gewonnen werden können, genannt.

Als synthetische Tenside können insbesondere Fettalkoholsulfonate, Fettalkoholsulfate, sulfonierte Benzimidazolderivate oder Alkylsulfonate Verwendung finden. Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Die nachfolgenden Beispiele dienen zur näheren Erläuterung der Erfindung, ohne dieselbe einzuschränken.

1. Herstellungsbeispiele

1.1 Herstellung von Benzo-1,2,3-thiadiazol-7-thiocarbonsäure-O-ethylester

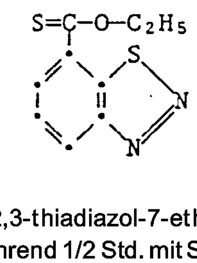

Eine Suspension von 7,8 g Benzo-1,2,3-thiadiazol-7-ethylester-imid-hydrochlorid in 100 ml abs. Toluol wird unter Stickstoffatmosphäre bei 0°C während 1/2 Std. mit Schwefelwasserstoff gesättigt und anschliessend unter weiterem Einleiten von Schwefelwasserstoff innerhalb 3/4 Std. tropfenweise mit einer Losung von 6,1 g Pyridin in 5 ml Toluol versetzt. Nach beendetem Zutropfen wird noch während 2 Std. Schwefelwasserstoff eingeleitet und anschliessend über Nacht weiter bei Raumtemperatur gerührt. Zur Aufarbeitung wird der entstandene Niederschlag abfiltriert, mit Toluol gut gewaschen und das Filtrat am Vakuum eingedampft. Der verbleibende Rückstand wird an Flash-Kieselgel (Hexan/Essigsäureethylester) gereinigt. Es resultiert 6,0 g der Titelverbindung vom Smp. 114 - 115°C.

1.2 Herstellung von Benzo-1,2,3-thiadiazol-7-dithiocarbonsäure--methylester

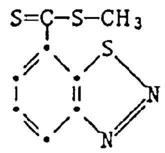

2,10 g Benzo-1,2,3-thiadiazol-7-thiomethylester und 2,4 g Lawesson-Reagens werden in 40 ml Xylol während 28 Std. auf 110°C erwärmt, dann nochmals mit 2,4 g Lawesson-Reagens versetzt und weitere 4 Std. auf 130°C erwärmt. Dann wird das Reaktionsgemisch eingedampft und der Rückstand an Kieselgel (Flash-Chromatographie, Methylenchlorid) gereinigt. Die Titelverbindung wird in einer Ausbeute von 1,9 g mit einem Smp. von 124 - 126°C erhalten.

1.3 Herstellung von Benzo-1,2,3-thiadiazol-7-thiocarbonsäureamid (Verb. 2.1)

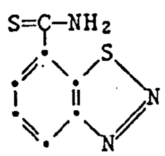

Eine Suspension von 2,42 g p-Methoxyphenylthiono-phosphinsulfid und 1,79 g Benzo-1,2,3-thiadiazol-7-carbonsäureamid in 95 ml Toluol wird während 6 Std. unter Stickstoffatmosphäre am Rückfluss gekocht. Dann wird unter Vakuum eingedampft, der Rückstand mit Essigsäureethylester aufgeschlämmt und filtriert. Der Niederschlag wird aus Essigsäureethylester/Tetrahydrofuran unter Zusatz von Aktivkohle umkristallisiert. Es resultieren 1,1 g gelbe Kristalle vom Smp. 129 - 131°C.

1.4 Herstellung von 7-Benzo-1,2,3-thiadiazol-N',N'-dimethylthiohydrazid (Verb. 3.1)

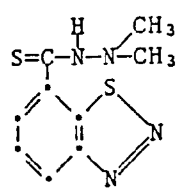

4,52 g Benzo-1,2,3-thiadiazol-7-dithiocarbonsäuremethylester in 30 ml Tetrahydrofuran werden unter Rühren bei Raumtemperatur tropfenweise mit 3,2 g N,N-Dimethylhydrazin versetzt. Nach kurzer Zeit beginnt die Abspaltung von Methylmercaptan unter Abscheidung eines beigen Niederschlages. Ueber Nacht wird auf 50°C erwärmt, anderntags der Niederschlag abfiltriert und mit Methylenchlorid gewaschen. Nach Trocknen verbleiben 2,2 g der Titelverbindung vom Smp. 195 - 197°C.

1.5 Herstellung von 7-Carboxymethylthio-thiono-benz-1,2,3-thiadiazol (Verb. 1.45)

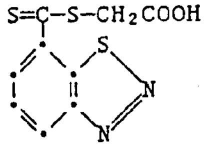

Zu einer Suspension von 1 g fein verriebenem Schwefel in 8 ml Methanol sowie 6,4 ml

Natriummethylatlösung (30 %-ig in Methanol) werden bei Raumtemperatur innerhalb 15 Min. portionenweise 2,9 g festes 7-Chlormethylbenz-1,2,3-thiadiazol eingetragen, wobei die Temperatur auf 45°C ansteigt. Anschliessend wird die dunkle Suspension aufgeheizt und während 16 Stdn. bei 90°C gerührt. Dann wird auf 0°C abgekühlt, vom Natriumchlorid abfiltriert und das Filtrat eingedampft. Der Rückstand wird in 14 ml Wasser gelöst, bei 0°C mit 1,9 g festem Natriumchloracetat versetzt und während 18 Stdn. bei Raumtemperatur weitergerührt. Zur Aufarbeitung wird die Lösung ferner mit Methylenchlorid und Wasser versetzt, die organische Phase abgetrennt und diese nochmals mit Wasser extrahiert. Die wässrigen Extrakte werden abgekühlt und bei 0°C mit konz. Salzsäure angesäuert. Der ausgefallene Niederschlag wird abfiltriert, mit Wasser gewaschen und aus Ethanol umkristallisiert, wobei die Titelverbindung mit einem Smp. von 207-209°C resultiert.

1.6 Herstellung von Benzo-1,2,3-thiadiazol-7-ethylester-imid-hydrochlorid (Verb. 5.2) (Zwischenprodukt)

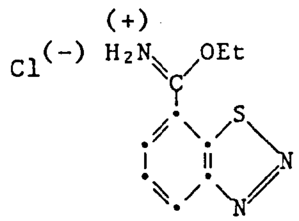

7.2 g 7-Cyano-benzo-1,2,3-thiadiazol werden in 60 ml abs. Ethanol und 20 ml Tetrahydrofuran bei 45°C gelöst, auf 0°C abgekühlt und unter Feuchtigkeits-Ausschluss bei 0° bis 15°C mit gasförmiger Salzsäure innerhalb 1 Std. gesättigt. Dann wird das Reaktionsgemisch während 24 Std. im Kühlschrank aufbewahrt, mit 70 ml abs. Diethylether versetzt und weitere 4 Tage im Kühlschrank stehen gelassen. Dann wird abfiltriert, mit Diethylether gewaschen und getrocknet. Es werden 9,4 g der Verbindung 5.2 als beige Kristalle vom Smp. 270 - 272°C erhalten.

1.7 Herstellung von 7-Hydroxymethyl-benzo-1,2,3-thiadiazol (Verb. 6.1) (Zwischenprodukt)

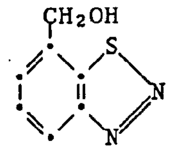

Zu einer Suspension von 54 g Carboxybenz-1,2,3-thiadiazol in 420 ml Tetrahydrofuran werden unter Stickstoffatmosphäre und Rühren bei Raumtemperatur 110 ml Triethylborat zugetropft, 1 Std. weitergerührt und anschliessend unter leichtem Kühlen tropfenweise mit 45,6 ml Borandimethylsulfid-Komplex in 60 ml Tetrahydrofuran versetzt, wobei heftige Gasentwicklung beobachtet wird. Nach Rühren über Nacht und Stehenlassen bei Raumtemperatur wird wieder auf 5-10°C abgekühlt und unter Rühren und starkem Kühlen tropfenweise mit 200 ml Methanol versetzt, wobei wiederum starke Gasentwicklung auftritt. Anschliessend wird unter Vakuum eingedampft, nochmals 300 ml Methanol zugegeben und wieder eingedampft. Der Rückstand wird an Kieselgel (Lösungsmittel: Essigsäureethylester/Hexan) gereinigt und das erhaltene Produkt aus Essigsäureethylester/Hexan umkristallisiert. Die erhaltene Titelverbindung schmilzt bei 79-81°C.

Verbindungen der Formel V (Tabelle 6) können auch durch Reduktion entsprechender Ester mit überschüssigem Natriumborhydrid in Gegenwart von Wasser in Tetrahydrofuran oder Dioxan bei Raumtemperatur oder bei erhöhter Temperatur (20-100°C) erhalten werden.

1.8 Herstellung von 6-Chlor-7-hydroxymethyl-benzo-1,2,3-thiadiazol (Verb. 6.1) (Zwischenprodukt)

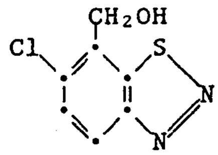

Zu einer Suspension von 4,5 g Natriumborhydrid in 30 ml Wasser und 70 ml Tetrahydrofuran wird unter Rühren und Erwärmen auf 40-50°C innerhalb einer halben Stunde eine Lösung von 1,9 g 6-Chlor-7-carbomethoxybenzo-1,2,3-thiadiazol in 30 ml Tetrahydrofuran (warm gelöst) zugetropft. Bis zur völligen Umsetzung wird weiter erwärmt, mit einem $CO_2$-Kühlbad auf -20° bis -30°C abgekühlt und tropfenweise mit 15 ml Aceton versetzt, wobei starke Gasentwicklung beobachtet wird. Die Reaktionsmischung wird anschliessend mit 15%iger Salzsäure unter weiterer kräftiger Kühlung bei -20°C bis -10°C auf pH 3 angesäuert (Gasentwicklung) und über Nacht weitergerührt. Dann wird der grösste Teil des Tetrahydrofurans am Rotationsverdampfer abgezogen und der Rückstand mit Essigsäureethylester extrahiert. Die Extrakte werden mit Wasser gewaschen, über Natriumsulfat getrocknet und eingedampft. Der Rückstand liefert die Titelverbindung vom Smp. 138-140°C.

1.9 Herstellung von 7-Chlormethyl-benzo-1,2,3-thiadiazol (Verb. 7.1) (Zwischenprodukt der Formel VI)

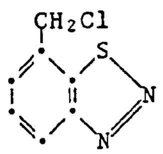

199,2 g der Verbindung von Beispiel 1.7 werden in 150 ml Methylenchlorid und 150 ml Pyridin bei 5°C gelöst, vorgelegt und unter Kühlen bei 5-10°C innerhalb 3/4 Stdn. mit einer Lösung von 120 ml Thionylchlorid in 200 ml Methylenchlorid versetzt. Dann wird über Nacht bei Raumtemperatur weitergerührt, die Suspension auf Eiswasser gegossen und mit Methylenchlorid extrahiert. Die Extrakte werden mit Eiswasser gewaschen, über Natriumsulfat getrocknet, filtriert und eingedampft. Die so erhaltene Titelverbindung schmilzt bei 78-80°C.

In den Verbindungstabellen werden für folgende Reste die dazugehörigen Symbole verwendet:

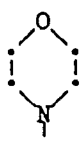
$Q_{11}$

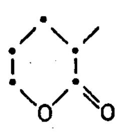
$Q_{12}$

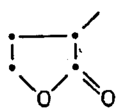
$Q_{13}$

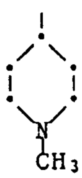
$Q_{14}$

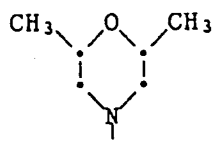
$Q_{15}$

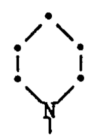
$Q_{16}$

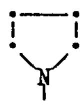
$Q_{17}$

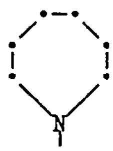
$Q_{18}$

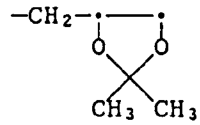
$Q_{19}$

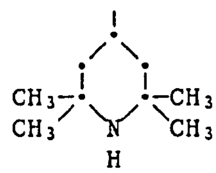
$Q_{20}$

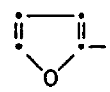
$Q_{21}$

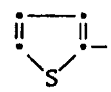
$Q_{22}$

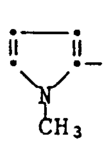
$Q_{23}$

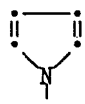
$Q_{24}$

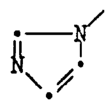
$Q_{31}$

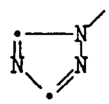
$Q_{32}$

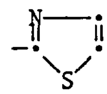
$Q_{33}$

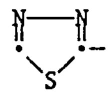
$Q_{34}$

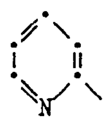
$Q_{41}$

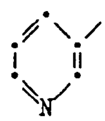
$Q_{42}$

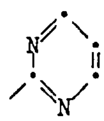
$Q_{43}$

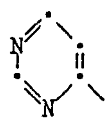
$Q_{44}$

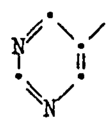
$Q_{45}$

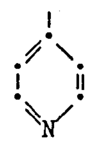
$Q_{46}$

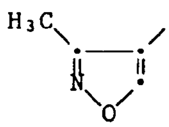
$Q_{47}$

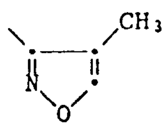
$Q_{48}$

Die vorstehend beschriebenen Heterocyclen können durch niedermolekulare Reste, wie aliphatische Reste mit bis zu 4 C-Atomen, oder durch Halogenatome oder andere Reste substituiert sein.

Tabelle 1

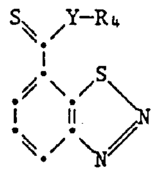

| Verbindung Nr. | Y | $R_4$ | physik. Daten |
|---|---|---|---|
| 1.1 | O | Methyl | Smp. 105 - 108°C |
| 1.2 | O | Ethyl | Smp. 114 - 115°C |
| 1.3 | O | i-Propyl | |
| 1.4 | O | n-Butyl | |
| 1.5 | O | n-Hexyl | |
| 1.6 | S | Methyl | Smp. 124 - 126°C |
| 1.7 | S | Ethyl | Smp. 130 - 132°C |
| 1.8 | S | n-Propyl | Smp. 92 - 94°C |
| 1.9 | S | i-Propyl | |
| 1.10 | S | n-Butyl | |
| 1.11 | S | n-Pentyl | |
| 1.12 | S | t-Butyl | |
| 1.13 | O | Cyclopentyl | |
| 1.14 | O | Cyclohexyl | |
| 1.15 | S | Cyclopentyl | Smp. 117 - 119°C |
| 1.16 | S | Cyclohexyl | |
| 1.17 | O | Cyclobutyl | |
| 1.18 | S | Benzyl | |
| 1.19 | O | Benzyl | Smp. 79-81°C |
| 1.20 | O | H | |
| 1.21 | S | H | |
| 1.22 | O | $CH_2$-Cyclopropyl | Smp. 73-75°C |
| 1.23 | S | $(CH_2)_2$-Phenyl | |
| 1.24 | S | $CH_2CH_2CH_2$-Phenyl | |
| 1.25 | S | $CH_2CH_2O$-Phenyl | |
| 1.26 | S | 4-Phenoxyphenyl | |
| 1.27 | S | 4-Biphenyl | |
| 1.28 | S | 1-Naphthyl | |
| 1.29 | S | 2-Naphthyl | |
| 1.30 | O | $CH_2CH_2O$-Phenyl | |
| 1.31 | O | 4-Phenoxyphenyl | Smp. 75-78°C |
| 1.32 | O | 1-Naphthyl | |
| 1.33 | S | Allyl | |
| 1.34 | S | Propargyl | |
| 1.35 | S | 2-Carboxyphenyl | |
| 1.36 | S | n-Octadecyl | |
| 1.37 | O | n-Triacontanyl | |
| 1.38 | S | $CH_2CH_2OCH_3$ | |
| 1.39 | O | $CH_2CH_2SCH_3$ | |
| 1.40 | S | $CH_2$-Naphth-1-yl | |
| 1.41 | O | 4-Chlorbenzyl | Smp. 116-118°C |
| 1.42 | O | $(CH_2)_2$-O-$(CH_2)_2$-O-Phenyl | |
| 1.43 | S | $(CH_2)_2$-O-$(CH_2)_2$-O-$Q_{46}$ | |
| 1.44 | O | Carboxymethyl | |

Tabelle 1 (Fortsetzung)

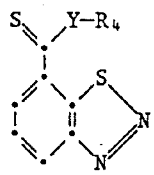

| Verbindung Nr. | Y | $R_4$ | physik. Daten |
|---|---|---|---|
| 1.45 | S | Carboxymethyl | Smp. 207-209°C |
| 1.46 | S | 2-Butenyl | |
| 1.47 | S | 4-Hexenyl | |
| 1.48 | S | Phenyl | |
| 1.49 | O | Phenyl | |
| 1.50 | O | 2-Chlorphenyl | |
| 1.51 | S | 4-Chlorphenyl | |
| 1.52 | O | 2-Methoxyphenyl | |
| 1.53 | O | 4-i-Propylphenyl | |
| 1.54 | S | Fluormethyl | |
| 1.55 | S | $CH_2CH_2-O-Q_{11}$ | |
| 1.56 | O | $Q_{12}$ | |
| 1.57 | S | $Q_{13}$ | |
| 1.58 | S | $Q_{14}$ | |
| 1.59 | O | $CH_2-Q_{21}$ | |
| 1.60 | S | $CH_2-Q_{22}$ | |
| 1.61 | S | $Q_{20}$ | |
| 1.62 | S | $CH_2-Q_{23}$ | |
| 1.63 | S | $Q_{24}$ | |
| 1.64 | S | $Q_{12}$ | |
| 1.65 | S | $CH_2-Q_{31}$ | |
| 1.66 | S | $CH_2-Q_{32}$ | |
| 1.67 | O | $Q_{41}$ | |
| 1.68 | O | $CH_2-Q_{42}$ | |
| 1.69 | O | $CH_2-Q_{45}$ | |
| 1.70 | S | $Q_{44}$ | |
| 1.71 | O | $Q_{46}$ | |
| 1.72 | S | $CH_2-Q_{46}$ | |
| 1.73 | S | Cycloheptyl | |
| 1.74 | O | $N=C(CH_3)_2$ | |
| 1.75 | O | $N=C(CH_3)$Ethyl | |
| 1.76 | O | $NH_2$ | |
| 1.77 | O | $N(Methyl)_2$ | |
| 1.78 | S | $N(Methyl)_2$ | |
| 1.79 | O | 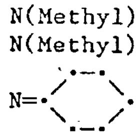 | |
| 1.80 | O | 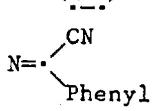 | |

Tabelle 1 (Fortsetzung)

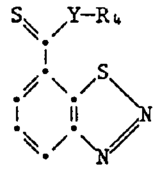

| Verbindung Nr. | Y | $R_4$ | physik. Daten |
|---|---|---|---|
| 1.81 | O | 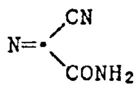 | |
| 1.82 | O | 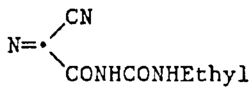 | |
| 1.83 | S | 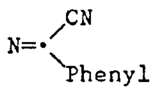 | |
| 1.84 | S | 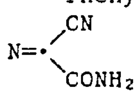 | |
| 1.85 | S | 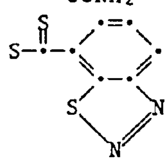 | Zers. ab 210°C |
| 1.86 | O | Cycloheptyl | |
| 1.87 | S | 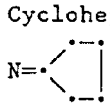 | |
| 1.88 | S | $Na^{\oplus}$ | |
| 1.89 | S | $K^{\oplus}$ | |
| 1.90 | S | $Li^{\oplus}$ | |
| 1.91 | S | 1/2 $Ca^{2\oplus}$ | |
| 1.92 | S | 1/2 $Mg^{2\oplus}$ | |
| 1.93 | S | $HN(Ethyl)_3^{\oplus}$ | |
| 1.94 | S | $H_2N(i\text{-}Propyl)_2^{\oplus}$ | Harz |
| 1.95 | S | $NH_4^{\oplus}$ | |
| 1.96 | S | $H\text{-}Q_{16}^{\oplus}$ | |
| 1.97 | S | $H\text{-}Q_{15}^{\oplus}$ | |
| 1.98 | S | $1/2(Ba)_2^{\oplus}$ | |
| 1.99 | O | $Na^{\oplus}$ | |
| 1.100 | O | $HN(Ethyl)_3^{\oplus}$ | Harz |
| 1.101 | S | $CH_2COOMethyl$ | Smp. 123 - 125°C |

Tabelle 2

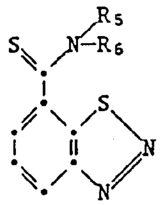

| Verbindung Nr. | $N(R_5)R_6$ | physik. Daten |
|---|---|---|
| 2.1 | $NH_2$ | Smp. 129-131°C |
| 2.2 | NH-Methyl | Smp. 210-212°C |
| 2.3 | $N(Methyl)_2$ | Smp. 118-121°C |
| 2.4 | NH-Ethyl | |
| 2.5 | NH-Phenyl | |
| 2.6 | $NH-CH_2-CN$ | |
| 2.7 | $N(CH_2CN)_2$ | |
| 2.8 | $NH-CH_2COOMethyl$ | |
| 2.9 | $NHCH_2C{\equiv}CH$ | |
| 2.10 | $NHCH_2CH{=}CH_2$ | |
| 2.11 | $N(CH_2CH{=}CH_2)_2$ | Smp. 82-84°C |
| 2.12 | $NHCH_2CH_2CN$ | |
| 2.13 | NH-CH(OEthyl)CN | |
| 2.14 | $Q_{15}$ | |
| 2.15 | $Q_{16}$ | Smp. 119 - 122°C |
| 2.16 | $Q_{17}$ | |
| 2.17 | $Q_{18}$ | |
| 2.18 | $NH-Q_{12}$ | |
| 2.19 | $Q_{31}$ | |
| 2.20 | $Q_{32}$ | |
| 2.21 | $NH-Q_{33}$ | |
| 2.22 | $NH-Q_{34}$ | |
| 2.23 | $NH-Q_{41}$ | |
| 2.24 | $NH-Q_{42}$ | |
| 2.25 | $NH-Q_{43}$ | |
| 2.26 | $NH-Q_{44}$ | |
| 2.27 | $N(CH_3)-Q_{45}$ | |
| 2.28 | $NH-Q_{46}$ | |
| 2.29 | $NH-Q_{47}$ | |
| 2.30 | $NH-Q_{48}$ | |
| 2.31 | $NH-Q_{12}$ | |
| 2.32 | $NHCH_2-Q_{21}$ | Smp. 174-176°C |
| 2.33 | $NHCH_2$-Phenyl | |
| 2.34 | $N(CH_2Phenyl)_2$ | |
| 2.35 | NH(3,5-Dichlorphenyl) | |
| 2.36 | NH(2-Methylphenyl) | |
| 2.37 | NHOH | Zers. 133°C |
| 2.38 | N(Methyl)-O-Methyl | |
| 2.39 | NH-O-Cyclohexyl | |
| 2.40 | NH-O-n-Octyl | |
| 2.41 | N(O-Alkyl)-O-Allyl | |
| 2.42 | NH-O-Phenyl | |
| 2.43 | $Q_{11}$ | Smp. 178 - 180°C |

Tabelle 3

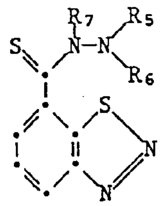

| Verb. Nr. | $R_7$ | $R_5$ | $R_6$ | physik. Daten |
|---|---|---|---|---|
| 3.1 | H | Methyl | Methyl | Smp. 195-197°C |
| 3.2 | H | H | H | |
| 3.3 | H | Methyl | H | |
| 3.4 | Methyl | Methyl | Methyl | |
| 3.5 | Ethyl | Phenyl | H | |
| 3.6* | H | Phenyl | H | Smp. 110-111°C |
| 3.7 | H | Benzyl | Benzyl | |
| 3.8 | H | Methyl | Phenyl | |
| 3.9 | H | Phenyl | Phenyl | |
| 3.10 | H | 1-Naphthyl | H | |
| 3.11 | H | H | Benzyl | |
| 3.12 | H | $-CH_2CH_2CH_2CH_2-$ | | |
| 3.13 | H | $-CH_2CH_2CH_2CH_2CH_2-$ | | Smp. 166-168°C |
| 3.14 | Me | $-CH_2CH_2CH_2CH_2CH_2CH_2-$ | | |
| 3.15 | H | 2-Fluorphenyl | H | |
| 3.16 | H | 4-Methylphenyl | H | |
| 3.17 | H | 4-Chlorbenzyl | H | |
| 3.18 | H | $Q_{41}$ | H | |
| 3.19 | H | $Q_{43}$ | H | |
| 3.20 | Ethyl | $Q_{41}$ | Ethyl | |
| 3.21 | H | $Q_{33}$ | H | |
| 3.22 | H | 2-Naphthyl | H | |

* in Form des Tetrabutylammoniumsalzes

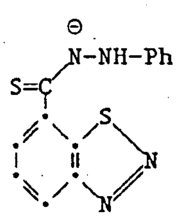

$(n\text{-Butyl})_4N^{\oplus}$

Tabelle 4

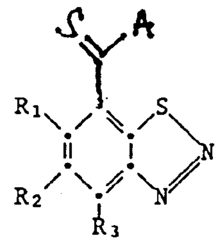

| Verbindung Nr. | $R_1$, $R_2$, $R_3$ | A | physik. Daten |
|---|---|---|---|
| 4.1 | 5-Fluor | $NH-NH_2$ | |
| 4.2 | 5-Fluor | $NH-N(Methyl)_2$ | |
| 4.3 | 5-Fluor | $N(Methyl)-N(Methyl)_2$ | |
| 4.4 | 5-Fluor | S-n-Propyl | |
| 4.5 | 5-Fluor | S-Methyl | Smp. 122-124°C |
| 4.6 | 5-Fluor | S-Benzyl | |
| 4.7 | 5-Fluor | S-Phenyl | |
| 4.8 | 5-Fluor | $S-CH_2-Q_{21}$ | |
| 4.9 | 5-Fluor | S-Cyclopentyl | |
| 4.10 | 5-Fluor | S-Cyclohexyl | |
| 4.11 | 5-Fluor | SK | |
| 4.12 | 5-Fluor | SH | |
| 4.13 | 5-Fluor | $SH \cdot NH_3$ | |
| 4.14 | 5-Fluor | NHOH | |
| 4.15 | 5-Fluor | N(Methyl)O-Methyl | |
| 4.16 | 5-Fluor | 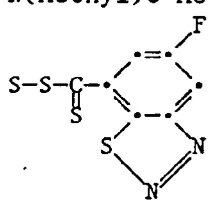 | |
| 4.17 | 5-Fluor | O-Methyl | Smp. 104-107°C |
| 4.18 | 5-Fluor | O-i-Propyl | |
| 4.19 | 5-Fluor | O-Benzyl | |
| 4.20 | 5-Fluor | OH | |
| 4.21 | 5-Fluor | $O-N=C(CN)CONH_2$ | |
| 4.22 | 5-Fluor | $S-Q_{13}$ | |
| 4.23 | 6-Fluor | O-Methyl | |
| 4.24 | 6-Fluor | S-Methyl | |
| 4.25 | 6-Fluor | S-Ethyl | |
| 4.26 | 6-Fluor | S-Phenyl | |
| 4.27 | 6-Fluor | $NH_2$ | |
| 4.28 | 6-Fluor | NH-Methyl | |
| 4.29 | 6-Fluor | NH-Phenyl | |
| 4.30 | 6-Fluor | $NH-CH_2-Q_{21}$ | |
| 4.31 | 6-Fluor | N(Methyl)-O-methyl | |
| 4.32 | 6-Fluor | S-n-propyl | |
| 4.33 | 6-Fluor | S-Benzyl | |
| 4.34 | 6-Fluor | O-Benzyl | |
| 4.35 | 6-Fluor | O-4-Methoxybenzyl | |
| 4.36 | 6-Fluor | S-4-Phenoxyphenyl | |
| 4.37 | 6-Fluor | SH | |

Tabelle 4 (Fortsetzung)

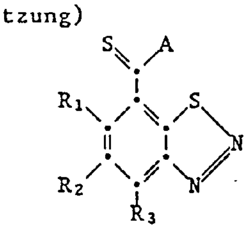

| Verbindung Nr. | $R_1$, $R_2$, $R_3$ | A | physik. Daten |
|---|---|---|---|
| 4.38 | 6-Fluor | SNa | |
| 4.39 | 6-Fluor | $SH \cdot N(Ethyl)_3$ | |
| 4.40 | 6-Fluor | 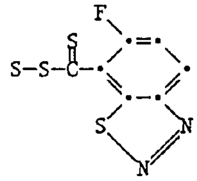 | |
| 4.41 | 6-Fluor | $NH-NH_2$ | |
| 4.42 | 6-Fluor | $NH-N(Methyl)_2$ | |
| 4.43 | 5-Fluor | $NH_2$ | |
| 4.44 | 5-Fluor | $Q_{16}$ | |
| 4.45 | 5-Fluor | $NH-Q_{45}$ | |
| 4.46 | 5-Fluor | $NH-Q_{43}$ | |
| 4.47 | 5-Fluor | $NH-Q_{48}$ | |
| 4.48 | 5-Fluor | $NH-Q_{33}$ | |
| 4.49 | 5-Fluor | NH-OH | |
| 4.50 | 4-Fluor | O-Methyl | |
| 4.51 | 4-Fluor | S-Methyl | |
| 4.52 | 4,6-Difluor | O-Ethyl | |
| 4.53 | 4,6-Difluor | O-Benzyl | |
| 4.54 | 4,6-Difluor | S-Phenyl | |
| 4.55 | 4,6-Difluor | OH | |
| 4.56 | 4,6-Difluor | $NH_2$ | |
| 4.57 | 4,6-Difluor | OLi | |
| 4.58 | 4,5,6-Trifluor | O-Methyl | |
| 4.59 | 4,5,6-Trifluor | S-Methyl | |
| 4.60 | 4,5,6-Trifluor | OH | |
| 4.61 | 4,5,6-Trifluor | 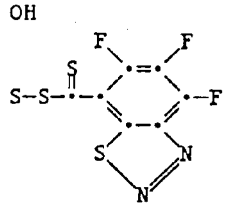 | |
| 4.62 | 6-Chlor | O-Methyl | |
| 4.63 | 5-Chlor | S-Methyl | |
| 4.64 | 4-Brom | O-Methyl | |
| 4.65 | 6-Fluor | $NH-CH_2CN$ | |
| 4.66 | 6-Fluor | NH-CH(OEthyl)CN | |
| 4.67 | 5-Fluor | $Q_{11}$ | |
| 4.68 | 6-Fluor | $Q_{11}$ | |

Tabelle 5

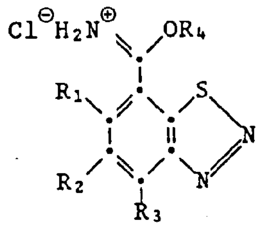

| Verbindung Nr. | $R_1$, $R_2$, $R_3$ | $R_4$ | physik. Daten |
|---|---|---|---|
| 5.1 | H | Methyl | |
| 5.2 | H | Ethyl | Smp. 270-272°C |
| 5.3 | H | n-Propyl | |
| 5.4 | H | i-Propyl | |
| 5.5 | 5-F | n-Butyl | |
| 5.6 | 4,6-Di-F | sec.-Butyl | |
| 5.7 | 6-F | n-Octyl | |
| 5.8 | H | n-Dodecyl | |
| 5.9 | H | Cyclopentyl | |
| 5.10 | 6-F | Cyclohexyl | |
| 5.11 | 5-F | Methyl | |
| 5.12 | 5-F | Ethyl | |
| 5.13 | 6-F | Ethyl | |
| 5.14 | 4,5,6-Tri-F | n-Propyl | |
| 5.15 | 5-Br | n-Butyl | |
| 5.16 | 6-Cl | Methyl | |
| 5.17 | 4-F | Methyl | |

Tabelle 6

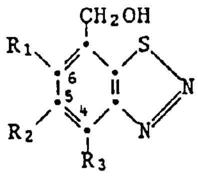

| Verbindung Nr. | $R_1$, $R_2$, $R_3$ | physik. Daten |
|---|---|---|
| 6.1 | H | Smp. 79-81°C |
| 6.2 | 5-F | |
| 6.3 | 5-Cl | |
| 6.4 | 5-Br | |
| 6.5 | 6-F | |
| 6.6 | 6-Cl | Smp. 138-140°C |
| 6.7 | 6-Br | |
| 6.8 | 6-J | |
| 6.9 | 5-J | |
| 6.10 | 4-F | |
| 6.11 | 5,6-Di-F | |
| 6.12 | 4,5-Di-F | |
| 6.13 | 4,6-Di-Cl | |
| 6.14 | 6-Methoxy | |
| 6.15 | 5-Methylthio | |
| 6.16 | 6-Methylthio | |
| 6.17 | 4-Methoxy | |
| 6.18 | 4-Methyl | |

Tabelle 7

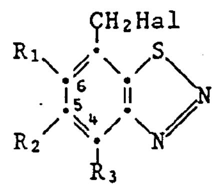

| Verbindung Nr. | $R_1$, $R_2$, $R_3$ | Hal | physik. Daten |
|---|---|---|---|
| 7.1 | H | Cl | Smp. 78-80°C |
| 7.2 | H | Br | |
| 7.3 | H | J | |
| 7.4 | 6-F | Cl | |
| 7.5 | 6-Cl | Cl | |
| 7.6 | 6-Br | Br | |
| 7.7 | 6-J | Cl | |
| 7.8 | 5-F | Cl | |
| 7.9 | 4,5-Di-F | Cl | |
| 7.10 | 5-F | Br | |
| 7.11 | 4-F | Cl | |
| 7.12 | 4,6-Di-Cl | Cl | |
| 7.13 | 5,6-Di-F | Cl | |

Formulierungsbeispiele für Wirkstoffe aus den Tabellen (% = Gewichtsprozent)

| 2.1 Spritzpulver | a) | b) | c) |
|---|---|---|---|
| Wirkstoff aus den Tabellen | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

| 2.2 Emulsions-Konzentrat | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.3 Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

| 2.4 Extruder Granulat | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.5 Umhüllungs-Granulat | |
|---|---|
| Wirkstoff aus den Tabellen | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.6 Suspensions-Konzentrat

| | | |
|---|---|---|
| Wirkstoff aus den Tabellen | 40 | % |
| Ethylenglykol | 10 | % |
| Nonylphenolpolyethylenglykolether (15 Mol Ethylenoxid) | 6 | % |
| Na-Ligninsulfonat | 10 | % |
| Carboxymethylcellulose | 1 | % |
| 37%ige wässrige Formaldehyd-Lösung | 0,2 | % |
| Silikonöl in Form einer 75%igen wässrigen Emulsion | 0,8 | % |
| Wasser | 32 | % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Konzentration hergestellt werden können.

3. Biologische Beispiele

Beispiel 3.1: Wirkung gegen Colletotrichum lagenarium auf Cucumis sativus L.

a) Gurkenpflanzen werden nach 2-wöchiger Anzucht mit einer aus Spritzpulver des Wirkstoffes hergestellten Spritzbrühe besprüht (Konzentration: 200 ppm). Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23°C inkubiert. Die Inkubation wird dann bei normaler Luftfeuchtigkeit und bei 22°C bis 23°C weitergeführt.

Die Beurteilung der Schutzwirkung erfolgt aufgrund des Pilzbefalls 7-8 Tage nach der Infektion.

b) Gurkenpflanzen werden nach 2-wöchiger Anzucht mit einer aus Spritzpulver des Wirkstoffes hergestellten Spritzbrühe durch Bodenapplikation behandelt (Konzentration: 60 oder 20 ppm bezogen auf das Bodenvolumen). Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23°C inkubiert. Die Inkubation wird dann bei normaler Luftfeuchtigkeit und bei 22°C weitergeführt.

Die Beurteilung der Schutzwirkung erfolgt aufgrund des Pilzbefalls 7-8 Tage nach der Infektion.

c) Gurkenpflanzen werden nach 2-wöchiger Anzucht mit einer aus Spritzpulver des Wirkstoffes hergestellten Spritzbrühe besprüht (Konzentration: 200 ppm).

Nach 3 Wochen werden die Pflanzen mit einer Sporensuspension $1.5 \cdot 10^5$ Sporen/ml) des Pilzes infiziert und für 36 Stunden bei hoher Luftfeuchtigkeit und einer Temperatur von 23°C inkubiert. Die Inkubation wird dann bei normaler Luftfeuchigkeit und bei 22° bis 23°C weitergeführt.

Die Beurteilung der Schutzwirkung erfolgt aufgrund des Pilzbefalls 7-8 Tage nach der Infektion.

Verbindungen aus den Tabellen 1 bis 7 zeigen in den Tests (a) und (b) gute Wirkung. So reduzierten z.B. die Verbindungen 1.1, 1.6, 1.7, 1.8, 1.15, 1.101, 2.1, 2.11, 2.16, 3.6, 5.2, 6.1 und 7.1 den Pilzbefall auf 0 bis 20 %. Unbehandelte jedoch infizierte Kontrollpflanzen weisen dagegen einen Colletotrichum-Befall von 100 % auf.

Beispiel 3.2: Wirkung gegen Puccinia graminis auf Weizen

a) Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

b) Zu Weizenpflanzen wird 5 Tage nach Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

Verbindungen aus den Tabellen 1 bis 7 zeigen gegen Puccinia-Pilze eine gute Wirkung. So reduzierten z.B. die Verbindungen 1.1, 1.2, 1.6, 1.8, 1.101, 2.11, 2.16 und 5.2 den Pilzbefall auf 0 bis 20 %. Unbehandelte jedoch infizierte Kontrollpflanzen weisen dagegen einen Puccinia-Befall von 100 % auf.

Beispiel 3.3: Wirkung gegen Phytophthora infestans auf Tomatenpflanzen

a) Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.
b) Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

Verbindungen aus den Tabellen 1 bis 7 zeigen gegen den Phytophthora-Pilz eine gute Schutzwirkung. So reduzierten z.B. die Verbindungen 1.1, 1.2, 1.101, 2.1, 2.11, 2.16, 5.2 und 6.1 den Pilzbefall auf 0 bis 20 %. Unbehandelte jedoch infizierte Kontrollpflanzen weisen dagegen einen 100 %igen Phytophthora-Befall auf.

Beispiel 3.4: Wirkung gegen Pyricularia oryzae auf Reispflanzen

a) Reispflanzen werden nach 2-wöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24°C wird der Pilzbefall beurteilt.
b) Zu 2-wöchigen Reispflanzen wird eine aus Spritzpulver des Wirkstoffes hergestellte Spritzbrühe gegossen (0,006% Aktivsubstanz bezogen auf das Erdvolumen). Darauf werden die Töpfe mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95 - 100 % relativer Luftfeuchtigkeit und ca. 24°C wird der Pilzbefall beurteilt.

Reispflanzen, die mit einer Spritzbrühe behandelt werden, die als Aktivsubstanz eine Verbindung aus den Tabellen 1 bis 7 enthält, zeigen im Vergleich zu unbehandelten Kontrollpflanzen (100 % Befall) nur geringen Pilzbefall. So reduzierten z.B. im Test (a) die Verbindungen 1.1, 1.2, 1.7, 1.8, 2.1, 2.16, 5.2, 6.1 und 7.1 und im Test (b) die Verbindungen 1.1, 1.6, 1.7, 2.11, 2.16, 3.1 und 6.1 den Befall auf 0 bis 20 %.

Beispiel 3.5: Wirkung gegen Peronospora tabacina an Tabakpflanzen

A) Blattapplikation

Tabakpflanzen (8 Wochen alt) werden mit einer formulierten Lösung des Wirkungsstoffes besprüht (Konzentration: 0,02 % Aktivsubstanz). Vier Tage nach der Behandlung werden die Pflanzen mit einer Sporangiensuspension von Peronospora tabacina ($10^4$ Sporangien/ml) inokuliert, 20 Stunden im Dunkeln bei 25°C und hoher Luftfeuchtigkeit aufbewahrt und dann bei normaler Tag/Nacht Wechselfolge weiterinkubiert.

B) Bodenapplikation

Tabakpflanzen (8 Wochen alt) werden mit einer formulierten Lösung des Wirkungsstoffes durch Bodenapplikation behandelt (Konzentration: 0,006 % Aktivsubstanz bezogen auf das Bodenvolumen). Nach 4 Tagen werden die Pflanzen mit einer Sporangiensuspension von Peronospora tabacina ($10^4$ Sporangien/ml) inokuliert, 20 Stunden im Dunkeln bei 25°C und hoher Luftfeuchtigkeit aufbewahrt und dann bei normaler Tag/Nacht Wechselfolge weiterinkubiert.

Die Beurteilung der Symptome in den Tests A und B erfolgt aufgrund der mit Pilz befallenen Blattoberfläche.

Die Kontrollpflanzen zeigen einen Befall von 90 bis 100 %. Pflanzen, welche in Test A mit der Verbindung 2.1 behandelt wurden, zeigten einen Befall von 0-30 %.

Beispiel 3.6: Wirkung gegen Bremia letucae auf Salat

Zu zweiwöchigen Salatpflanzen wird eine formulierte Lösung des Wirkstoffes (0,002 % Aktivsubstanz bezogen auf das Erdvolumen) gegossen. Nach 5 Tagen werden die behandelten Pflanzen mit einer Sporensuspension des Pilzes ($5 \times 10^4$ s/ml) inokuliert. Die Pflanzen werden bei 18°C zuerst unter einer Haube (relative Luftfeuchtigkeit von 90-100 %) während 2 Tagen, dann ohne Haube während 7 Tagen inkubiert. Um den Pilz zur Sporulation zu bringen, werden die Pflanzen wieder für 3 Tage unter eine Haube gestellt.

Die Beurteilung des Pilzbefalls erfolgt 12 Tage nach der Inokulation aufgrund der mit Pilz befallenen Blattoberfläche.

Verbindungen aus den Tabellen 1-7 zeigen eine gute Wirkung gegen Bremia. So blieben Pflanzen, die z.B. mit der Verbindung 2.1 behandelt wurden, weitgehend befallsfrei (0-30 % Schädigung). Unbehandelte, aber infizierte Pflanzen (Kontrolle) wiesen dagegen einen Bremia-Befall von 100 % auf.

Beispiel 3.7: Wirkung gegen Erysiphe graminis auf Weizen

Weizenpflanzen werden nach 5-tägiger Anzucht mit einer aus Spritzpulver des Wirkstoffes hergestellten Spritzbrühe besprüht (Konzentration: 0,02 %). Einen Tag später werden die Pflanzen mit Konidien von Erysiphe graminis infiziert und bei 20°C inkubiert.

Die Beurteilung der Schutzwirkung erfolgt aufgrund des Pilzbefalls 8-10 Tage nach der Infektion.

Als Wirkstoff eingesetzte Verbindungen aus den Tabellen 1 bis 7 zeigen in diesem Test eine gute Wirkung gegen Erysiphe graminis. So blieben Pflanzen, die z.B. mit der Verbindung 1.6 oder 2.1 behandelt wurden, weitgehend frei von Erysiphe-Befall (0 bis 30 % Schädigung). Unbehandelte aber infizierte Pflanzen (Kontrolle) weisen dagegen einen Erysiphe-Befall von 100 % auf.

## Patentansprüche

### Patentansprüche für folgende Vertragsstaaten : DE, GB, FR, IT, NL, SE, CH, LI, BE, AT, LU, GR, DK

1. Verbindungen der Formel I

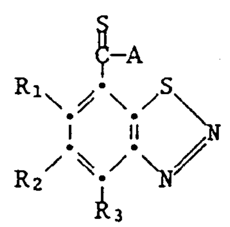

(I),

worin bedeuten:

$R_1$, $R_2$ und $R_3$ voneinander unabhängig Wasserstoff, Halogen, Methyl, Methoxy oder Methylthio;

A $-OR_4$, $-SR_4$, $-N(R_5)R_6$, $-N(R_7)-N(R_6)R_6$, $-O-N=C(R_8)R_9$, $-N(R_7)-OR_{10}$, oder

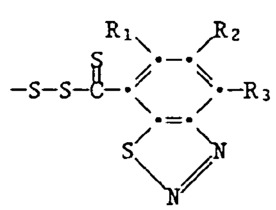

,

$R_4$ $C_1$-$C_{18}$-Alkyl, durch ein Sauerstoff- oder Schwefelatom unterbrochenes $C_3$-$C_{35}$-Alkyl, durch Halogen oder $-COOR_7$ substituiertes $C_1$-$C_8$-Alkyl, unsubstituiertes oder durch Halogen substituiertes $C_3$-$C_6$-Alkenyl, unsubstituiertes oder durch Halogen substituiertes $C_3$-$C_6$-Alkinyl, unsubstituiertes oder durch Halogen oder Methyl substituiertes $C_4$-$C_7$-Cycloalkyl, einen Aryl- oder heterocyclischen Rest U mit maximal

3 Heteroatomen O, N und/oder S, einen über eine maximal 6 Kohlenstoffatome und bis zu 2 Sauerstoffatome enthaltende Alkylenbrücke verknüpften Rest U;
U unsubstituiertes oder durch Halogen, $C_1$-$C_3$-Alkyl oder $C_1$-$C_4$-Alkoxy substituiertes Phenyl, Phenoxyphenyl, Biphenyl, 1- oder 2-Naphthyl, oder einen unsubstituierten oder durch Halogen, $C_1$-$C_3$-Alkyl und/oder $C_1$-$C_3$-Alkoxy ein- oder mehrfach substituierten gesättigten oder ungesättigten 5- bis 7-gliedrigen Heterocyclus T;
$R_5$ und $R_6$ unabhängig voneinander Wasserstoff, $C_1$-$C_8$-Alkyl, durch $C_1$-$C_3$-Alkoxy und/oder Cyano substituiertes $C_1$-$C_6$-Alkyl, oder $C_3$-$C_6$-Alkenyl, $C_3$-$C_6$-Alkinyl, $C_3$-$C_7$-Cycloalkyl, einen Rest U, einen über eine maximal 2 C-Atome umfassende Alkylenbrücke verknüpften Rest U;
oder $R_5$ und $R_6$ mit dem Stickstoffatom zusammen einen 5- bis 7-gliedrigen unsubstituierten oder durch Methyl substituierten Heterocyclus W mit maximal 2 weiteren Heteroatomen O, N und/oder S;
$R_7$ Wasserstoff oder $C_1$-$C_4$-Alkyl;
$R_8$ und $R_9$ unabhängig voneinander Wasserstoff, $C_1$-$C_6$-Alkyl, Cyano, $CONH_2$, $CONHCONHR_7$, unsubstituiertes oder durch Halogen, $C_1$-$C_3$-Alkyl oder $C_1$-$C_3$-Alkoxy substituiertes Phenyl; oder, zusammen mit dem verbindenden Kohlenstoffatom auch einen 5-7-gliedrigen Carbocyclus; und
$R_{10}$ Wasserstoff, $C_1$-$C_6$-Alkyl, $C_3$-$C_6$-Alkenyl, $C_5$-$C_7$-Cycloalkyl, Phenyl oder Benzyl.

2. Verbindungen der Formel I gemäss Anspruch 1, worin bedeuten:
$R_1$, $R_2$ und $R_3$ unabhängig voneinander Wasserstoff, Fluor oder Methyl; A -$OR_4$, -$SR_4$, -$N(R_5)R_5$, -$N(R_7)$-$N(R_5)R_5$, -$O$-$N{=}C(R_8)R_9$, -$N(R_7)$-$OR_{10}$,

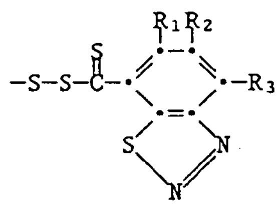 ;

$R_4$ $C_1$-$C_6$-Alkyl, $C_5$-$C_6$-Cycloalkyl, Phenyl, Benzyl, Tetrahydrofuran-2-on-3-yl, 2-, 3- oder 4-Pyridyl, 2-, 4- oder 5-Pyrimidyl, (2,2-Dimethyldioxolan-4-yl)-methyl;
$R_5$ und $R_5$ unabhängig voneinander Wasserstoff, $C_1$-$C_4$-Alkyl, durch Methoxy, Ethoxy und/oder Cyano substituiertes $C_1$-$C_3$-Alkyl, oder Allyl, Propargyl, $C_3$-$C_6$-Cycloalkyl, Phenyl, Benzyl, Phenethyl, 2-Furfurylmethyl, Tetrahydrofuran-2-on-3-yl, 2-, 3- oder 4-Pyridyl, 2-, 4- oder 5-Pyrimidyl, $R_7$ Wasserstoff, Methyl oder Ethyl; oder
die Gruppe $N(R_5)R_5$ zusammen einen Pyrrol-, Pyrrolidin-, Imidazol-1-yl, 1,2,4-Triazol-1-yl-, Piperidin-, Morpholin- oder 2,6-Dimethylmorpholin-Ring bildet;
$R_8$ und $R_9$ unabhängig voneinander Wasserstoff, $C_1$-$C_6$-Alkyl, Cyano, die Reste $CONH_2$, $CONHCONHR_7$, Phenyl oder zusammen mit dem verbindenden Kohlenstoffatom einen 5- bis 7-gliedrigen Carbocyclus bilden; und $R_{10}$ Wasserstoff, $C_1$-$C_3$-Alkyl, Allyl, $C_5$-$C_6$-Cycloalkyl, Phenyl oder Benzyl.

3. Verbindungen der Formel I gemäss Anspruch 1, worin bedeuten:
$R_1$, $R_2$ und $R_3$ unabhängig voneinander Wasserstoff, Fluor oder Methyl; A -$OR_4$, -$SR_4$, -$N(R_5)R_5$, -$N(R_7)$-$N(R_5)R_5$, -$O$-$N{=}C(R_8)R_9$ oder -$N(R_7)$-$OR_{10}$;
$R_4$ $C_1$-$C_6$-Alkyl, $C_5$-$C_6$-Cycloalkyl, Phenyl, Benzyl, Tetrahydrofuran-2-on-3-yl, 2-, 3- oder 4-Pyridyl;
$R_5$ und $R_6$ unabhängig voneinander Wasserstoff, $C_1$-$C_3$-Alkyl, durch Methoxy und/oder Cyano substituiertes $C_1$-$C_3$-Alkyl, oder Allyl, Propargyl, $C_3$-$C_5$-Cycloalkyl, Phenyl, Benzyl, 2-Furfuryl, Tetrahydrofuran-2-on-3-yl, 2-, 3- oder 4-Pyridyl, 2-, 4-oder 5-Pyrimidyl, Piperidin-1-yl-, oder die Gruppe $N(R_5)R_5$ zusammen einen Pyrrolidin-, Imidazol-1-yl-, Piperidin-, Morpholin- oder 2,6-Dimethylmorpholin-Ring darstellt;
$R_7$ Wasserstoff, Methyl oder Ethyl;
$R_8$ und $R_9$ unabhängig voneinander Wasserstoff, Methyl, Ethyl oder die Reste Cyano, $CONH_2$, Phenyl, Benzyl oder zusammen mit dem verbindenden Kohlenstoffatom Cyclopentyl oder Cyclohexyl; und
$R_{10}$ Wasserstoff, Methyl, Ethyl, Allyl, $C_3$-$C_5$-Cycloalkyl oder Phenyl.

4. Verbindungen der Formel I gemäss Anspruch 1, worin bedeuten:
$R_1$, $R_2$ und $R_3$ unabhängig voneinander Wasserstoff oder Fluor; A -$OR_4$, -$SR_4$, -$N(R_5)R_6$, -$N(R_7)$-$N(R_5)R_5$;
$R_4$ $C_1$-$C_4$-Alkyl, $C_5$-$C_6$-Cycloalkyl, Phenyl, Benzyl, 2-, 3- oder 4-Pyridyl, 2-, 4- oder 5-Pyrimidyl;
$R_5$ und $R_6$ unabhängig voneinander Wasserstoff, Methyl, Ethyl, Allyl, Propargyl, Phenyl, Benzyl, 2-Furanylmethyl, 2-, 3- oder 4-Pyridyl, oder die Gruppe $N(R_5)R_6$ gemeinsam für einen Pyrrolidin-, Piperidin-, Morpholin-, 2,6-Dimethylmorpholin- oder Imidazol-1-yl-Ring steht; und $R_7$ Wasserstoff, Methyl oder Ethyl.

**5.** Eine Verbindung der Formel I gemäss Anspruch 1 aus der Gruppe:
Benzo-1,2,3-thiadiazol-7-thio-carbonsäure-O-ethylester ;
Benzo-1,2,3-thiadiazol-7-dithio-carbonsäure--methylester ;
Benzo-1,2,3-thiadiazol-7-carbonsäurethioamid ;
7-[Benzo-1,2,3-thiadiazol]-N',N'-dimethylthiohydrazid ;
Benzo-1,2,3-thiadiazol-7-dithiocarbonsäure--ethylester ;
Benzo-1,2,3-thiadiazol-7-dithiocarbonsäure--propylester ;
Benzo-1,2,3-thiadiazol-7-dithiocarbonsäure--cyclopentylester ;
Benzo-1,2,3-thiadiazol-7-thiocarbonsäure-morpholid ;
Benzo-1,2,3-thiadiazol-7-thiocarbonsäure-piperidid.

**6.** Verfahren zur Herstellung von Verbindungen der Formel I in Anspruch 1, gekennzeichnet durch
A) Umsetzung einer Verbindung der Formel IIa

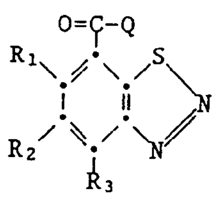

(IIa)

mit einem Thionierungsreagens, z.B. mit Phosphorpentasulfid ($P_4S_{10}$) oder Lawesson-Reagens [2,4-Bis(4-methoxyphenyl)-1,3,2,4-dithiadiphosphetan-2,4-disulfid] in einem aprotischen unpolaren oder leicht polaren Lösungsmittel bei Temperaturen von 20° bis 200°C, bevorzugt 60° bis 150°C, wobei Q die Reste $-OR_4$, $-SR_4$ oder $-N(R_5)R_5$ darstellt, zu einer Verbindung der Formel Ia

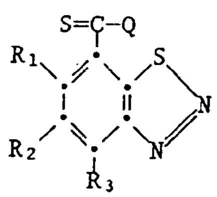

(Ia)

worin $R_1$, $R_2$ und $R_3$ die unter Formel I angegebenen Bedeutungen besitzen;
$B_1$) Umsetzung der Lösung eines Nitrils der Formel II

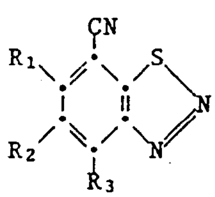

(II)

in einem Alkohol der Formel $R_4$'OH, wobei $R_4$' die unter Formel I angegebene Bedeutung von $R_4$ hat, durch Einleiten von gasförmigem Halogenwasserstoff bei Temperaturen von -20° bis 80°C, vorzugsweise -10° bis 40°C, zu einem Imidat der Formel III

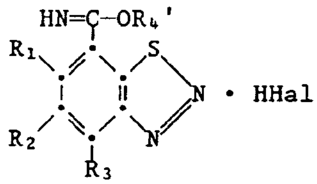

(III),

worin Hal Halogen bedeutet;
und
$B_2$) anschliessende Umsetzung der erhaltenen Verbindung der Formel III mit Schwefelwasserstoff in unpolaren oder schwach polaren Lösungsmitteln in Gegenwart einer Base bei Temperaturen von -20° bis 80°C, vorzugsweise -5° bis 40°C, zu Thiocarbonsäureestern der Formel Ia'

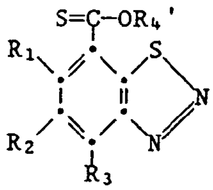

(Ia')

wobei $R_1$, $R_2$, $R_3$ die unter Formel I angegebenen Bedeutungen besitzen, und $R_4$' die unter Formel I angegebenen Bedeutungen von $R_4$ darstellt;
C) Umsetzung einer Thiono-Verbindung der Formel IV

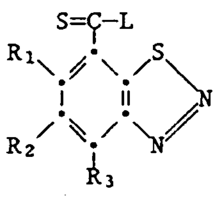

(IV),

worin L die Bedeutung von Chlor oder eines Azolids der Formel

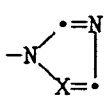

,

in welcher X CH oder N darstellt, sowie die Bedeutung von -S-$C_1$-$C_4$-Alkyl, -S-$CH_2COOM$ oder SM hat, wobei M für Wasserstoff oder ein Alkaliion steht;
($C_1$) mit einem Alkohol oder einem Thiol der Formel $R_4$'OH oder $R_4$'SH, wobei $R_4$' die Bedeutungen von $R_4$ hat, zu einer Verbindung der Formel Ia' oder Ia"

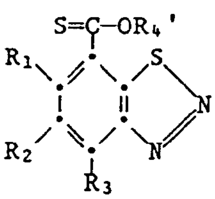

oder

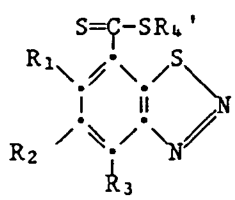

(Ia') (Ia")

oder
($C_2$) mit einem Oxim der Formel HON=C($R_7$)$R_8$ zu einer Verbindung der Formel Ib

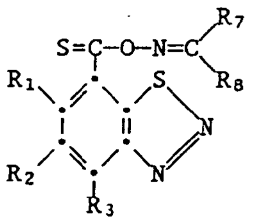

(Ib);

oder

($C_3$) mit einem Amin der Formel $HN(R_5)R_6$ zu einer Verbindung der Formel Ic

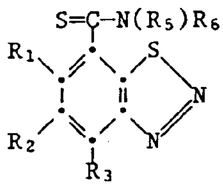

(Ic);

oder

($C_4$) mit einem Hydroxylamin-Derivat der Formel $HN(R_7)$-O-$R_{10}$ zu einer Verbindung der Formel Id

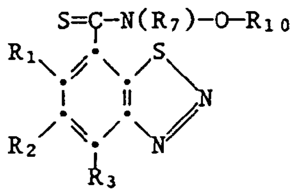

(Id);

oder

($C_5$) mit einem Hydrazin-Derivat der Formel $HN(R_7)$-$N(R_5)R_6$ zu einer Verbindung der Formel Ie

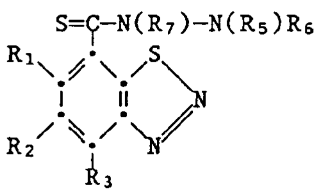

(Ie)

in inerten Lösungsmitteln mit oder ohne Base bei einer Temperatur von -20° bis 170°C;

D) Alkylierung der Dithiosäure oder ihres Salzes der Formel IVa

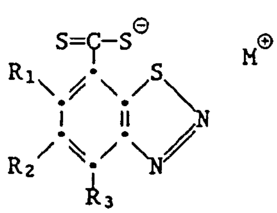

(IVa)

($M^{\oplus}$ = Wasserstoff oder Alkaliion) mit einem Alkylierungsmittel der Formel $R_4'$-L, wobei $R_4'$ die Bedeutung von $R_4$ hat und L eine Abgangsgruppe bedeutet, in Gegenwart einer Base in inerten Lösungsmitteln bei Temperaturen von -30° bis 110°C zu einer Verbindung der Formel Ia"

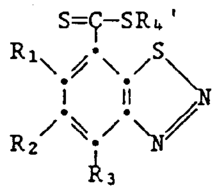

(Ia")

E) Oxidation der Dithiosäure oder ihres Salzes der Formel IVa mit Luftsauerstoff, Kaliumhexacyanoferrat(III) oder $KJ/J_2$ in wässriger Lösung bei Temperaturen von 10° bis 60°C zu einer Verbindung der Formel If

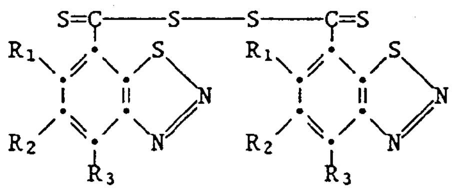

(If),

wobei in den vorstehenden Formeln $R_1$ bis $R_{10}$ die unter Formel I angegebenen Bedeutungen haben.

**7.** Mittel zum Schutz von Pflanzen gegen den Befall durch Mikroorganismen, dadurch gekennzeichnet, dass es neben üblichen Träger- und Hilfsstoffen als aktive Komponente mindestens eine Verbindung der Formel I gemäss Anspruch 1 enthält.

**8.** Mittel gemäss Anspruch 7, dadurch gekennzeichnet, dass es als aktive Komponente mindestens eine Verbindung gemäss den Ansprüchen 2 bis 4 enthält.

**9.** Mittel gemäss Anspruch 7, dadurch gekennzeichnet, dass es als aktive Komponente die Verbindung der Formel I gemäss Anspruch 5 enthält.

**10.** Verfahren zur Herstellung eines agrochemischen Mittels von Anspruch 7, dadurch gekennzeichnet, dass man mindestens eine gemäss Anspruch 1 definierte Verbindung mit geeigneten festen oder flüssigen Träger- und Hilfsstoffen innig vermischt.

**11.** Verwendung von Verbindungen der Formel I gemäss Anspruch 1 zum Schutz von Pflanzen gegen den Befall durch phytopathogene Mikroorganismen.

**12.** Verwendung von Verbindungen gemäss einem der Ansprüche 2 bis 5 zum Schutz von Pflanzen gegen den Befall durch phytopathogene Mikroorganismen.

**13.** Verfahren zum Schutz von Pflanzen gegen den Befall durch phytopathogene Mikroorganismen, dadurch gekennzeichnet, dass man als Wirkstoff eine Verbindung der Formel I gemäss Anspruch 1 auf die Pflanze oder deren Standort appliziert.

**14.** Verfahren zum Schutz von Pflanzen gegen den Befall durch phytopathogene Mikroorganismen, dadurch gekennzeichnet, dass man als Wirkstoff eine Verbindung gemäss einem der Ansprüche 2 bis 5 auf die Pflanze oder deren Standort appliziert.

**15.** Verfahren gemäss den Ansprüchen 13 und 14, dadurch gekennzeichnet, dass es sich bei den phytopathogenen Mikroorganismen um Pilzorganismen handelt.

**16.** Verbindungen der Formel III sowie deren Salze mit einer Halogenwasserstoffsäure

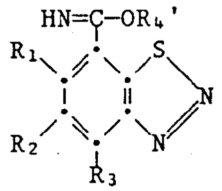

(• HHal) (III),

worin bedeuten:
$R_1$, $R_2$ und $R_3$ unabhängig voneinander Wasserstoff, Halogen, Methyl, Methoxy oder Methylthio;
$R_4$' $C_1$-$C_{18}$-Alkyl, durch ein Sauerstoff- oder Schwefelatom unterbrochenes $C_3$-$C_{35}$-Alkyl, durch Halogen oder -$COOR_7$ substituiertes $C_1$-$C_8$-Alkyl, unsubstituiertes oder durch Halogen substituiertes $C_3$-$C_6$-Alkenyl, unsubstituiertes oder durch Halogen substituiertes $C_3$-$C_6$-Alkinyl, unsubstituiertes oder durch Halogen oder Methyl substituiertes $C_4$-$C_7$-Cycloalkyl, einen Aryl- oder heterocyclischen Rest U mit maximal 3 Heteroatomen O, N und/oder S, einen über eine maximal 6 Kohlenstoffatome und bis zu 2 Sauerstoffatome enthaltende Alkylenbrücke verknüpften Rest U;
U unsubstituiertes oder durch Halogen, $C_1$-$C_3$-Alkyl oder $C_1$-$C_4$-Alkoxy substituiertes Phenyl, Phenoxyphenyl, Biphenyl, 1- oder 2-Naphthyl, oder einen unsubstituierten oder durch Halogen, $C_1$-$C_3$-Alkyl und/oder $C_1$-$C_3$-Alkoxy ein- oder mehrfach substituierten gesättigten oder ungesättigten 5- bis 7-gliedrigen Heterocyclus T; und
Hal Halogen.

**17.** Verbindungen der Formel V

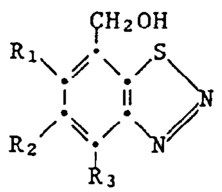

(V), ,

worin bedeuten:
$R_1$, $R_2$ und $R_3$ unabhängig voneinander Wasserstoff, Halogen, Methyl, Methoxy oder Methylthio.

**18.** Verbindungen der Formel VI

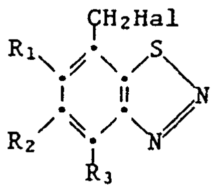

(VI)

worin bedeuten:
$R_1$, $R_2$ und $R_3$ unabhängig voneinander Wasserstoff, Halogen, Methyl, Methoxy oder Methylthio und Hal Halogen.

**19.** Verfahren zur Herstellung von Verbindungen der Formel V

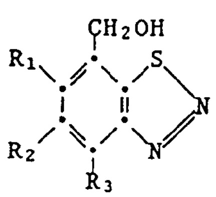

(V), ,

gekennzeichnet durch Reduktion einer Verbindung der Formel

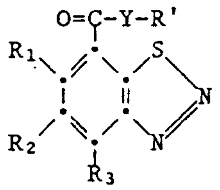

,

in welcher $R_1$, $R_2$ und $R_3$ unabhängig voneinander Wasserstoff, Halogen, Methyl, Methoxy oder Methylthio bedeuten, R' Wasserstoff oder $C_1$-$C_4$-Alkyl und Y Sauerstoff oder Schwefel darstellen, mit einem Reduktionsmittel in einem inerten Lösungsmittel bei Temperaturen von 0° bis 60°C.

**20.** Verfahren zur Herstellung von Verbindungen der Formel VI

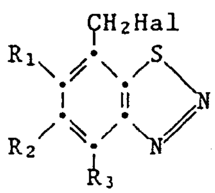

(VI)

gekennzeichnet durch die Halogenierung einer Verbindung der Formel V

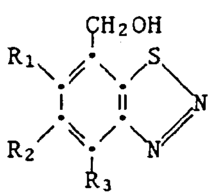

(V), ,

in welcher $R_1$, $R_2$ und $R_3$ unabhängig voneinander Wasserstoff, Halogen, Methyl, Methoxy oder Methylthio und Hal Halogen bedeuten, mittels eines Halogenierungsmittels in Gegenwart einer organischen Base in einem inerten Lösungsmittel bei Temperaturen von -10° bis 100°C.

**21.** Mittel zum Schutz von Pflanzen gegen den Befall durch Mikroorganismen, dadurch gekennzeichnet, dass es neben üblichen Träger- und Hilfsstoffen als aktive Komponente mindestens eine Verbindung gemäss Anspruch 17 oder 18 enthält.

**22.** Verwendung einer Verbindung gemäss Anspruch 17 oder 18 zum Schutz von Pflanzen gegen den Befall durch phytopathogene Mikroorganismen.

**23.** Verfahren zum Schutz von Pflanzen gegen den Befall durch phytopathogene Mikroorganismen, dadurch gekennzeichnet, dass als Wirkstoff eine Verbindung gemäss Anspruch 17 oder 18 auf die Pflanze oder deren Standort appliziert wird.

**Patentansprüche für folgenden Vertragsstaat : ES**

**1.** Mittel zum Schutz von Pflanzen gegen den Befall durch Mikroorganismen, dadurch gekennzeichnet, dass es als aktive Komponente mindestens eine Verbindung der Formel I

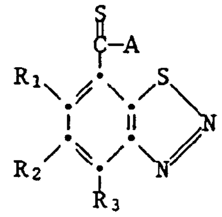

(I),

enthält, worin bedeuten:

$R_1$, $R_2$ und $R_3$ voneinander unabhängig Wasserstoff, Halogen, Methyl, Methoxy oder Methylthio;

A -$OR_4$, -$SR_4$, -$N(R_5)R_6$, -$N(R_7)$-$N(R_6)R_6$, -O-N=$C(R_8)R_9$, -$N(R_7)$-$OR_{10}$, oder

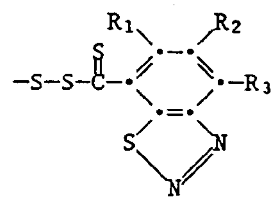

,

$R_4$ $C_1$-$C_{18}$-Alkyl, durch ein Sauerstoff- oder Schwefelatom unterbrochenes $C_3$-$C_{35}$-Alkyl, durch Halogen oder -$COOR_7$ substituiertes $C_1$-$C_8$-Alkyl, unsubstituiertes oder durch Halogen substituiertes $C_3$-$C_6$-Alkenyl, unsubstituiertes oder durch Halogen substituiertes $C_3$-$C_6$-Alkinyl, unsubstituiertes oder durch Halogen oder Methyl substituiertes $C_4$-$C_7$-Cycloalkyl, einen Aryl- oder heterocyclischen Rest U mit maximal 3 Heteroatomen O, N und/oder S, einen über eine maximal 6 Kohlenstoffatome und bis zu 2 Sauerstoffatome enthaltende Alkylenbrücke verknüpften Rest U;

U unsubstituiertes oder durch Halogen, $C_1$-$C_3$-Alkyl oder $C_1$-$C_4$-Alkoxy substituiertes Phenyl, Phenoxyphenyl, Biphenyl, 1- oder 2-Naphthyl, oder einen unsubstituierten oder durch Halogen, $C_1$-$C_3$-Alkyl und/oder $C_1$-$C_3$-Alkoxy ein- oder mehrfach substituierten gesättigten oder ungesättigten 5- bis 7-gliedrigen Heterocyclus T;

$R_5$ und $R_6$ unabhängig voneinander Wasserstoff, $C_1$-$C_8$-Alkyl, durch $C_1$-$C_3$-Alkoxy und/oder Cyano substituiertes $C_1$-$C_6$-Alkyl, oder $C_3$-$C_6$-Alkenyl, $C_3$-$C_6$-Alkinyl, $C_3$-$C_7$-Cycloalkyl, einen Rest U, einen über eine maximal 2 C-Atome umfassende Alkylenbrücke verknüpften Rest U;

oder $R_6$ und $R_6$ mit dem Stickstoffatom zusammen einen 5- bis 7-gliedrigen unsubstituierten oder durch Methyl substituierten Heterocyclus W mit maximal 2 weiteren Heteroatomen O, N und/oder S;

$R_7$ Wasserstoff oder $C_1$-$C_4$-Alkyl;

$R_8$ und $R_9$ unabhängig voneinander Wasserstoff, $C_1$-$C_6$-Alkyl, Cyano, $CONH_2$, $CONHCONHR_7$, unsubstituiertes oder durch Halogen, $C_1$-$C_3$-Alkyl oder $C_1$-$C_3$-Alkoxy substituiertes Phenyl; oder, zusammen mit dem verbindenden Kohlenstoffatom auch einen 5-7-gliedrigen Carbocyclus; und

$R_{10}$ Wasserstoff, $C_1$-$C_6$-Alkyl, $C_3$-$C_6$-Alkenyl, $C_5$-$C_7$-Cycloalkyl, Phenyl oder Benzyl.

2. Mittel gemäss Anspruch 1, dadurch gekennzeichnet, dass es als aktive Komponente mindestens eine Verbindung der Formel I enthält, worin bedeuten:

$R_1$, $R_2$ und $R_3$ unabhängig voneinander Wasserstoff, Fluor oder Methyl;

A -$OR_4$, -$SR_4$, -$N(R_5)R_6$, -N(R7)-$N(R_6)R_6$, -O-N=$C(R_8)R_9$, -$N(R_7)$-$OR_{10}$,

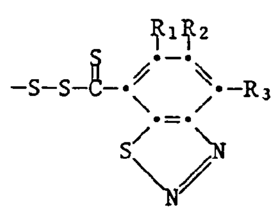

;

$R_4$ $C_1$-$C_6$-Alkyl, $C_5$-$C_6$-Cycloalkyl, Phenyl, Benzyl, Tetrahydrofuran-2-on-3-yl, 2-, 3- oder 4-Pyridyl, 2-, 4- oder 5-Pyrimidyl, (2,2-Dimethyldioxolan-4-yl)-methyl;

$R_5$ und $R_6$ unabhängig voneinander Wasserstoff, $C_1$-$C_4$-Alkyl, durch Methoxy, Ethoxy und/oder Cyano

substituiertes $C_1$-$C_3$-Alkyl, oder Allyl, Propargyl, $C_3$-$C_6$-Cycloalkyl, Phenyl, Benzyl, Phenethyl, 2-Furfurylmethyl, Tetrahydrofuran-2-on-3-yl, 2-, 3- oder 4-Pyridyl, 2-, 4- oder 5-Pyrimidyl, $R_7$ Wasserstoff, Methyl oder Ethyl; oder
die Gruppe $N(R_5)R_6$ zusammen einen Pyrrol-, Pyrrolidin-, Imidazol-1-yl, 1,2,4-Triazol-1-yl-, Piperidin-, Morpholin- oder 2,6-Dimethylmorpholin-Ring bildet;
$R_8$ und $R_9$ unabhängig voneinander Wasserstoff, $C_1$-$C_6$-Alkyl, Cyano, die Reste $CONH_2$, $CONHCONHR_7$, Phenyl oder zusammen mit dem verbindenden Kohlenstoffatom einen 5- bis 7-gliedrigen Carbocyclus bilden; und
$R_{10}$ Wasserstoff, $C_1$-$C_3$-Alkyl, Allyl, $C_5$-$C_6$-Cycloalkyl, Phenyl oder Benzyl.

3. Mittel gemäss Anspruch 1, dadurch gekennzeichnet, dass es als aktive Komponente mindestens eine Verbindung enthält, worin bedeuten:
$R_1$, $R_2$ und $R_3$ unabhängig voneinander Wasserstoff, Fluor oder Methyl; A $-OR_4$, $-SR_4$, $-N(R_5)R_6$, $-N(R_7)-N(R_5)R_6$, $-O-N=C(R_8)R_9$ oder $-N(R_7)-OR_{10}$;
$R_4$ $C_1$-$C_6$-Alkyl, $C_5$-$C_6$-Cycloalkyl, Phenyl, Benzyl, Tetrahydrofuran-2-on-3-yl, 2-, 3- oder 4-Pyridyl;
$R_5$ und $R_6$ unabhängig voneinander Wasserstoff, $C_1$-$C_3$-Alkyl, durch Methoxy und/oder Cyano substituiertes $C_1$-$C_3$-Alkyl, oder Allyl, Propargyl, $C_3$-$C_5$-Cycloalkyl, Phenyl, Benzyl, 2-Furfuryl, Tetrahydrofuran-2-on-3-yl, 2-, 3- oder 4-Pyridyl, 2-, 4-oder 5-Pyrimidyl, Piperidin-1-yl-, oder die Gruppe $N(R_5)R_6$ zusammen einen Pyrrolidin-, Imidazol-1-yl-, Piperidin-, Morpholin- oder 2,6-Dimethylmorpholin-Ring darstellt;
$R_7$ Wasserstoff, Methyl oder Ethyl;
$R_8$ und $R_9$ unabhängig voneinander Wasserstoff, Methyl, Ethyl oder die Reste Cyano, $CONH_2$, Phenyl, Benzyl oder zusammen mit dem verbindenden Kohlenstoffatom Cyclopentyl oder Cyclohexyl; und
$R_{10}$ Wasserstoff, Methyl, Ethyl, Allyl, $C_3$-$C_5$-Cycloalkyl oder Phenyl.

4. Mittel gemäss Anspruch 1, dadurch gekennzeichnet, dass es als aktive Komponente mindestens eine Verbindung der Formel I enthält, worin bedeuten:
$R_1$, $R_2$ und $R_3$ unabhängig voneinander Wasserstoff oder Fluor;
A $-OR_4$, $-SR_4$, $-N(R_5)R_6$, $-N(R_7)-N(R_6)R_6$;
$R_4$ $C_1$-$C_4$-Alkyl, $C_5$-$C_6$-Cycloalkyl, Phenyl, Benzyl, 2-, 3- oder 4-Pyridyl, 2-, 4- oder 5-Pyrimidyl;
$R_5$ und $R_6$ unabhängig voneinander Wasserstoff, Methyl, Ethyl, Allyl, Propargyl, Phenyl, Benzyl, 2-Furanylmethyl, 2-, 3- oder 4-Pyridyl, oder die Gruppe $N(R_6)R_6$ gemeinsam für einen Pyrrolidin-, Piperidin-, Morpholin-, 2,6-Dimethylmorpholin- oder Imidazol-1-yl-Ring steht; und $R_7$ Wasserstoff, Methyl oder Ethyl.

5. Mittel gemäss Anspruch 1, dadurch gekennzeichnet, dass es als aktive Komponente mindestens eine Verbindung der Formel I aus der Gruppe enthält:
Benzo-1,2,3-thiadiazol-7-thio-carbonsäure-O-ethylester ;
Benzo-1,2,3-thiadiazol-7-dithio-carbonsäure--methylester ;
Benzo-1,2,3-thiadiazol-7-carbonsäurethioamid ;
7-[Benzo-1,2,3-thiadiazol]-N',N'-dimethylthiohydrazid ;
Benzo-1,2,3-thiadiazol-7-dithiocarbonsäure--ethylester ;
Benzo-1,2,3-thiadiazol-7dithiocarbonsäure--propylester ;
Benzo-1,2,3-thiadiazol-7-dithiocarbonsäure--cyclopentylester ;
Benzo-1,2,3-thiadiazol-7-thiocarbonsäure-morpholid ;
Benzo-1,2,3-thiadiazol-7-thiocarbonsäure-piperidid.

6. Verfahren zur Herstellung von Verbindungen der Formel I in Anspruch 1, gekennzeichnet durch
A) Umsetzung einer Verbindung der Formel IIa

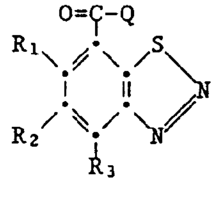

(IIa)

mit einem Thionierungsreagens, z.B. mit Phosphorpentasulfid ($P_4S_{10}$) oder Lawesson-Reagens [2,4-Bis(4-methoxyphenyl)-1,3,2,4-dithiadiphosphetan-2,4-disulfid] in einem aprotischen unpolaren oder leicht polaren Lösungsmittel bei Temperaturen von 20° bis 200°C, bevorzugt 60° bis 150°C, wobei Q die Reste $-OR_4$, $-SR_4$ oder $-N(R_5)R_6$ darstellt, zu einer Verbindung der Formel Ia

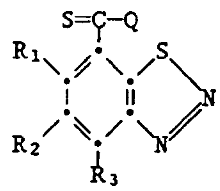

(Ia)

worin $R_1$, $R_2$ und $R_3$ die unter Formel I angegebenen Bedeutungen besitzen;

$B_1$) Umsetzung der Lösung eines Nitrils der Formel II

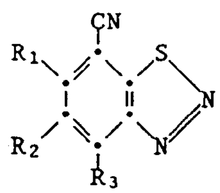

(II)

in einem Alkohol der Formel $R_4$'OH wobei $R_4$' die unter Formel I angegebene Bedeutung von $R_4$ hat durch Einleiten von gasförmigem Halogenwasserstoff bei Temperaturen von -20° bis 80°C, vorzugsweise -10° bis 40°C, zu einem Imidat der Formel III

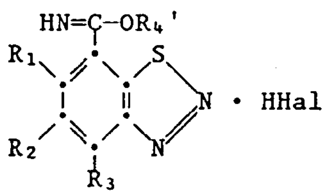

(III),

worin Hal Halogen bedeutet;

und

$B_2$) anschliessende Umsetzung der erhaltenen Verbindung der Formel III mit Schwefelwasserstoff in unpolaren oder schwach polaren Lösungsmitteln in Gegenwart einer Base bei Temperaturen von -20° bis 80°C, vorzugsweise -5° bis 40°C, zu Thiocarbonsäureestern der Formel Ia'

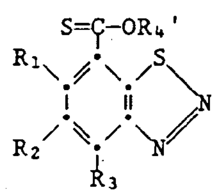

(Ia')

wobei $R_1$, $R_2$, $R_3$ die unter Formel I angegebenen Bedeutungen besitzen, und $R_4$' die unter Formel I angegebenen Bedeutungen von $R_4$ darstellt;

C) Umsetzung einer Thiono-Verbindung der Formel IV

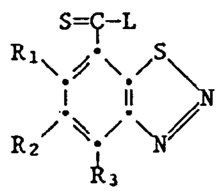

(IV),

worin L die Bedeutung von Chlor oder eines Azolids der Formel

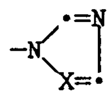

,

in welcher X CH oder N darstellt, sowie die Bedeutung von -S-$C_1$-$C_4$-Alkyl, -S-$CH_2COOM$ oder SM hat, wobei M für Wasserstoff oder ein Alkaliion steht;
($C_1$) mit einem Alkohol oder einem Thiol der Formel $R_4$'OH oder $R_4$'SH, wobei $R_4$' die Bedeutungen von $R_4$ hat, zu einer Verbindung der Formel Ia' oder Ia"

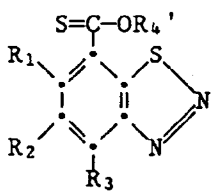

oder

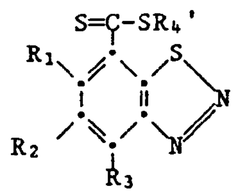

(Ia') (Ia")

oder
($C_2$) mit einem Oxim der Formel HON=C($R_7$)$R_8$ zu einer Verbindung der Formel Ib

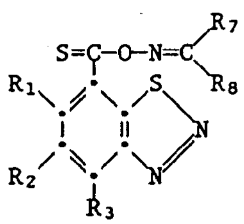

(Ib);

oder
($C_3$) mit einem Amin der Formel HN($R_5$)$R_6$ zu einer Verbindung der Formel Ic

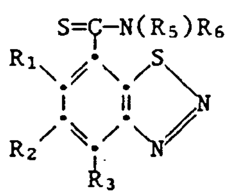

(Ic);

oder
($C_4$) mit einem Hydroxylamin-Derivat der Formel HN($R_7$)-O-$R_{10}$ zu einer Verbindung der Formel Id

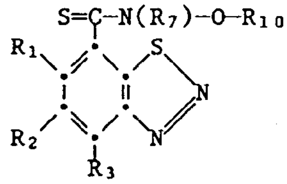

(Id);

oder

($C_5$) mit einem Hydrazin-Derivat der Formel $HN(R_7)$-$N(R_5)R_6$ zu einer Verbindung der Formel Ie

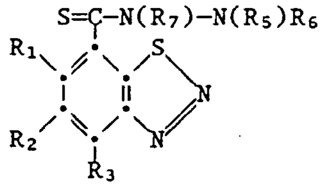

(Ie)

in inerten Lösungsmitteln mit oder ohne Base bei einer Temperatur von -20° bis 170°C;

D) Alkylierung der Dithiosäure oder ihres Salzes der Formel IVa

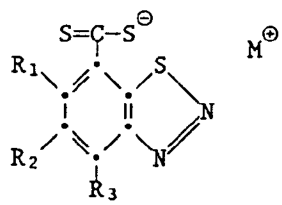

(IVa)

($M^{\oplus}$ = Wasserstoff oder Alkaliion)

mit einem Alkylierungsmittel der Formel $R_4'$-L, wobei $R_4'$ die Bedeutung von $R_4$ hat und L eine Abgangsgruppe bedeutet, in Gegenwart einer Base in inerten Lösungsmitteln bei Temperaturen von -30° bis 110°C zu einer Verbindung der Formel Ia"

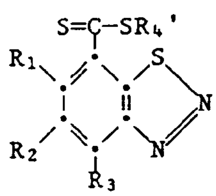

(Ia")

E) Oxidation der Dithiosäure oder ihres Salzes der Formel IVa mit Luftsauerstoff, Kaliumhexacyanoferrat(III) oder $KJ/J_2$ in wässriger Lösung bei Temperaturen von 10° bis 60°C zu einer Verbindung der Formel If

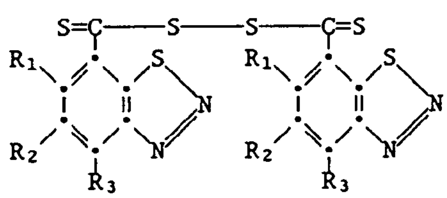

(If),

wobei in den vorstehenden Formeln $R_1$ bis $R_{10}$ die unter Formel I angegebenen Bedeutungen haben.

**7.** Verwendung von Verbindungen der Formel I gemäss Anspruch 1 zum Schutz von Pflanzen gegen den

Befall durch phytopathogene Mikroorganismen.

8. Verwendung von Verbindungen gemäss einem der Ansprüche 2 bis 5 zum Schutz von Pflanzen gegen den Befall durch phytopathogene Mikroorganismen.

9. Verfahren zum Schutz von Pflanzen gegen den Befall durch phytopathogene Mikroorganismen, dadurch gekennzeichnet, dass man als Wirkstoff eine Verbindung der Formel I gemäss Anspruch 1 auf die Pflanze oder deren Standort appliziert.

10. Verfahren zum Schutz von Pflanzen gegen den Befall durch phytopathogene Mikroorganismen, dadurch gekennzeichnet, dass man als Wirkstoff eine Verbindung gemäss einem der Ansprüche 2 bis 5 auf die Pflanze oder deren Standort appliziert.

11. Verfahren gemäss den Ansprüchen 9 und 10, dadurch gekennzeichnet, dass es sich bei den phytopathogenen Mikroorganismen um Pilzorganismen handelt.

12. Mittel zum Schutz von Pflanzen gegen den Befall durch Mikroorganismen, dadurch gekennzeichnet, dass es als aktive Komponente mindestens eine Verbindung der Formel V

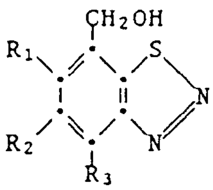

(V), ,

enthält, worin bedeuten:
$R_1$, $R_2$ und $R_3$ unabhängig voneinander Wasserstoff, Halogen, Methyl, Methoxy oder Methylthio.

13. Mittel zum Schutz von Pflanzen gegen den Befall durch Mikroorganismen, dadurch gekennzeichnet, dass es als aktive Komponente mindestens eine Verbindung der Formel VI

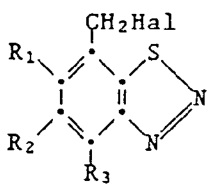

(VI)

enthält, worin bedeuten:
$R_1$, $R_2$ und $R_3$ unabhängig voneinander Wasserstoff, Halogen, Methyl, Methoxy oder Methylthio und Hal Halogen.

14. Verfahren zur Herstellung von Verbindungen der Formel V

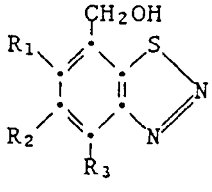

(V), ,

gekennzeichnet durch Reduktion einer Verbindung der Formel

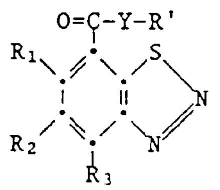

,

in welcher $R_1$, $R_2$ und $R_3$ unabhängig voneinander Wasserstoff, Halogen, Methyl, Methoxy oder Methylthio bedeuten, R' Wasserstoff oder $C_1$-$C_4$-Alkyl und Y Sauerstoff oder Schwefel darstellen, mit einem Reduktionsmittel in einem inerten Losungsmittel bei Temperaturen von 0° bis 60°C.

**15.** Verfahren zur Herstellung von Verbindungen der Formel VI

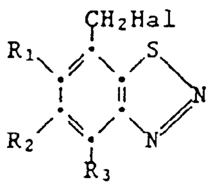

(VI)

gekennzeichnet durch die Halogenierung einer Verbindung der Formel V

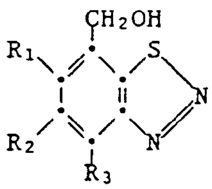

(V), ,

in welcher $R_1$, $R_2$ und $R_3$ unabhängig voneinander Wasserstoff, Halogen, Methyl, Methoxy oder Methylthio und Hal Halogen bedeuten, mittels eines Halogenierungsmittels in Gegenwart einer organischen Base in einem inerten Lösungsmittel bei Temperaturen von -10° bis 100°C.

**16.** Verwendung einer Verbindung gemäss Anspruch 12 oder 13 zum Schutz von Pflanzen gegen den Befall durch phytopathogene Mikroorganismen.

**17.** Verfahren zum Schutz von Pflanzen gegen den Befall durch phytopathogene Mikroorganismen, dadurch gekennzeichnet, dass als Wirkstoff eine Verbindung gemäss Anspruch 12 oder 13 auf die Pflanze oder deren Standort appliziert wird.

## Claims

### Claims for the following Contracting States : DE, GB, FR, IT, NL, SE, CH, LI, BE, AT, LU, GR, DK

**1.** A compound of the formula I

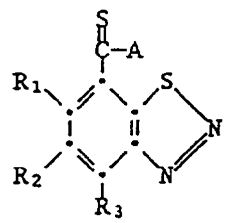

(I),

in which $R_1$, $R_2$ and $R_3$ independently of one another are hydrogen, halogen, methyl, methoxy or methylthio; A is $-OR_4$, $-SR_4$, $-N(R_5)R_6$, $-N(R_7)-N(R_5)R_6$, $-O-N-C(R_8)R_9$, $-N(R_7)-OR_{10}$, or

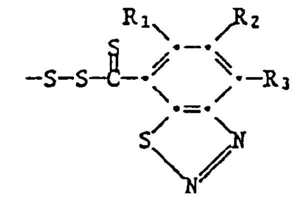

,

$R_4$ is $C_1$-$C_{18}$alkyl, $C_3$-$C_{35}$alkyl which is interrupted by an oxygen or sulfur atom, $C_1$-$C_8$alkyl which is substituted by halogen or $-COOR_7$, $C_3$-$C_6$alkenyl which is unsubstituted or substituted by halogen, $C_3$-$C_6$alkinyl which is unsubstituted or substituted by halogen, $C_4$-$C_7$cycloalkyl which is unsubstituted or substituted by halogen or methyl, an aryl or heterocyclic radical U having not more than 3 heteroatoms O, N and/or S, or a radical U which is linked via an alkylene bridge containing not more than 6 carbon atoms and up to 2 oxygen atoms; U is phenyl, phenoxyphenyl, biphenyl or 1- or 2-naphthyl, each of which is unsubstituted or substituted by halogen, $C_1$-$C_3$alkyl or $C_1$-$C_4$alkoxy, or a saturated or unsaturated 5- to 7-membered heterocyclic radical T which is unsubstituted or substituted by one or more substituents from the group comprising halogen, $C_1$-$C_3$alkyl and/or $C_1$-$C_3$alkoxy; $R_5$ and $R_5$ independently of one another are hydrogen, $C_1$-$C_8$alkyl, $C_1$-$C_6$alkyl which is substituted by $C_1$-$C_3$alkoxy and/or cyano, or $C_3$-$C_6$alkenyl, $C_3$-$C_6$alkinyl, $C_3$-$C_7$cycloalkyl, a radical U or a radical U which is linked via an aklylene bridge containing not more than 2 C atoms; or $R_5$ and $R_5$, together with the nitrogen atom, are a 5- to 7-membered heterocyclic radical W which is unsubstituted or substituted by methyl and has not more than 2 further heteroatoms O, N and/or S; $R_7$ is hydrogen or $C_1$-$C_4$alkyl; $R_5$ and $R_9$ independently of one another are hydrogen, $C_1$-$C_6$alkyl, cyano, $CONH_2$, $CONHCONHR_7$, or phenyl which is unsubstituted or substituted by halogen, $C_1$-$C_3$alkyl or $C_1$-$C_3$alkoxy; or, together with the connecting carbon atom, are also a 5- to 7-membered carbocyclic radical; and $R_{10}$ is hydrogen, $C_1$-$C_6$alkyl, $C_3$-$C_6$alkenyl, $C_5$-$C_7$cycloalkyl, phenyl or benzyl.

2. A compound of the formula I according to claim 1, in which: $R_1$, $R_2$ and $R_3$ independently of one another are hydrogen, fluorine or methyl; A is $-OR_4$, $-SR_4$, $-N(R_5)R_5$, $-N(R_7)-N(R_5)R_5$, $-O-N=C(R_5)R_9$, $-N(R_7)-OR_{10}$ or

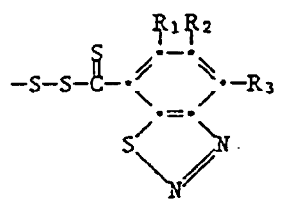

;

$R_4$ is $C_1$-$C_6$alkyl, $C_5$-$C_6$cycloalkyl, phenyl, benzyl, tetrahydrofuran-2-on-3-yl, 2-, 3- or 4-pyridyl, 2-, 4- or 5-pyrimidyl or (2,2-dimethyldioxolan-4-yl)-methyl; $R_5$ and $R_5$ independently of one another are hydrogen, $C_1$-$C_4$alkyl, $C_1$-$C_3$alkyl which is substituted by methoxy, ethoxy and/or cyano, or allyl, propargyl, $C_3$-$C_6$cycloalkyl, phenyl, benzyl, phenethyl, 2-furfurylmethyl, tetrahydrofuran-2-on-3-yl, 2,3- or 4-pyridyl, 2-, 4- or 5-pyrimidyl, $R_7$ is hydrogen, methyl or ethyl; or the group $N(R_5)R_6$ together forms a pyrrole, pyrrolidine, imidazol-1-yl, 1,2,4-triazol-1-yl, piperidine, morpholine or 2,6-dimethylmorpholine ring; $R_5$ and $R_9$ independently of one another are hydrogen, $C_1$-$C_6$alkyl, cyano, the radicals $CONH_2$, $CONHCONHR_7$ or phenyl, or, together with the connecting carbon atom, form a 5- to 7-membered carbocyclic radical; and $R_{10}$ is hydrogen, $C_1$-$C_3$alkyl, allyl, $C_5$-$C_6$cycloalkyl, phenyl or benzyl.

**3.** A compound of the formula I according to claim 1, in which: $R_1$, $R_2$ and $R_3$ independently of one another are hydrogen, fluorine or methyl; A is $-OR_4$, $-SR_4$, $-N(R_5)R_6$, $-N(R_7)-N(R_5)R_6$, $-O-N=C(R_8)R_9$ or $-N(R_7)-OR_{10}$; $R_4$ is $C_1$-$C_6$alkyl, $C_5$-$C_6$cycloalkyl, phenyl, benzyl, tetrahydrofuran-2-on-3-yl or 2-, 3- or 4-pyridyl; $R_6$ and $R_6$ independently of one another are hydrogen, $C_1$-$C_3$alkyl, $C_1$-$C_3$alkyl which is substituted by methoxy and/or cyano, or allyl, propargyl, $C_3$-$C_5$cycloalkyl, phenyl, benzyl, 2-furfuryl, tetrahydrofuran-2-on-3-yl, 2-, 3- or 4-pyridyl, 2-, 4- or 5-pyrimidyl or piperidin-1-yl, or the group $N(R_5)R_6$ together is a pyrrolidine, imidazol-1-yl, piperidine, morpholine or 2,6-dimethylmorpholine ring; $R_7$ is hydrogen, methyl or ethyl; $R_8$ and $R_9$ independently of one another are hydrogen, methyl, ethyl or the radicals cyano, $CONH_2$, phenyl or benzyl, or, together with the connecting carbon atom, are cyclopentyl or cyclohexyl; and $R_{10}$ is hydrogen, methyl, ethyl, allyl, $C_3$-$C_5$cycloalkyl or phenyl.

**4.** A compound of the formula I according to claim 1, in which: $R_1$, $R_2$ and $R_3$ independently of one another are hydrogen or fluorine; A is $-OR_4$, $-SR_4$, $-N(R_5)R_6$ or $-N(R_7)-N(R_5)R_6$; $R_4$ is $C_1$-$C_4$alkyl, $C_5$-$C_6$cycloalkyl, phenyl, benzyl, 2-, 3- or 4-pyridyl or 2-, 4-, or 5-pyrimidyl; $R_6$ and $R_6$ independently of one another are hydrogen, methyl, ethyl, allyl, propargyl, phenyl, benzyl, 2-furanylmethyl or 2-, 3- or 4-pyridyl, or the group $N(R_5)R_6$ together is a pyrrolidine, piperidine, morpholine, 2,6-dimethylmorpholine or imidazol-1-yl ring; and $R_7$ is hydrogen, methyl or ethyl.

**5.** A compound of the formula I according to claim 1 from the group comprising:
O-ethyl benzo-1,2,3-thiadiazole-7-thiocarboxylate
S-methyl benzo-1,2,3-thiadiazole-7-dithiocarboxylate
benzo-1,2,3-thiadiazole-7-carboxylic acid thioamide
7-[benzo-l,2,3-thiadiazole]-N',N'-dimethylthiohydrazide
ethyl benzo-1,2,3-thiadiazole-7-dithiocarboxylate
propyl benzo-1,2,3-thiadiazole-7-dithiocarboxylate
cyclopentyl benzo-1,2,3-thiadiazole-7-dithiocarboxylate
benzo-1,2,3-thiadiazole-7-thiocarboxylic acid morpholide
benzo-1,2,3-thiadiazole-7-thiocarboxylic acid piperidide

**6.** A process for the preparation of a compound of the formula I in claim 1, which comprises
A) reaction of a compound of the formula IIa

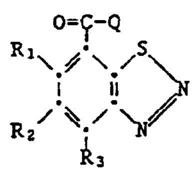

(IIa)

with a thionating reagent, for example with phosphorus pentasulfide ($P_4S_{10}$) or Lawesson's reagent [2,4-bis(4-methoxyphenyl)-1,3,2, 4-dithiadiphosphetane 2,4-disulfide] in an aprotic non-polar or slightly polar solvent at temperatures of 20° to 200°C, preferably 60° to 150°C, Q being the radicals $-OR_4$, $-SR_4$ or $-N(R_6)R_6$, to give a compound of the formula Ia

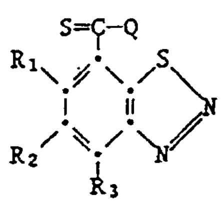

(Ia)

wherein $R_1$, $R_2$ and $R_3$ are as defined under formula I;
$B_1$) reaction of the solution of a nitrile of the formula II

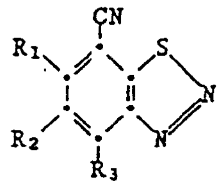

(II)

in an alcohol of the formula $R_4$'OH, in which $R_4$' is as defined for $R_4$ under formula I, by passing gaseous hydrogen halide in at temperatures of -20°C to 80°C, preferably -10° to 40°C, to give an imidate of the formula III

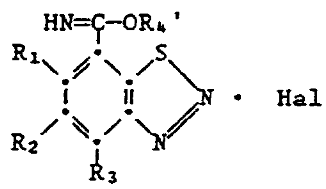

(III),

in which Hal is halogen

and

$B_2$) subsequent reaction of the resulting compound of the formula III with hydrogen sulfide in non-polar or weakly polar solvents in the presence of a base at temperatures of -20° to 80°C, preferably -5°C to 40°C, to give the thiocarboxylic acid esters of the formula Ia'

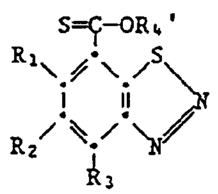

(Ia')

in which $R_1$, $R_2$ and $R_3$ are as defined under formula I and $R_4$' is as defined for $R_4$ under formula I;

C) reaction of a thiono compound of the formula IV

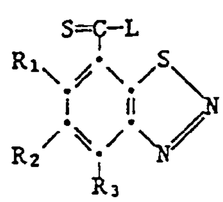

(IV),

in which L has the meaning of chlorine, or of an azolide of the formula

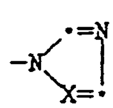

,

in which X is CH or N or has the meaning of -S-$C_1$-$C_4$alkyl, -S-$CH_2$COOM or SM, in which M is hydrogen or an alkali metal ion;

($C_1$) with an alcohol or a thiol of the formula $R_4$'OH or $R_4$'SH, in which $R_4$' is as defined for $R_4$, to give a compound of the formula Ia' or Ia"

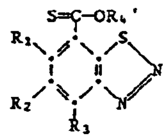

(Ia')

or

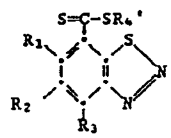

(Ia")

or

($C_2$) with an oxime of the formula $HON=C(R_7)R_8$ to give a compound of the formula Ib

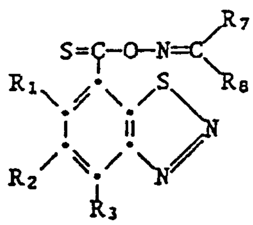

(Ib);

or

($C_3$) with an amine of the formula $HN(R_5)R_6$, to give a compound of the formula Ic

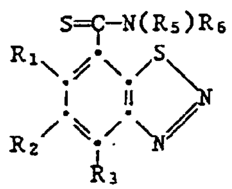

(Ic);

or

($C_4$) with a hydroxylamine derivative of the formula $HN(R_7)\text{-}O\text{-}R_{10}$, to give a compound of the formula Id

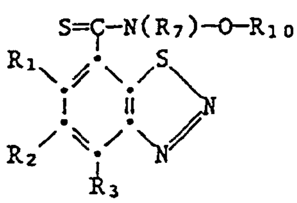

(Id);

or

($C_5$) with a hydrazine derivative of the formula $HN(R_7)\text{-}N(R_5)R_6$, to give a compound of the formula Ie

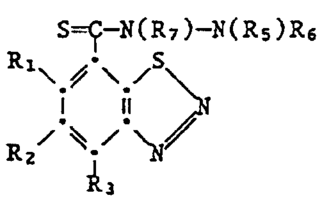

(Ie)

in inert solvents with or without a base at a temperature of -20°C to 170°C;

D) alkylation of the dithio acid or its salt of the formula IVa

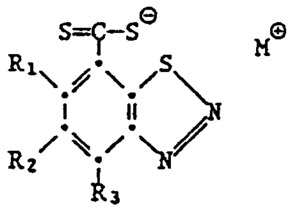

(IVa)

($M^{\oplus}$ = hydrogen or an alkali metal ion)
with an alkylating agent of the formula $R_4$'-L, in which $R_4$' is as defined for $R_4$ and L is a leaving group, in the presence of a base in inert solvents at temperatures of -30° to 110°C to give a compound of the formula Ia"

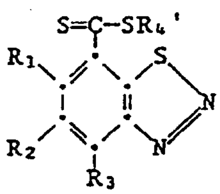

(Ia")

E) oxidation of the dithio acid of its salt of the formula IVa with atmospheric oxygen, potassium hexacyanoferrate (III) or $KI/I_2$ in aqueous solution at temperatures of 10° to 60°C to give a compound of the formula If

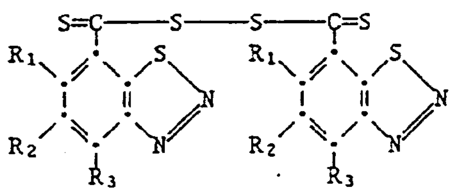

(If),

in which in the above formulae $R_1$ to $R_{10}$ are as defined under formula I.

7. A composition for protecting plants from attack by microorganisms, which contains, in addition to customary carriers and auxiliaries, at least one compound according to claim 1 as the active component.

8. A composition according to claim 7, which contains at least one compound according to any of claims 2 to 4 as the active component.

9. A composition according to claim 7, which contains a compound of the formula I according to claim 5 as the active component.

10. A process for the preparation of an agrochemical composition according to claim 7, which comprises intimately mixing at least one compound defined according to claim 1 with suitable solid or liquid carriers and auxiliaries.

11. The use of a compound according to claim 1 for protecting plants from attack by phytopathogenic microorganisms.

12. The use of a compound according to any one of claims 2 to 5 for protecting plants from attack by phytopathagenic microorganisms.

13. A process for protecting plants from attack by phytopathogenic microorganisms, which comprises applying a compound of the formula I according to claim 1 to the plant or its location as the active substance.

14. A process for protecting plants from attack by phytopathogenic microorganisms, which comprises applying a compound according to any one of claims 2 to 5 to the plants or their location as the active substance.

**15.** The process as claimed in either of claims 13 and 14, wherein the phytopathogenic microorganisms are fungal organisms.

**16.** A compound of the formula III or a salt thereof with a hydrogen halide acid

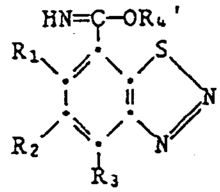

(· HHal) (III),

in which: $R_1$, $R_2$ and $R_3$ independently of one another are hydrogen, halogen, methyl, methoxy or methylthio; $R_4$' is $C_1$-$C_{18}$alkyl, $C_3$-$C_{35}$alkyl which is interrupted by an oxygen or sulfur atom, $C_1$-$C_8$alkyl which is substituted by halogen or -$COOR_7$, $C_3$-$C_6$alkenyl which is unsubstituted or substituted by halogen, $C_3$-$C_6$alkinyl which is unsubstituted or substituted by halogen, $C_4$-$C_7$cycloalkyl which is unsubstituted or substituted by halogen or methyl, an aryl or heterocyclic radical U having not more than 3 heteroatoms O,N and/or S, or a radical U which is linked via an alkylene bridge containing not more than 6 carbon atoms and up to 2 oxygen atoms; U is phenyl, phenoxyphenyl, biphenyl or 1- or 2-naphthyl, each of which is unsubstituted or substituted by halogen, $C_1$-$C_3$alkyl or $C_1$-$C_4$alkoxy, or a saturated or unsaturated 5- to 7-membered heterocyclic radical T which is unsubstituted or substituted by one or more substituents from the group comprising halogen, $C_1$-$C_3$alkyl and/or $C_1$-$C_3$alkoxy; and Hal is halogen.

**17.** A compound of the formula V

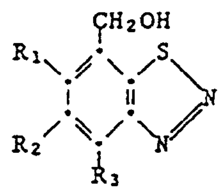

(V),

in which: $R_1$, $R_2$ and $R_3$ independently of one another are hydrogen, halogen, methyl, methoxy or methylthio.

**18.** A compound of the formula VI

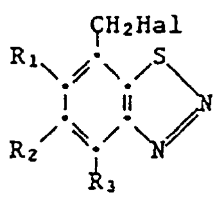

(VI)

in which: $R_1$, $R_2$ and $R_3$ independently of one another are hydrogen, halogen, methyl, methoxy or methylthio and Hal is halogen.

**19.** A process for the preparation of a compound of the formula V

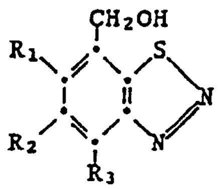

(V),

which comprises reduction of a compound of the formula

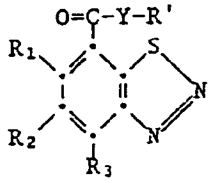

,

in which $R_1$, $R_2$ and $R_3$ independently of one another are hydrogen, halogen, methyl, methoxy or methylthio, R' is hydrogen or $C_1$-$C_4$alkyl and Y is oxygen or sulfur, with a reducing agent in an inert solvent at temperatures of 0° to 60°C.

**20.** A process for the preparation of a compound of the formula VI

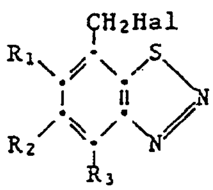

(VI)

which comprises halogenation of a compound of the formula V

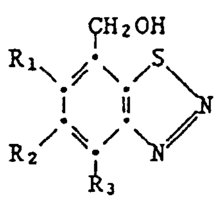

(V),

in which $R_1$, $R_2$ and $R_3$ independently of one another are hydrogen, halogen, methyl, methoxy or methylthio and Hal is halogen, by means of a halogenating agent in the presence of an organic base in an inert solvent at temperatures of -10° to 100°C.

**21.** A composition for protecting plants from attack from microorganisms, which contains, in addition to customary carriers and auxiliaries, at least one compound according to claim 17 or 18 as the active component.

**22.** The use of a compound according to claim 17 or 18 for protecting plants from attack by phytopathogenic microorganisms.

**23.** A process for protecting plants from attack by phytopathogenic microorganisms, which comprises applying a compound according to claim 17 or 18 to the plant or its location as the active substance.

**Claims for the following Contracting State : ES**

**1.** A composition for protecting plants from attack by microorganisms, which contains at least one compound of the formula I

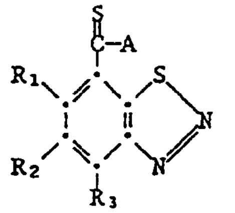

(I),

in which $R_1$, $R_2$ and $R_3$ independently of one another are hydrogen, halogen, methyl, methoxy or methylthio; A is $-OR_4$, $-SR_4$, $-N(R_5)R_6$, $-N(R_7)-N(R_5)R_6$, $-O-N=C(R_5)R_9$, $-N(R_7)-OR_{10}$, or

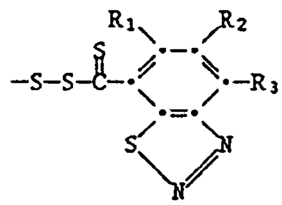

,

$R_4$ is $C_1$-$C_{18}$alkyl, $C_3$-$C_{35}$alkyl which is interrupted by an oxygen or sulfur atom, $C_1$-$C_8$alkyl which is substituted by halogen or $-COOR_7$, $C_3$-$C_6$alkenyl which is unsubstituted or substituted by halogen, $C_3$-$C_6$alkinyl which is unsubstituted or substituted by halogen, $C_4$-$C_7$cycloalkyl which is unsubstituted or substituted by halogen or methyl, an aryl or heterocyclic radical U having not more than 3 heteroatoms O, N and/or S, or a radical U which is linked via an alkylene bridge containing not more than 6 carbon atoms and up to 2 oxygen atoms; U is phenyl, phenoxyphenyl, biphenyl or 1- or 2-naphthyl, each of which is unsubstituted or substituted by halogen, $C_1$-$C_3$alkyl or $C_1$-$C_4$alkoxy, or a saturated or unsaturated 5- to 7-membered heterocyclic radical T which is unsubstituted or substituted by one or more substituents from the group comprising halogen, $C_1$-$C_3$alkyl and/or $C_1$-$C_3$alkoxy; $R_5$ and $R_5$ independently of one another are hydrogen, $C_1$-$C_8$alkyl, $C_1$-$C_6$alkyl which is substituted by $C_1$-$C_3$alkoxy and/or cyano, or $C_3$-$C_6$alkenyl, $C_3$-$C_6$alkinyl, $C_3$-$C_7$cycloalkyl, a radical U or a radical U which is linked via an aklylene bridge containing not more than 2 C atoms; or $R_5$ and $R_5$, together with the nitrogen atom, are a 5- to 7-membered heterocyclic radical W which is unsubstituted or substituted by methyl and has not more than 2 further heteroatoms O, N and/or S; $R_7$ is hydrogen or $C_1$-$C_4$alkyl; $R_5$ and $R_9$ independently of one another are hydrogen, $C_1$-$C_6$alkyl, cyano, $CONH_2$, $CONHCONHR_7$, or phenyl which is unsubstituted or substituted by halogen, $C_1$-$C_3$alkyl or $C_1$-$C_3$alkoxy; or, together with the connecting carbon atom, are also a 5- to 7-membered carbocyclic radical; and $R_{10}$ is hydrogen, $C_1$-$C_6$alkyl, $C_3$-$C_6$alkenyl, $C_5$-$C_7$cycloalkyl, phenyl or benzyl.

2. A composition according to claim 1 which contains as active component at least one compound of the formula I, in which: $R_1$, $R_2$ and $R_3$ independently of one another are hydrogen, fluorine or methyl; A is $-OR_4$, $-SR_4$, $-N(R_5)R_5$, $-N(R_7)-N(R_5)R_6$, $-O-N=C(R_5)R_9$, $-N(R_7)-OR_{10}$
or

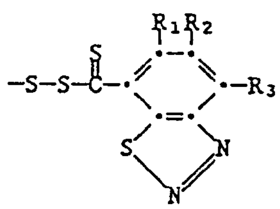

;

$R_4$ is $C_1$-$C_6$alkyl, $C_5$-$C_6$cycloalkyl, phenyl, benzyl, tetrahydrofuran-2-on-3-yl, 2-, 3- or 4-pyridyl, 2-, 4- or 5-pyrimidyl or (2,2-dimethyldioxolan-4-yl)-methyl; $R_5$ and $R_5$ independently of one another are hydrogen, $C_1$-$C_4$alkyl, $C_1$-$C_3$alkyl which is substituted by methoxy, ethoxy and/or cyano, or allyl, propargyl, $C_3$-$C_6$cycloalkyl, phenyl, benzyl, phenethyl, 2-furfurylmethyl, tetrahydrofuran-2-on-3-yl, 2,3- or 4-pyridyl, 2-, 4- or 5-pyrimidyl, $R_7$ is hydrogen, methyl or ethyl; or the group $N(R_5)R_6$ together forms a pyrrole, pyrrolidine, imidazol-1-yl, 1,2,4-triazol-1-yl, piperidine, morpholine or 2,6-dimethylmorpholine ring; $R_5$ and $R_9$ independently of one another are hydrogen, $C_1$-$C_6$alkyl, cyano, the radicals $CONH_2$, $CONHCONHR_7$ or phenyl,

or, together with the connecting carbon atom, form a 5- to 7-membered carbocyclic radical; and $R_{10}$ is hydrogen, $C_1$-$C_3$alkyl, allyl, $C_5$-$C_6$cycloalkyl, phenyl or benzyl.

**3.** A composition according to claim 1 which contains as active component at least one compound of the formula I, in which: $R_1$, $R_2$ and $R_3$ independently of one another are hydrogen, fluorine or methyl; A is -$OR_4$, -$SR_4$, -$N(R_5)R_6$, -$N(R_7)$-$N(R_5)R_6$, -O-N=$C(R_6)R_9$ or -$N(R_7)$-$OR_{10}$; $R_4$ is $C_1$-$C_6$alkyl, $C_5$-$C_6$cycloalkyl, phenyl, benzyl, tetrahydrofuran-2-on-3-yl or 2-, 3- or 4-pyridyl; $R_5$ and $R_6$ independently of one another are hydrogen, $C_1$-$C_3$alkyl, $C_1$-$C_3$alkyl which is substituted by methoxy and/or cyano, or allyl, propargyl, $C_3$-$C_5$cycloalkyl, phenyl, benzyl, 2-furfuryl, tetrahydrofuran-2-on-3-yl, 2-, 3- or 4-pyridyl, 2-, 4- or 5-pyrimidyl or piperidin-l-yl, or the group $N(R_6)R_6$ together is a pyrrolidine, imidazol-l-yl, piperidine, morpholine or 2,6-dimethylmorpholine ring; $R_7$ is hydrogen, methyl or ethyl; $R_8$ and $R_9$ independently of one another are hydrogen, methyl, ethyl or the radicals cyano, $CONH_2$, phenyl or benzyl, or, together with the connecting carbon atom, are cyclopentyl or cyclohexyl; and $R_{10}$ is hydrogen, methyl, ethyl, allyl, $C_3$-$C_5$cycloalkyl or phenyl.

**4.** A composition according to claim 1 which contains as active component at least one compound of the formula I, in which: $R_1$, $R_2$ and $R_3$ independently of one another are hydrogen or fluorine; A is -$OR_4$, -$SR_4$, -$N(R_5)R_6$ or -N(R7)-$N(R_6)R_6$; $R_4$ is $C_1$-$C_4$alkyl, $C_5$-$C_6$cycloalkyl, phenyl, benzyl, 2-, 3- or 4-pyridyl or 2-, 4-, or 5-pyrimidyl; $R_5$ and $R_6$ independently of one another are hydrogen, methyl, ethyl, allyl, propargyl, phenyl, benzyl, 2-furanylmethyl or 2-, 3- or 4-pyridyl, or the group $N(R_5)R_6$ together is a pyrrolidine, piperidine, morpholine, 2,6-dimethylmorpholine or imidazol-1-yl ring; and $R_7$ is hydrogen, methyl or ethyl.

**5.** A composition according to claim 1 which contains as active component at least one compound of the formula I from the group comprising:
O-ethyl benzo-1,2,3-thiadiazole-7-thiocarboxylate
S-methyl benzo-1,2,3-thiadiazole-7-dithiocarboxylate
benzo-1,2,3-thiadiazole-7-carboxylic acid thioamide
7-[benzo-1,2,3-thiadiazole]-N',N'-dimethylthiohydrazide
ethyl benzo-1,2,3-thiadiazole-7-dithiocarboxylate
propyl benzo-1,2,3-thiadiazole-7-dithiocarboxylate
cyclopentyl benzo-1,2,3-thiadiazole-7-dithiocarboxylate
benzo-1,2,3-thiadiazole-7-thiocarboxylic acid morpholide
benzo-1,2,3-thiadiazole-7-thiocarboxylic acid piperidide

**6.** A process for the preparation of a compound of the formula I in claim 1, which comprises
A) reaction of a compound of the formula IIa

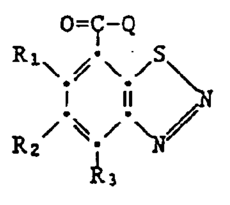

(IIa)

with a thionating reagent, for example with phosphorus pentasulfide ($P_4S_{10}$) or Lawesson's reagent [2,4-bis(4-methoxyphenyl)-1,3,2, 4-dithiadiphosphetane 2,4-disulfide] in an aprotic non-polar or slightly polar solvent at temperatures of 20° to 200°C, preferably 60° to 150°C, Q being the radicals -$OR_4$, -$SR_4$ or -$N(R_6)R_6$, to give a compound of the formula Ia

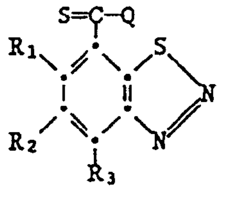

(Ia)

wherein $R_1$, $R_2$ and $R_3$ are as defined under formula I;
$B_1$) reaction of the solution of a nitrile of the formula II

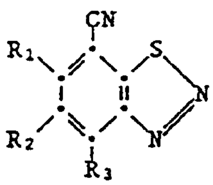

(II)

in an alcohol of the formula $R_4$'OH, in which $R_4$' is as defined for $R_4$ under formula I, by passing gaseous hydrogen halide in at temperatures of -20°C to 80°C, preferably -10° to 40°C, to give an imidate of the formula III

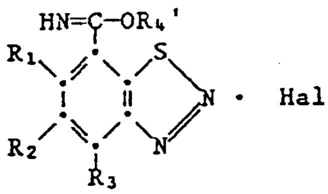

(III),

in which Hal is halogen

and

$B_2$) subsequent reaction of the resulting compound of the formula III with hydrogen sulfide in non-polar or weakly polar solvents in the presence of a base at temperatures of -20° to 80°C, preferably -5°C to 40°C, to give the thiocarboxylic acid esters of the formula Ia'

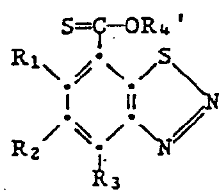

(Ia')

in which $R_1$, $R_2$ and $R_3$ are as defined under formula I and $R_4$' is as defined for $R_4$ under formula I;
C) reaction of a thiono compound of the formula IV

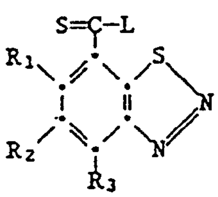

(IV),

in which L has the meaning of chlorine, or of an azolide of the formula

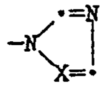

,

in which X is CH or N or has the meaning of -S-$C_1$-$C_4$alkyl, -S-$CH_2$COOM or SM, in which M is hydrogen or an alkali metal ion;
($C_1$) with an alcohol or a thiol of the formula $R_4$'OH or $R_4$'SH, in which $R_4$' is as defined for $R_4$, to give a compound of the formula Ia' or Ia"

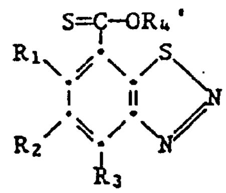

or

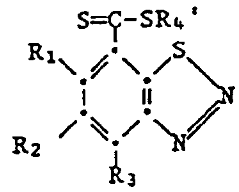

(Ia')

(Ia")

or

($C_2$) with an oxime of the formula HON-C($R_7$)$R_8$ to give a compound of the formula Ib

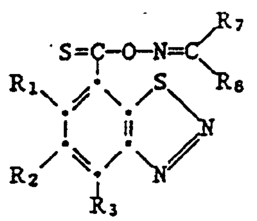

(Ib);

or

($C_3$) with an amine of the formula HN($R_5$)$R_8$, to give a compound of the formula Ic

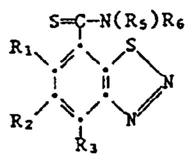

(Ic);

or

($C_4$) with a hydroxylamine derivative of the formula HN($R_7$)-O-$R_{10}$, to give a compound of the formula Id

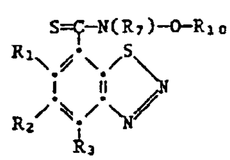

(Id);

or

($C_5$) with a hydrazine derivative of the formula HN($R_7$)-N($R_5$)$R_8$, to give a compound of the formula Ie

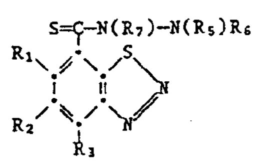

(Ie)

in inert solvents with or without a base at a temperature of -20°C to 170°C;

D) alkylation of the dithio acid or its salt of the formula IVa

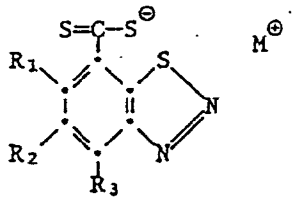

(IVa)

($M^{\oplus}$ = hydrogen or an alkali metal ion)
with an alkylating agent of the formula $R_4$'-L, in which $R_4$' is as defined for $R_4$ and L is a leaving group, in the presence of a base in inert solvents at temperatures of -30° to 110°C to give a compound of the formula Ia"

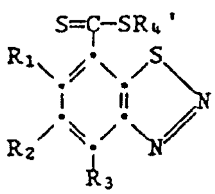

(Ia")

E) oxidation of the dithio acid or its salt of the formula IVa with atmospheric oxygen, potassium hexacyanoferrate (III) or $KI/I_2$ in aqueous solution at temperatures of 10° to 60°C to give a compound of the formula If

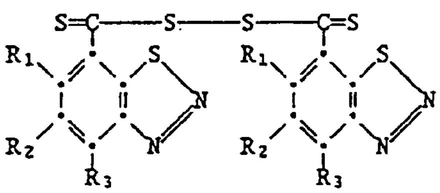

(If),

in which in the above formulae $R_1$ to $R_{10}$ are as defined under formula I.

7. The use of a compound according to claim 1 for protecting plants from attack by phytopathogenic microorganisms.

8. The use of a compound according to any one of claims 2 to 5 for protecting plants from attack by phytopathogenic microorganisms.

9. A process for protecting plants from attack by phytopathogenic microorganisms, which comprises applying a compound according to claim 1 to the plants or their location as the active substance.

10. A process for protecting plants from attack by phytopathogenic microorganisms, which comprises applying a compound according to any one of claims 2 to 5 to the plants or their location as the active substance.

11. The process as claimed in either of claims 9 and 10, wherein the phytopathogenic microorganisms are fungal organisms.

12. A composition for protecting plants from attack by microorganisms, which contains as active component at least one compound of the formula V

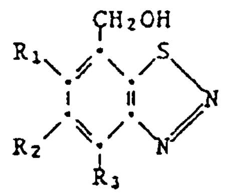

(V),

in which: $R_1$, $R_2$ and $R_3$ independently of one another are hydrogen, halogen, methyl, methoxy or methylthio.

**13.** A composition for protecting plants from attack by mircoorganisms, which contains as active component at least one compound of the formula VI

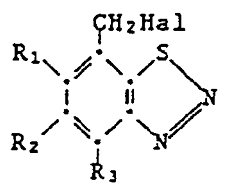

(VI)

in which: $R_1$, $R_2$ and $R_3$ independently of one another are hydrogen, halogen, methyl, methoxy or methylthio and Hal is halogen.

**14.** A process for the preparation of a compound of the formula V

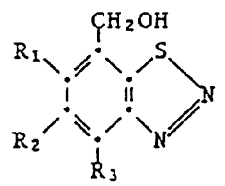

(V),

which comprises reduction of a compound of the formula

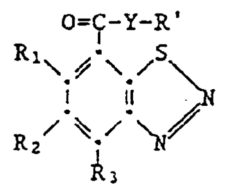

,

in which $R_1$, $R_2$ and $R_3$ independently of one another are hydrogen, halogen, methyl, methoxy or methylthio, R' is hydrogen or $C_1$-$C_4$alkyl and Y is oxygen or sulfur, with a reducing agent in an inert solvent at temperatures of 0° to 60°C.

**15.** A process for the preparation of a compound of the formula VI

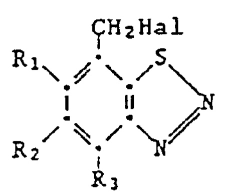

(VI)

which comprises halogenation of a compound of the formula V

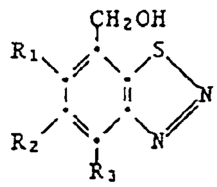

(V),

in which $R_1$, $R_2$ and $R_3$ independently of one another are hydrogen, halogen, methyl, methoxy or methylthio and Hal is halogen, by means of a halogenating agent in the presence of an organic base in an inert solvent at temperatures of -10° to 100°C.

**16.** The use of a compound according to claim 12 or 13 for protecting plants from attack by phytopathogenic microorganisms.

**17.** A process for protecting plants from attack by phytopathogenic microorganisms, which comprises applying a compound according to claim 12 or 13 to the plant or its location as the active substance.

## Revendications

**Revendications pour les Etats contractants suivants : DE, GB, FR, IT, NL, SE, CH, LI, BE, AT, LU, GR, DK**

**1.** Composés de formule I

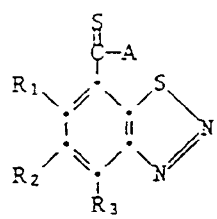

(I),

dans laquelle

$R_1$, $R_2$ et $R_3$ représentent chacun, indépendamment les uns des autres, l'hydrogène, un halogène, un groupe méthyle, méthoxy ou méthylthio;

A représente $-OR_4$, $-SR_4$, $-N(R_5)R_6$, $-N(R_7)-N(R_5)R_6$, $-O-N=C(R_5)R_9$, $-N(R_7)-OR_{10}$, ou

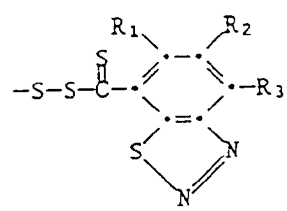

,

$R_4$ représente un groupe alkyle en C 1-C 18, un groupe alkyle en C 3-C 35 interrompu par un atome d'oxygène ou de soufre, un groupe alkyle en C 1-C 8 substitué par un halogène ou par un groupe $-COOR_7$, un groupe alcényle en C3-C 6 non substitué ou substitué par un halogène, un groupe alcynyle en C 3-C 6 non substitué ou substitué par un halogène, un groupe cycloalkyle en C 4-C 7 non substitué ou substitué par un halogène ou par un groupe méthyle, un radical arylique ou hétérocyclique U contenant au maximum 3 hétéroatomes O, N et/ou S, un radical U relié par un pont alkylène contenant au maximum 6 atomes de carbone et jusqu'à 2 atomes d'oxygène;

U représente un groupe phényle, phénoxyphényle, biphényle, 1- ou 2-naphtyle non substitué ou substitué par un halogène, un groupe alkyle en C 1-C 3 ou alcoxy en C 1-C 4, ou bien un hétérocycle saturé ou insaturé de 5 à 7 chaînons, T, non substitué

ou portant un ou plusieurs substituants halogéno, alkyle en C 1-C 3 et/ou alcoxy en C 1-C 3;

$R_5$ et $R_6$ représentent chacun, indépendamment l'un de l'autre, l'hydrogène, un groupe alkyle en C 1-C 8, un groupe alkyle en C 1-C 6 substitué par un groupe alcoxy en C 1-C 3 et/ou cyano, ou un groupe alcényle en C 3-C 6, alcynyle en C 3-C 6, cycloalkyle en C 3-C 7, un radical U, un radical U relié par un pont alkylène contenant au maximum 2 atomes de carbone; ou bien $R_5$ et $R_5$ forment ensemble et avec l'atome d'azote un hétérocycle W de 5 à 7 chaînons, non substitué ou substitué par un groupe méthyle, et contenant au maximum 2 autres hétéroatomes O, N et/ou S;

$R_7$ représente l'hydrogène ou un groupe alkyle en C 1-C 4;

$R_8$ et $R_9$ représentent chacun, indépendamment l'un de l'autre, l'hydrogène, un groupe alkyle en C 1-C 6, cyano, $CONH_2$, $CONHCONHR_7$, un groupe phényle non substitué ou substitué par un halogène, un groupe alkyle en C 1-C 3 ou alcoxy en C 1-C 3; ou bien encore, ensemble et avec l'atome de carbone qui les relie, un carbocycle de 5 à 7 chaînons; et

$R_{10}$ représente l'hydrogène, un groupe alkyle en C 1-C 6, alcényle en C 3-C 6, cycloalkyle en C 5-C 7, phényle ou benzyle.

**2.** Composés de formule I de la revendication 1, pour lesquels :

$R_1$, $R_2$ et $R_3$ représentent chacun, indépendamment les uns des autres, l'hydrogène, le fluor ou un groupe méthyle;

A représente $-OR_4$, $-SR_4$, $-N(R_5)R_6$, $-N(R_7)-N(R_5)R_6$, $-O-N=C(R_8)R_9$, $-N(R_7)-OR_{10}$,

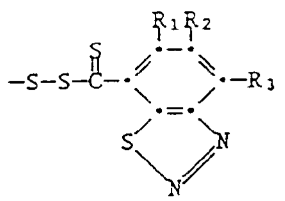

;

$R_4$ représente un groupe alkyle en C 1-C 6, cycloalkyle en C 5-C 6, phényle, benzyle, tétrahydrofuranne-2-one-3-yle, 2-, 3- ou 4-pyridyle, 2-, 4- ou 5-pyrimidyle ou (2,2-diméthyldioxolanne-4-yl)-méthyle;

$R_5$ et $R_6$ représentent chacun, indépendamment l'un de l'autre, l'hydrogène, un groupe alkyle en C 1-C 4, un groupe alkyle en C 1-C 3 substitué par un groupe méthoxy, éthoxy et/ou cyano, ou un groupe allyle, propargyle, cycloalkyle en C 3-C 6, phényle, benzyle, phénéthyle, 2-furfurylméthyle, tétrahydrofuranne-2-one-3-yle, 2-, 3- ou 4-pyridyle, 2-, 4- ou 5-pyrimidyle,

$R_7$ représente l'hydrogène, un groupe méthyle ou éthyle, ou bien le groupe $N(R_5)R_6$ forme un cycle pyrrole, pyrrolidine, imidazole-1-yle, 1,2,4-triazole-1-yle, pipéridine, morpholine ou 2,6-diméthylmorpholine;

$R_8$ et $R_9$ représentent chacun, indépendamment l'un de l'autre, l'hydrogène, un groupe alkyle en C 1-C 6, cyano, $CONH_2$, $CONHCONHR_7$, phényle ou bien, ensemble et avec l'atome de carbone qui les relie, un carbocycle de 5 à 7 chaînons; et

$R_{10}$ représente l'hydrogène, un groupe alkyle en C 1-C 3, allyle, cycloalkyle en C 5-C 6, phényle ou benzyle.

**3.** Composés de formule I de la revendication 1, pour lesquels :

$R_1$, $R_2$ et $R_3$, représentent chacun, indépendamment les uns des autres, l'hydrogène, le fluor ou un groupe méthyle;

A représente $-OR_4$, $-SR_4$, $-N(R_5)R_6$, $-N(R_7)-N(R_5)R_6$;

$R_4$ représente un groupe alkyle en C 1-C 6, cycloalkyle en C 5-C 6, phényle, benzyle, tétrahydrofuranne-2-one-3-yle, 2-, 3- ou 4-pyridyle;

$R_5$ et $R_6$ représentent chacun, indépendamment l'un de l'autre, l'hydrogène, un groupe alkyle en C 1-C 3, un groupe alkyle en C 1-C 3 substitué par un groupe méthoxy et/ou cyano, ou un groupe allyle, propargyle, cycloalkyle en C 3-C 5, phényle, benzyle, 2-furfuryle, tétrahydrofuranne-2-one-3-yle, 2-, 3- ou 4-pyridyle, 2-, 4- ou 5-pyrimidyle, pipéridine-1-yle, ou bien le groupe $N(R_5)R_6$ constitue un cycle pyrrolidine, imidazole-1-yle, pipéridine, morpholine ou 2,6-diméthylmorpholine;

$R_7$ représente l'hydrogène, un groupe méthyle ou éthyie;

$R_8$ et $R_9$ représentent chacun, indépendamment l'un de l'autre, l'hydrogène, un groupe méthyle, éthyle ou un groupe cyano, $CONH_2$, phényle, benzyle, ou bien, ensemble et avec l'atome de carbone qui les relie, un groupe cyclopentyle ou cyclohexyle; et

$R_{10}$ représente l'hydrogène, un groupe méthyle, éthyle, allyle, cycloalkyle en C 3-C 5 ou phényle.

4. Composés de formule I de la revendication 1, pour lesquels

$R_1$, $R_2$ et $R_3$ représentent chacun, indépendamment les uns des autres, l'hydrogène ou le fluor;

A représente $-OR_4$, $-SR_4$, $-N(R_5)R_6$, $-N(R_7)-N(R_5)R_6$;

$R_4$ représente un groupe alkyle en C 1-C 4, cycloalkyle en C 5-C 6, phényle, benzyle, 2-, 3- ou 4-pyridyle, 2-, 4- ou 5-pyrimidyle;

$R_5$ et $R_6$ représentent chacun, indépendamment l'un de l'autre, l'hydrogène, un groupe méthyle, éthyle, allyle, propargyle, phényle, benzyle, 2-furannylméthyle, 2-, 3- ou 4-pyridyle, ou bien le groupe $N(R_5)R_6$ constitue un cycle pyrrolidine, pipéridine, morpholine, 2,6-diméthylmorpholine ou imidazole-1-yle; et

$R_7$ représente l'hydrogène, un groupe méthyle ou éthyle.

5. Un composé de formule I de la revendication 1, pris dans le groupe suivant :
benzo-1,2,3-thiadiazole-7-thiocarboxylate d'O-éthyle,
benzo-1,2,3-thiadiazole-7-dithiocarboxylate de méthyle,
benzo-1,2,3-thiadiazole-7-carboxythioamide,
7-(benzo-1,2,3-thiadiazole)-N',N'-diméthylthiohydrazide,
benzo-1,2,3-thiadiazole-7-dithiocarboxylate d'éthyle,
benzo-1,2,3-thiadiazole-7-dithiocarboxylate de propyle,
benzo-1,2,3-thiadiazole-7-dithiocarboxylate de cyclopentyle,
benzo-1,2,3-thiadiazole-7-thiocarboxymorpholide,
benzo-1,2,3-thiadiazole-7-thiocarboxypipéridide.

6. Procédé de préparation des composés de formule I de la revendication 1, caractérisé en ce que :
A. on fait réagir un composé de formule IIa

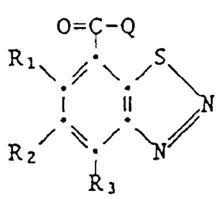

(IIa)

avec un réactif de thionation, par exemple le pentasulfure de phosphore ($P_4S_{10}$) ou le réactif de Lawesson [2,4-bisulfure de 2,4-bis-(4-méthoxyphényl)-1,3,2,4-dithiadiphosphéthanne] dans un solvant aprotonique non polaire ou peu polaire à des températures de 20 à 200°C, de préférence de 60 à 150°C, Q représentant les groupes -OR4, $-SR_4$ ou $-N(R_5)R_5$, ce qui donne un composé de formule Ia

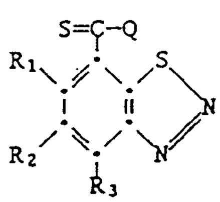

(Ia)

dans laquelle $R_1$, $R_2$ et $R_3$ ont les significations indiquées en référence à la formule I,
$B_1$) on convertit la solution d'un nitrile de formule II

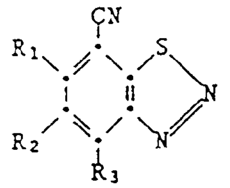

(II)

dans un alcool de formule $R'_4OH$, dans laquelle $R'_4$ a les significations de $R_4$ indiquées en référence à la formule I, par injection d'un halogénure d'hydrogène gazeux, à des températures de -20 à 80°C, de préférence de -10 à 40°C, en un imidate de formule III

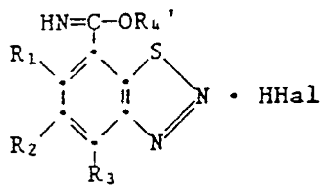

(III),

dans laquelle Hal représente un halogène;

et

$B_2$) on fait ensuite réagir le composé obtenu, répondant à la formule III, avec le sulfure d'hydrogène dans des solvants non polaires ou peu polaires en présence d'une base, à des températures de -20 à 80°C, de préférence de -5 à 40°C, ce qui donne un ester d'acide thiocarboxylique de formule I'a

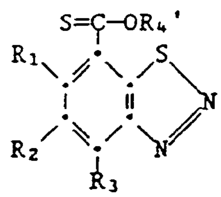

(Ia')

dans laquelle $R_1$, $R_2$ et $R_3$ ont les significations indiquées en référence à la formule I et $R'_4$ les significations de $R_4$ indiquées en référence à la formule I;

C) on fait réagir un thiono-dérivé de formule IV

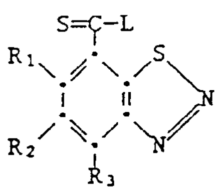

(IV),

dans laquelle L représente le chlore ou un azolide de formule

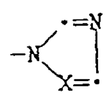

,

dans laquelle X représente CH ou N, ou encore un groupe -S-alkyle en C 1-C 4, $-S-CH_2COOM$ ou SM, M représentant l'hydrogène ou un ion alcalin;

$C_1$) avec un alcool ou un thiol de formules respectives $R'_4OH$ et $R'_4SH$, $R'_4$ ayant les significations de $R_4$, ce qui donne un composé de formule I'a ou I"a

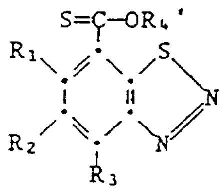

ou

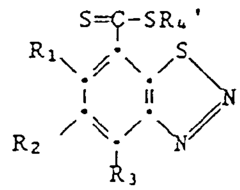

(Ia')

(Ia")

ou bien

$C_2$) avec une oxime de formule $HON=C(R_7)R_8$, ce qui donne un composé de formule Ib

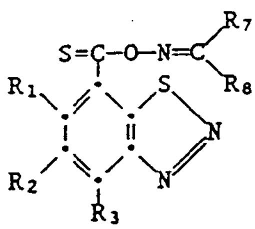

(Ib);

ou bien

$C_3$) avec une amine de formule $HN(R_5)R_6$, ce qui donne un composé de formule Ic

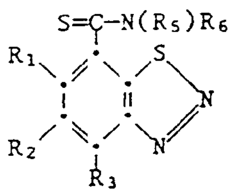

(Ic);

ou bien

$C_4$) avec un dérivé de l'hydroxylamine de formule $HN(R_7)$-O-$R_{10}$, ce qui donne un composé de formule Id

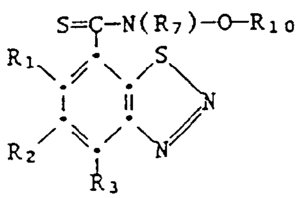

(Id);

ou bien

$C_5$) avec un dérivé de l'hydrazine de formule $NH(R_7)$-$N(R_5)R_6$, ce qui donne un composé de formule Ie

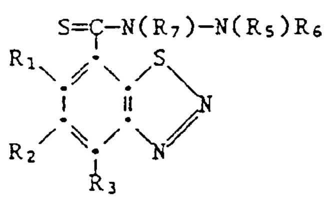

(Ie)

dans un solvant inerte, en présence ou non d'une base, à une température de -20 à 170°C;

D) on alkyle un dithioacide ou l'un de ses sels, de formule IVa

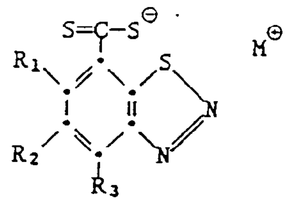

(IVa)

($M^{\oplus}$ = hydrogène ou ion alcalin)
à l'aide d'un agent aikylant de formule $R'_4$-L dans laquelle $R'_4$ a les significations de $R_4$ et L représente un substituant éliminable, en présence d'une base, dans des solvants inertes, à des températures de -30 à 110°C, ce qui donne un composé de formule I"a

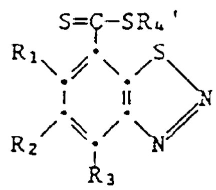

(Ia")

E) On oxyde un dithioacide ou l'un de ses sels de formule IVa par l'oxygène de l'air, l'hexacyanoferrate-III de potassium ou $KI/I_2$ en solution aqueuse à des températures de 10 à 60°C, ce qui donne un composé de formule If

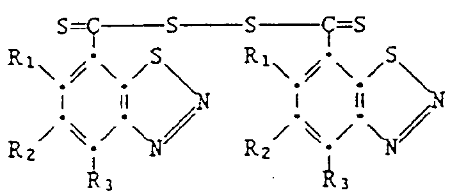

(If),

les symboles $R_1$ à $R_{10}$ ayant, dans les formules ci-dessus, les significations indiquées en référence à la formule I.

7. Produit servant à la protection des végétaux contre l'attaque par des microorganismes, caractérisé en ce qu'il contient, avec des véhicules et produits auxiliaires usuels, au moins un composant actif consistant en un composé de formule I de la revendication 1.

8. Produit selon revendication 7, caractérisé en ce qu'il contient au moins un composant actif consistant en un composé selon les revendications 2 à 4.

9. Produit selon la revendication 7, caractérisé en ce qu'il contient en tant que composant actif le composé de formule I de la revendication 5.

10. Procédé de préparation d'un produit agrochimique selon revendication 7, caractérisé en ce que l'on mélange intimement au moins un composé défini dans la revendication 1 avec des véhicules et produits auxiliaires solides ou liquides appropriés.

11. Utilisation des composés de formule I de la revendication 1 pour la protection des végétaux contre l'attaque par des microorganismes phytopathogènes.

12. Utilisation des composés de formule I selon l'une des revendications 2 à 5 pour la protection des végétaux contre l'attaque par des microorganismes phytopathogènes.

13. Procédé pour protéger les végétaux contre l'attaque par des microorganismes phytopathogènes, caractérisé en ce que l'on applique sur la plante ou son habitat une substance active consistant en un composé de formule I de la revendication 1.

**14.** Procédé pour protéger les végétaux contre l'attaque par des microorganismes phytopathogènes, caractérisé en ce que l'on applique sur la plante ou son habitat une substance active consistant en un composé selon l'une des revendications 2 à 5.

**15.** Procédé selon les revendications 13 et 14, caractérisé en ce que les microorganismes phytopathogènes sont des mycètes.

**16.** Composés de formule III et leurs sels d'hydracides halogénés

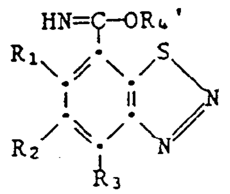

(· HHal) (III),

pour lesquels :

| | |
|---|---|
| $R_1$, $R_2$ et $R_3$ | représentent chacun, indépendamment les uns des autres, l'hydrogène, un halogène, un groupe méthyle, méthoxy ou méthylthio; |
| $R'_4$ | représente un groupe alkyle en C 1-C 18, un groupe alkyle en C 3-C 35 interrompu par un atome d'oxygène ou de soufre, un groupe alkyle en C 1-C 8 substitué par un halogène ou un groupe $-COOR_7$, un groupe alcényle en C 3-C 6 non substitué ou substitué par un halogène, un groupe alcynyle en C 3-C 6 non substitué ou substitué par un halogène, un groupe cycloalkyle en C 4-C 7 non substitué ou substitué par un halogène ou un groupe méthyle, un radical arylique ou hétérocyclique U contenant au maximum 3 hétéroatomes O, N et/ou S, un radical U relié par un pont alkylène contenant au maximum 6 atomes de carbone et jusqu'à 2 atomes d'oxygène; |
| U | représente un groupe phényle, phénoxyphényle, biphényle, 1- ou 2-naphtyle non substitué ou substitué par un groupe alkyle en C 1-C 3 ou alcoxy en C 1-C 4, ou un hétérocycle saturé ou insaturé de 5 à 7 chaînons, T, non substitué ou portant un ou plusieurs substituants halogéno, alkyle en C 1-C 3 et/ou alcoxy en C 1-C 3; et |
| Hal | représente un halogène. |

**17.** Composés de formule V

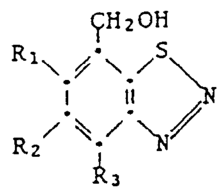

(V),

dans laquelle

| | |
|---|---|
| $R_1$, $R_2$ et $R_3$ | représentent chacun, indépendamment les uns des autres, l'hydrogène, un halogène, un groupe méthyle, méthoxy ou méthylthio. |

**18.** Composés de formule VI

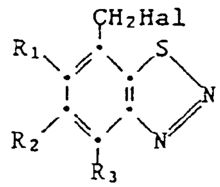

(VI)

dans laquelle

$R_1$, $R_2$ et $R_3$ représentent chacun, indépendamment les uns des autres, l'hydrogène, un halogène, un groupe méthyle, méthoxy ou méthylthio, et Hal représente un halogène.

**19.** Procédé de préparation des composés de formule V

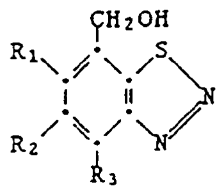

(V), ,

caractérisé en ce que l'on réduit un composé de formule

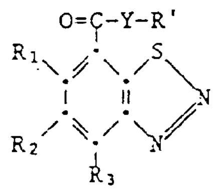

,

dans laquelle $R_1$, $R_2$ et $R_3$ représentent chacun, indépendamment les uns des autres, l'hydrogène, un halogène, un groupe méthyle, méthoxy ou méthylthio, R' représente l'hydrogène ou un groupe alkyle en C 1-C 4 et Y l'oxygène ou le soufre, à l'aide d'un agent réducteur, dans un solvant inerte, à des températures de 0 à 60°C.

**20.** Procédé de préparation des composés de formule VI

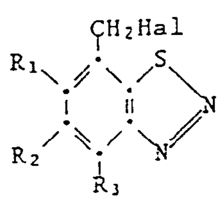

(VI)

caractérisé en ce que l'on halogène un composé de formule V

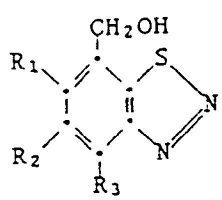

(V), ,

dans laquelle $R_1$, $R_2$ et $R_3$ représentent chacun, indépendamment les uns des autres, l'hydrogène, un halogène, un groupe méthyle, méthoxy ou méthylthio et Hal représente un halogène, à l'aide d'un agent halogénant, en présence d'une base organique, dans un solvant inerte, à des températures de -10 à 100°C.

**21.** Produit pour la protection des végétaux contre l'attaque par des microorganismes, caractérisé en ce qu'il contient, avec des véhicules et produits auxiliaires usuels, au moins un composant actif consistant en un composé selon revendication 17 ou 18.

**22.** Utilisation d'un composé selon revendication 17 ou 18 pour la protection des végétaux contre l'attaque par des microorganismes phytopathogènes.

**23.** Procédé pour protéger les végétaux contre l'attaque par des microorganismes phytopathogènes, caractérisé en ce que l'on applique sur la plante ou son habitat une substance active consistant en un composé selon revendication 17 ou 18.

**Revendications pour l'Etat contractant suivant : ES**

**1.** Produit pour protéger les végétaux contre l'attaque par des microorganismes, caractérisé en ce qu'il contient au moins un composant actif consistant en un composé de formule I

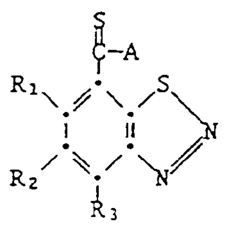 (I),

dans laquelle :

| | |
|---|---|
| $R_1$, $R_2$ et $R_3$ | représentent chacun, indépendamment les uns des autres, l'hydrogène, un halogène, un groupe méthyle, méthoxy ou méthylthio; |
| A | représente $-OR_4$, $-SR_4$, $-N(R_5)R_6$, $-N(R_7)-N(R_5)R_6$, $-O-N=C(R_5)R_9$, $-N(R_7)-OR_{10}$, ou 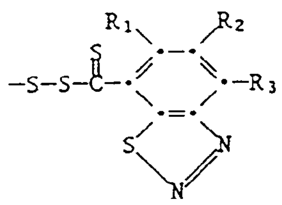 , |
| $R_4$ | représente un groupe alkyle en C 1-C 18, un groupe alkyle en C 3-C 35 interrompu par un atome d'oxygène ou de soufre, un groupe alkyle en C 1-C 8 substitué par un halogène ou par un groupe $-COOR_7$, un groupe alcényle en C 3-C 6 non substitué ou substitué par un halogène, un groupe alcynyle en C 3-C 6 non substitué ou substitué par un halogène, un groupe cycloalkyle en C 4-C 7 non substitué ou substitué par un halogène ou par un groupe méthyle, un radical arylique ou hétérocyclique U contenant au maximum 3 hétéroatomes O, N et/ou S, un radical U relié par un pont alkylène contenant au maximum 6 atomes de carbone et jusqu'à 2 atomes d'oxygène; |
| U | représente un groupe phényle, phénoxyphényle, biphényle, 1- ou 2-naphtyle non substitué ou substitué par un halogène, un groupe alkyle en C 1-C 3 ou alcoxy en C 1-C 4, ou bien un hétérocycle saturé ou insaturé de 5 à 7 chainons, T, non substitué ou portant un ou plusieurs substituants halogéno, alkyle en C 1-C 3 et/ou alcoxy en C 1-C 3; |
| $R_5$ et $R_6$ | représentent chacun, indépendamment l'un de l'autre, l'hydrogène, un groupe alkyle en C 1-C 8, un groupe alkyle en C 1-C 6 substitué par un groupe alcoxy en C 1-C 3 et/ou cyano, ou un groupe alcényle en C 3-C 6, alcynyle en C 3-C 6, cycloalkyle en C 3-C 7, un radical U, un radical U relié par un pont alkylène contenant au maximum |

2 atomes de carbone; ou bien $R_5$ et $R_5$ forment ensemble et avec l'atome d'azote un hétérocycle W de 5 à 7 chaînons, non substitué ou substitué par un groupe méthyle, et contenant au maximum 2 autres hétéroatomes O, N et/ou S;

$R_7$ représente l'hydrogène ou un groupe alkyle en C 1-C 4;

$R_8$ et $R_9$ représentent chacun, indépendamment l'un de l'autre, l'hydrogène, un groupe alkyle en C 1-C 6, cyano, $CONH_2$, $CONHCONHR_7$, un groupe phényle non substitué ou substitué par un halogène, un groupe alkyle en C 1-C 3 ou alcoxy en C 1-C 3; ou bien encore, ensemble et avec l'atome de carbone qui les relie, un carbocycle de 5 à 7 chaînons; et

$R_{10}$ représente l'hydrogène, un groupe alkyle en C 1-C 6, alcényle en C 3-C 6, cycloalkyle en C 5-C 7, phényle ou benzyle.

**2.** Produit selon revendication 1, caractérisé en ce qu'il contient au moins un composant actif consistant en un composé de formule I dans laquelle :

$R_1$, $R_2$ et $R_3$ représentent chacun, indépendamment les uns des autres, l'hydrogène, le fluor ou un groupe méthyle;

A représente $-OR_4$, $-SR_4$, $-N(R_5)R_6$, $-N(R_7)-N(R_5)R_6$, $-O-N=C(R_8)R_9$, $-N(R_7)-OR_{10}$,

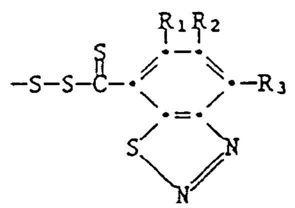 ;

$R_4$ représente un groupe alkyle en C 1-C 6, cycloalkyle en C 5-C 6, phényle, benzyle, tétrahydrofuranne-2-one-3-yle, 2-, 3- ou 4-pyridyle, 2-, 4- ou 5-pyrimidyle ou (2,2-diméthyldioxolanne-4-yl)-méthyle;

$R_5$ et $R_6$ représentent chacun, indépendamment l'un de l'autre, l'hydrogène, un groupe alkyle en C 1-C 4, un groupe alkyle en C 1-C 3 substitué par un groupe méthoxy, éthoxy et/ou cyano, ou un groupe allyle, propargyle, cycloalkyle en C 3-C 6, phényle, benzyle, phénéthyle, 2-furfurylméthyle, tétrahydrofuranne-2-one-3-yle, 2-, 3- ou 4-pyridyle, 2-, 4- ou 5-pyrimidyle,

$R_7$ représente l'hydrogène, un groupe méthyle ou éthyle, ou bien le groupe $N(R_5)R_6$ forme un cycle pyrrole, pyrrolidine, imidazole-1-yle, 1,2,4-triazole-1-yle, pipéridine, morpholine ou 2,6-diméthylmorpholine;

$R_8$ et $R_9$ représentent chacun, indépendamment l'un de l'autre, l'hydrogène, un groupe alkyle en C 1-C 6, cyano, $CONH_2$, $CONHCONHR_7$, phényle ou bien, ensemble et avec l'atome de carbone qui les relie, un carbocycle de 5 à 7 chaînons; et

$R_{10}$ représente l'hydrogène, un groupe alkyle en C 1-C 3, allyle, cycloalkyle en C 5-C 6, phényle ou benzyle.

**3.** Produit selon revendication 1, caractérisé en ce qu'il contient au moins un composant actif consistant en un composé pour lequel :

$R_1$, $R_2$ et $R_3$, représentent chacun, indépendamment les uns des autres, l'hydrogène, le fluor ou un groupe méthyle;

A représente $-OR_4$, $-SR_4$, $-N(R_5)R_6$, $-N(R_7)-N(R_5)R_6$;

$R_4$ représente un groupe alkyle en C 1-C 6, cycloalkyle en C 5-C 6, phényle, benzyle, tétrahydrofuranne-2-one-3-yle, 2-, 3- ou 4-pyridyle;

$R_5$ et $R_6$ représentent chacun, indépendamment l'un de l'autre, l'hydrogène, un groupe alkyle en C 1-C 3, un groupe alkyle en C 1-C 3 substitué par un groupe méthoxy et/ou cyano, ou un groupe allyle, propargyle, cycloalkyle en C 3-C 5, phényle, benzyle, 2-furfuryle, tétrahydrofuranne-2-one-3-yle, 2-, 3- ou 4-pyridyle, 2-, 4- ou 5-pyrimidyle, pipéridine-1-yle, ou bien le groupe $N(R_5)R_6$ constitue un cycle pyrrolidine, imidazole-1-yle, pipéridine, morpholine ou 2,6-diméthylmorpholine;

$R_7$ représente l'hydrogène, un groupe méthyle ou éthyle;

$R_8$ et $R_9$ représentent chacun, indépendamment l'un de l'autre, l'hydrogène, un groupe méthyle, éthyle ou un groupe cyano, $CONH_2$, phényle, benzyle, ou bien, ensemble et avec l'atome de carbone qui les relie, un groupe cyclopentyle ou cyclohexyle; et

$R_{10}$ représente l'hydrogène, un groupe méthyle, éthyle, allyle, cycloalkyle en C 3-C 5 ou phényle.

**4.** Produit selon revendication 1, caractérisé en ce qu'il contient au moins un composant actif consistant en un composé de formule I dans laquelle :

$R_1$, $R_2$ et $R_3$ représentent chacun, indépendamment les uns des autres, l'hydrogène ou le fluor;
A représente $-OR_4$, $-SR_4$, $-N(R_5)R_6$, $-N(R_7)-N(R_5)R_6$;
$R_4$ représente un groupe alkyle en C 1-C 4, cycloalkyle en C 5-C 6, phényle, benzyle, 2-, 3- ou 4-pyridyle, 2-, 4- ou 5-pyrimidyle;
$R_5$ et $R_6$ représentent chacun, indépendamment l'un de l'autre, l'hydrogène,-un groupe méthyle, éthyle, allyle, propargyle, phényle, benzyle, 2-furannylméthyle, 2-, 3- ou 4-pyridyle, ou bien le groupe $N(R_5)R_6$ constitue un cycle pyrrolidine, pipéridine, morpholine, 2,6-diméthylmorpholine ou imidazole-1-yle; et
$R_7$ représente l'hydrogène, un groupe méthyle ou éthyle.

**5.** Produit selon revendication 1, caractérisé en ce qu'il contient au moins un composant actif consistant en un composé de formule I pris dans le groupe suivant :
benzo-1,2,3-thiadiazole-7-thiocarboxylate d'O-éthyle,
benzo-1,2,3-thiadiazole-7-dithiocarboxylate de méthyle,
benzo-1,2,3-thiadiazole-7-carboxythioamide,
7-(benzo-1,2,3-thiadiazole)-N',N'-diméthylthiohydrazide,
benzo-1,2,3-thiadiazole-7-dithiocarboxylate d'éthyle,
benzo-1,2,3-thiadiazole-7-dithiocarboxylate de propyle,
benzo-1,2,3-thiadiazole-7-dithiocarboxylate de cyclopentyle,
benzo-1,2,3-thiadiazole-7-thiocarboxymorpholide,
benzo-1,2,3-thiadiazole-7-thiocarboxypipéridide.

**6.** Procédé de préparation des composés de formule I de la revendication 1, caractérisé en ce que :
A. on fait réagir un composé de formule IIa

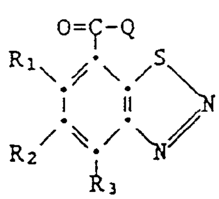

(IIa)

avec un réactif de thionation, par exemple le pentasulfure de phosphore ($P_4S_{10}$) ou le réactif de Lawesson [2,4-bisulfure de 2,4-bis-(4-méthoxyphényl)-1,3,2,4-dithiadiphosphéthanne] dans un solvant aprotonique non polaire ou peu polaire à des températures de 20 à 200°C, de préférence de 60 à 150°C, Q représentant les groupes $-OR_4$, $-SR_4$ ou $-N(R_5)R_5$, ce qui donne un composé de formule Ia

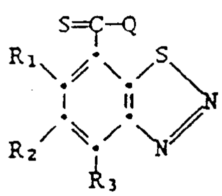

(Ia)

dans laquelle $R_1$, $R_2$ et $R_3$ ont les significations indiquées en référence à la formule I,
$B_1$) on convertit la solution d'un nitrile de formule II

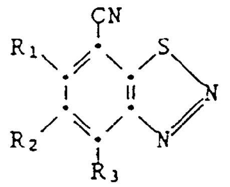

(II)

dans un alcool de formule $R'_4OH$, dans laquelle R'4 a les significations de $R_4$ indiquées en référence à la formule I, par injection d'un halogénure d'hydrogène gazeux, à des températures de -20 à 80°C, de préférence de -10 à 40°C, en un imidate de formule III

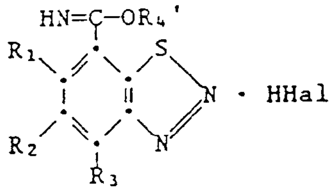

(III),

dans laquelle Hal représente un halogène;
et
$B_2$) on fait ensuite réagir le composé obtenu, répondant à la formule III, avec le sulfure d'hydrogène dans des solvants non polaires ou peu polaires en présence d'une base, à des températures de -20 à 80°C, de préférence de -5 à 40°C, ce qui donne un ester d'acide thiocarboxylique de formule I'a

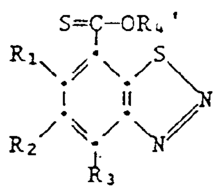

(Ia')

dans laquelle $R_1$, $R_2$ et $R_3$ ont les significations indiquées en référence à la formule I et $R'_4$ les significations de $R_4$ indiquées en référence à la formule I;
C) on fait réagir un thiono-dérivé de formule IV

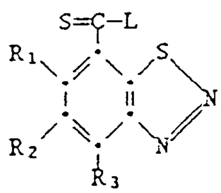

(IV),

dans laquelle L représente le chlore ou un azolide de formule

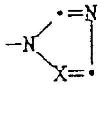

,

dans laquelle X représente CH ou N, ou encore un groupe -S-alkyle en C 1-C 4, $S-CH_2COOM$ ou SM, M représentant l'hydrogène ou un ion alcalin;
$C_1$) avec un alcool ou un thiol de formules respectives $R'_4OH$ et $R'_4SH$, $R'_4$ ayant les significations de $R_4$, ce qui donne un composé de formule I'a ou I''a

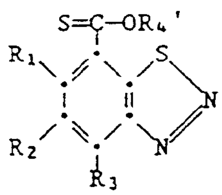

ou

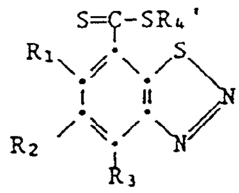

(Ia')

(Ia")

ou bien

$C_2$) avec une oxime de formule $HON{=}C(R_7)R_8$, ce qui donne un composé de formule Ib

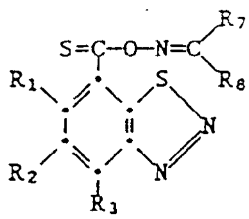

(Ib);

ou bien

$C_3$) avec une amine de formule $HN(R_5)R_6$, ce qui donne un composé de formule Ic

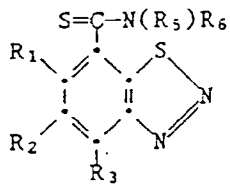

(Ic);

ou bien

$C_4$) avec un dérivé de l'hydroxylamine de formule $HN(R_7)$-O-$R_{10}$, ce qui donne un composé de formule Id

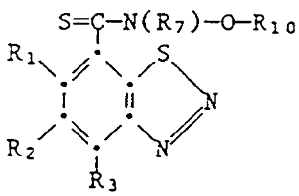

(Id);

ou bien

$C_5$) avec un dérivé de l'hydrazine de formule $NH(R_7)$-$N(R_5)R_6$, ce qui donne un composé de formule Ie

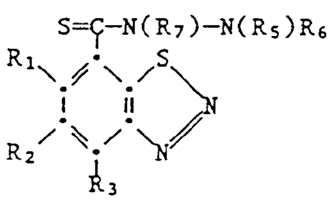

(Ie)

dans un solvant inerte, en présence ou non d'une base, à une température de -20 à 170°C;

D) on alkyle un dithioacide ou l'un de ses sels, de formule IVa

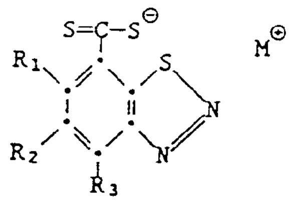

(IVa)

($M^{\oplus}$ = hydrogène ou ion alcalin)
à l'aide d'un agent alkylant de formule $R'_4$-L dans laquelle $R'_4$ a les significations de $R_4$ et L représente un substituant éliminable, en présence d'une base, dans des solvants inertes, à des températures de -30 à 110°C, ce qui donne un composé de formule I"a

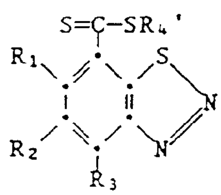

(Ia")

E) On oxyde un dithioacide ou l'un de ses sels de formule IVa par l'oxygène de l'air, l'hexacyanoferrate-III de potassium ou $KI/I_2$ en solution aqueuse à des températures de 10 à 60°C, ce qui donne un composé de formule If

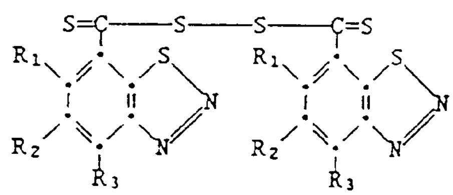

(If),

les symboles $R_1$ à $R_{10}$ ayant, dans les formules ci-dessus, les significations indiquées en référence à la formule I.

**7.** Utilisation des composés de formule I de la revendication 1 pour la protection des végétaux contre l'attaque par des microorganismes phytopathogènes.

**8.** Utilisation des composés selon l'une des revendications 2 à 5 pour la protection des végétaux contre l'attaque par des microorganismes phytopathogènes.

**9.** Procédé pour protéger les végétaux contre l'attaque par des microorganismes phytopathogènes, caractérisé en ce que l'on applique sur la plante ou son habitat une substance active consistant en un composé de formule I de la revendication 1.

**10.** Procédé pour protéger les végétaux contre l'attaque par des microorganismes phytopathogènes, caractérisé en ce que l'on applique sur la plante ou son habitat une substance active consistant en un composé selon l'une des revendications 2 à 5.

**11.** Procédé selon les revendications 9 et 10, caractérisé en ce que les microorganismes phytopathogènes sont des mycètes.

**12.** Produit pour protéger les végétaux contre l'attaque par des microorganismes, caractérisé en ce qu'il contient au moins un composant actif consistant en un composé de formule V

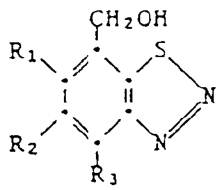

(V),

,

dans laquelle $R_1$, $R_2$ et $R_3$, représentent chacun, indépendamment les uns des autres, l'hydrogène, un halogène, un groupe méthyle, méthoxy ou méthylthio.

**13.** Produit pour protéger les végétaux contre l'attaque par des microorganismes, caractérisé en ce qu'il contient au moins un composant actif consistant en un composé de formule VI

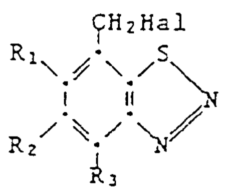

(VI)

dans laquelle

$R_1$, $R_2$ et $R_3$ représentent chacun, indépendamment les uns des autres, l'hydrogène, un halogène, un groupe méthyle, méthoxy ou méthylthio, et Hal un halogène.

**14.** Procédé de préparation des composés de formule V

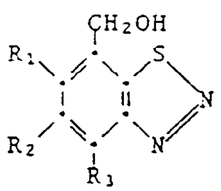

(V),

,

caractérisé en ce que l'on réduit un composé de formule

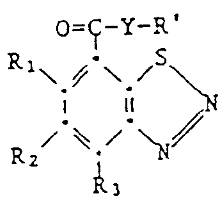

,

dans laquelle $R_1$, $R_2$ et $R_3$ représentent chacun, indépendamment les uns des autres, l'hydrogène, un halogène, un groupe méthyle, méthoxy ou méthylthio, R' représente l'hydrogène ou un groupe alkyle en C 1-C 4 et Y l'oxygène ou le soufre, à l'aide d'un agent réducteur dans un solvant inerte, à des températures de 0 à 60°C.

**15.** Procédé de préparation des composés de formule VI

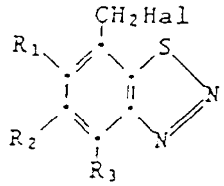

(VI)

caractérisé en ce que l'on halogène un composé de formule V

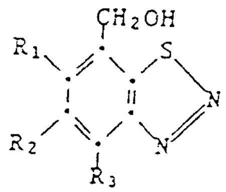

(V),

dans laquelle $R_1$, $R_2$ et $R_3$ représentent chacun, indépendamment les uns des autres, l'hydrogène, un halogène, un groupe méthyle, méthoxy ou méthylthio et Hal un halogène, à l'aide d'un agent halogénant en présence d'une base organique dans un solvant inerte, à des températures de -10 à 100°C.

**16.** Utilisation d'un composé selon revendication 12 ou 13 pour la protection des végétaux contre l'attaque par des microorganismes phytopathogènes.

**17.** Procédé pour protéger les végétaux contre l'attaque par des microorganismes phytopathogènes, caractérisé en ce que l'on applique sur la plante ou son habitat une substance active consistant en un composé selon revendication 12 ou 13.